(19) Europäisches Patentamt
European Patent Office
Office européen des brevets

(11) EP 1 232 167 B1

(12) FASCICULE DE BREVET EUROPEEN

(45) Date de publication et mention de la délivrance du brevet:
**05.05.2010 Bulletin 2010/18**

(21) Numéro de dépôt: **00985310.2**

(22) Date de dépôt: **23.11.2000**

(51) Int Cl.:
***C07H 3/06*** *(2006.01)* ***C07K 16/18*** *(2006.01)*

(86) Numéro de dépôt international:
**PCT/FR2000/003265**

(87) Numéro de publication internationale:
**WO 2001/038338 (31.05.2001 Gazette 2001/22)**

(54) **OLIGOMANNOSIDES DE SYNTHESE, LEUR PREPARATION ET LEURS UTILISATIONS**

SYNTHETISCHES OLIGOMANNOSID, SEINE HERSTELLUNG UND VERWENDUNG

SYNTHETIC OLIGOMANNOSIDES, PREPARATION AND USES THEREOF

(84) Etats contractants désignés:
**AT BE CH CY DE DK ES FI FR GB GR IE IT LI LU MC NL PT SE TR**

(30) Priorité: **23.11.1999 FR 9914747**

(43) Date de publication de la demande:
**21.08.2002 Bulletin 2002/34**

(73) Titulaire: **CENTRE HOSPITALIER REGIONAL ET UNIVERSITAIRE DE LILLE
59037 Lille Cédex (FR)**

(72) Inventeurs:
- **ESNAULT, Jacques
F-45100 Orléans (FR)**
- **SINAY, Pierre
F-75009 Paris (FR)**
- **CHEVALIER, Reynald
75014 Paris (FR)**
- **COLOMBEL, Jean-Frédéric
F-59110 La Madeleine (FR)**
- **MALLET, Jean-Maurice
94400 Vitry sur Seine (FR)**
- **SENDID, Boualem
F-59120 Loos (FR)**
- **JOUAULT, Thierry
F-59650 Villeneuve d'Ascq (FR)**
- **POULAIN, Daniel
F-59242 Templeuve (FR)**
- **TRINEL, Pierre-André
F-59650 Villeneuve d'Ascq (FR)**

(74) Mandataire: **Breese, Pierre et al
Novagraaf Technologies
122, rue Edouard Vaillant
92593 Levallois-Perret Cedex (FR)**

(56) Documents cités:
- **YUASA H ET AL: "Synthesis of 5-thiomannose-containing oligomannoside mimics: binding abilities to concanavalin A" BIOORGANIC & MEDICINAL CHEMISTRY LETTERS,GB, OXFORD, vol. 8, no. 11, 2 juin 1998 (1998-06-02), pages 1297-1300, XP004137191 ISSN: 0960-894X**
- **TSURUTA O ET AL: "Syntheses of two trimannose analogs each containing C-mannosyl or 5-thio-C-mannosyl residue: their affinities to concanavalin A" BIOORGANIC & MEDICINAL CHEMISTRY LETTERS,GB,OXFORD, vol. 9, no. 6, 22 mars 1999 (1999-03-22), pages 807-810, XP004159607 ISSN: 0960-894X**
- **YUASA H ET AL: "Solid phase synthesis of oligomannopeptoids that mimic the concanavalin A-binding trimannoside" BIOORGANIC & MEDICINAL CHEMISTRY LETTERS,GB,OXFORD, vol. 8, no. 16, 18 août 1998 (1998-08-18), pages 2139-2144, XP004137234 ISSN: 0960-894X**
- **P-A.TRINEL ET AL.: "Mapping of Candida albicans Oligomannosidic Epitopes by Using Monoclonal Antibodies." INFECTION AND IMMUNITY, vol. 60, no. 9, septembre 1992 (1992-09), pages 3845-3851, XP000907030 cité dans la demande**
- **Y.HAN ET AL.: "A Vaccine and Monoclonal Antibodies That Enhance Mouse Resistance to candida albicans Vaginal Infection." INFECTION AND IMMUNITY, vol. 66, no. 12, décembre 1998 (1998-12), pages 5771-5776, XP000907028**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 232 167 B1

- M.YOUNG ET AL.: "Characterization of Oligosaccharides from an Antigenic Mannan of Saccharomyces cerevisiae." GLYOCCONJUGATE JOURNAL, vol. 15, 1998, pages 815-822, XP000907013
- M.X.ZHANG ET AL.: "Contrasting Roles of Mannan-Specific Monoclonal Immunoglobulin M Antibodies in the Activation of Classical and Alternative Pathways by Candida albicans." INFECTION AND IMMUNITY, vol. 66, no. 12, décembre 1998 (1998-12), pages 6027-6029, XP000907029
- B.SENDID ET AL.: "Specific Antibody Response to Oligomannosidic Epitopes in Crohn's Disease." CLINICAL AND DIAGNOSTIC LABORATORY IMMUNOLOGY, vol. 3, no. 2, mars 1996 (1996-03), pages 219-226, XP000910700
- P.JACQUINOT ET AL.: "Nature of Candida albicans-derived Carbohydrate Antigen Recognized by a Monoclonal Antibody in Patient Sera and Distribution over Candida species." FEMS MICROBIOLOGY LETTERS, vol. 169, 1 décembre 1998 (1998-12-01), pages 131-138, XP000910713
- S.SUZUKI: "Immunochemical Study on mannans of Genus Candida. I. Structural Investigation of Antigenic Factors 1,4,5,6,8,9,11,13,13b and 34." CURRENT TOPICS IN MEDICINAL MYCOLOGY, vol. 8, no. 1-2, 1997, pages 57-70, XP000911218
- DATABASE WPI Section Ch, Week 199103 Derwent Publications Ltd., London, GB; Class B03, AN 1984-086117 XP002138925 & JP 59 036690 A (RIKAGAKU KENKYUSHO), 28 février 1984 (1984-02-28)
- DATABASE WPI Section Ch, Week 198414 Derwent Publications Ltd., London, GB; Class B03, AN 1984-086118 XP002138926 & JP 59 036691 A (RIKAGAKU KENKYUSHO), 28 février 1984 (1984-02-28)
- DATABASE WPI Section Ch, Week 198516 Derwent Publications Ltd., London, GB; Class B03, AN 1985-096680 XP002138927 & JP 60 045589 A (RIKAGAKU KENKYUSHO), 12 mars 1985 (1985-03-12)
- DATABASE WPI Section Ch, Week 198224 Derwent Publications Ltd., London, GB; Class B04, AN 1982-48914E XP002138928 & JP 57 072997 A (RIKAGAKU KENKYUSHO), 7 mai 1982 (1982-05-07)
- DATABASE WPI Section Ch, Week 198516 Derwent Publications Ltd., London, GB; Class B03, AN 1985-096679 XP002138929 & JP 60 045588 A (RIKAGAKU KENKYUSHO), 12 mars 1985 (1985-03-12)
- DATABASE WPI Section Ch, Week 198516 Derwent Publications Ltd., London, GB; Class B03, AN 1985-096678 XP002138930 & JP 60 045587 A (RIKAGAKU KENKYUSHO), 12 mars 1985 (1985-03-12)
- DATABASE WPI Section Ch, Week 198219 Derwent Publications Ltd., London, GB; Class B02, AN 1982-37937E XP002138931 & JP 57 054199 A (RIKAGAKU KENKYUSHO), 31 mars 1982 (1982-03-31)
- DATABASE WPI Section Ch, Week 198224 Derwent Publications Ltd., London, GB; Class B04, AN 1982-48913E XP002138932 & JP 57 072996 A (RIKAGAKU KENKYUSHO), 7 mai 1982 (1982-05-07)
- CHANTAL F. ET AL.: "BETA-1,2-LINKED OLIGOMANNOSIDES INHIBIT CANDIDA ALBICANS BINDING TO MURINE MACROPHAGE" JOURNAL OF LEUKOCYTE BIOLOGY, vol. 60, July 1996 (1996-07), pages 81-87, XP009064245

## Description

La présente invention a pour objet des compositions comprenant des oligomannosides de synthèse, leur préparation, et leur utilisation pour la préparation d'un médicament et à la prévention d'infections.

Chez la levure opportuniste *Candida albicans,* comme chez tous les champignons, une partie considérable du métabolisme est dérivée vers la synthèse des polysaccharides pariétaux dont l'organisation module finement l'adaptation de la cellule au milieu. De nombreux groupes ont établi l'importance prépondérante du mannane de *C. albicans* dans la physiopathologie des candidoses. Il a ainsi été montré que les diverses interactions du mannane avec les composants humoraux et cellulaires de l'hôte reposent sur une spécificité dépendante des séquences oligomannosidiques synthétisées par la levure. C'est l'anomérie de liaison des résidus mannose et la longueur de la chaîne oligomannosidique qui détermine la nature de l'interaction qui conditionne l'issue de l'infection. Les travaux de recherche concernant les propriétés biologiques et la structure de ces oligomannosides, de même que bien entendu la mise au point de méthode diagnostic ou de prévention de ces infections, nécessitent de disposer de quantités importantes d'oligomannosides. Or, la production d'oligomannosides naturels à partir de souches de *C. albicans* est compliquée et coûteuse. Il s'agit bien entendu de disposer des souches et de les conserver, de les cultiver en fermenteur dans des conditions très standardisées car la nature des sucres dépend étroitement du milieu, de la température, de l'oxygénation, du temps de culture, etc.... La culture en fermenteur de *C. albicans* nécessite une grande expertise et de nombreuses précautions d'ordre microbiologique. Les lots de mannane doivent être ensuite récupérés de la culture et caractérisés pour leur propriétés antigéniques mais surtout chimiquement. Celle-ci nécessite une dépolymérisation du mannane et l'analyse en RMN des sucres libérés.

Ces oligomannosides naturels sont bien entendu utilisés pour la préparation de tests de diagnostic immunologique des infections par *C. albicans.* Or, la sensibilisation des plaques avec l'antigène mannane s'effectue avec des lots de production différents et selon un protocole qui ne permet aucun contrôle de la quantitée déposée. Ainsi, plusieurs tests utilisent des mannanes naturels de levures pour le diagnostic des candidoses (Sendid, B. et al., 1999, J. Clin. Microbiol.37(5):1510-7) et de la maladie de Crohn (Sendid, B. et al., 1996, Clin. Diagn. Lab. Immunol. 3(2):219: 26). Ces tests détectent des anticorps dirigés contre les mélanges de séquences oligomannosidiques présentes dans les mannanes de *C. albicans* et de *S. cerevisiae.*

Afin de palier les inconvénients indiqués ci-dessus, les Inventeurs sont maintenant parvenus à produire par synthèse chimique en grande quantité et de manière reproductible des analogues des oligomannosides des enveloppes cellulaires des levures, ci-après désignés oligomannosides de synthèse, permettant de remplacer avantageusement les mannanes naturels. En effet, les travaux de recherche réalisés avec ces oligomannosides de synthèse ont montré qu'ils miment les propriétés biologiques des sucres naturels de *C. albicans* notamment en ce qui concerne l'antigénicité et l'adhérence aux cellules et molécules de mammifère et la stimulation cellulaire. Ainsi, les Inventeurs ont mis en évidence que des oligomannosides de synthèse pouvaient être utilisés avec succès pour la sensibilisation de plaques de microtitration par couplage covalent en vue de la détection d'anticorps spécifiques de chaque structure dont la signification diagnostique et pronostique est différente (Jouault, T., 1997, Clin. Diagn. Lab. Immunol. 4(3):328-33).

En outre, les inventeurs ont mis en évidence que ces oligomannosides de synthèse présentaient des propriétés remarquables d'inhibition de la colonisation par *C. albicans* permettant de les utiliser pour la prévention et le traitement des candidoses.

La présente invention a donc pour objet une composition pharmaceutique comprenant au moins un oligomannoside produit par synthèse chimique homologue à un oligomannoside de la paroi d'un organisme infectieux ou pathogène. Plus particulièrement ledit organisme est une levure, un champignon, un virus, une bactérie dont l'enveloppe cellulaire contient des oligomannosides. Par enveloppe cellulaire, on entend aussi bien la membrane, la paroi, la capsule cellulaire. L'invention concerne tout particulièrement les levures, et spécifiquement de *Candida albicans* ou *Saccharomyces cerevisiae.*

On entend par homologue, le fait que les oligomannosides de synthèse de l'invention présentent les mêmes motifs de mannose selon le même enchaînement de liaisons a ou β, (1-2), (1-3), (1-6), que les oligomannosides naturels des levures, notamment de *Candida albicans* ou de *Saccharomyces cerevisiae,* mais sont dépourvus des autres composants cellulaires, notamment les protéines, les sucres, les lipides, qui sont inévitablement associés aux oligomannosides naturels décrits dans l'art antérieur.

On entend par dérivé d'un oligomannoside de synthèse de l'invention, un oligomannoside dans lequel un ou plusieurs des groupements fonctionnels sont substitués, par exemple par un groupe protecteur, ou encore des oligomannosides de synthèse conjugués à un groupement de liaison, aussi désigné connecteur, pour le greffage sur un support comme une plaque de microtitration.

L'invention se rapporte plus particulièrement à une composition pharmaceutique comprenant au moins un oligommanoside de synthèse présentant les mêmes motifs de mannose selon le même enchaînement de liaison α ou β, (1-2), (1-3), (1-6) que les oligomannosides naturels des levures ; et répondant à la formule générale suivante :

[Manα(1-3)]p[Manα(1-2)]q[Manβ(1-2)]r(α ou β)Man-OR

dans laquelle :

- R représente, un alkyle $C_{15}$ à $C_{20}$, un groupe connecteur, éventuellement marqué par exemple par un groupe chromophore ou fluorescent, ou encore, comme il sera décrit plus loin une substance capable de rendre l'oligommanoside de synthèse immunogène,
- p, q, et r sont des nombres entiers entre 0 et 19 et de préférence entre 0 et 11 et la somme de p+q+r est comprise entre 1 et 19 et de préférence entre 1 et 11,
- les trois parties du polymère [Manα(1-3)]p[Manα(1-2)]q[Manβ(1-2)]r peuvent être inversées ou répétées.

Parmi les compositions comprenant les oligomannosides de synthèse ci-dessus, l'invention se rapporte plus particulièrement aux compositions comprenant les tétramannosides, et de manière toute spécifique aux tétramannosides de synthèse suivants :

- D-Man β(1-2) D-Man β(1-2) D-Man β(1-2) D-Man, de *C. albicans,* également désigné ci-après Mβ-1-2-tétramannosides, répondant à la formule (I) suivante :

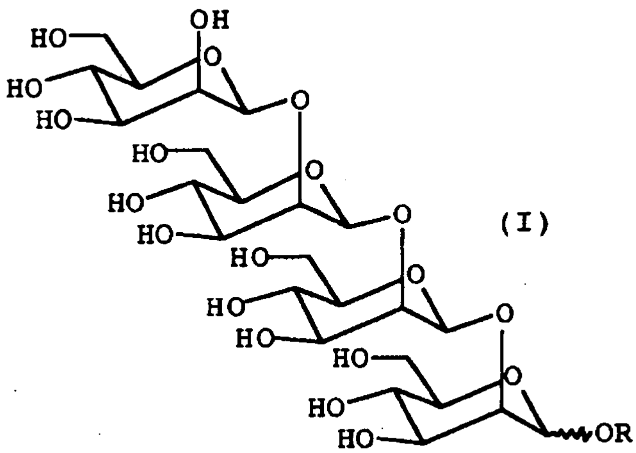

(I)

dans laquelle R représente un atome d'hydrogène, un alkyle en $C_{15}$ à $C_{20}$, ou un groupe connecteur, éventuellement marqué, ou encore une substance capable de rendre l'oligomannoside immunogène, ou un dérivé de celui-ci dont un ou plusieurs des groupes hydroxyles sont substitués.

- D-Man α(1-2) D-Man α(1-2) D-Man α(1-2) D-Man, de *C. albicans,* également désigné ci-après Mα-1-2-tétramannosides, répondant à la formule (II) suivante :

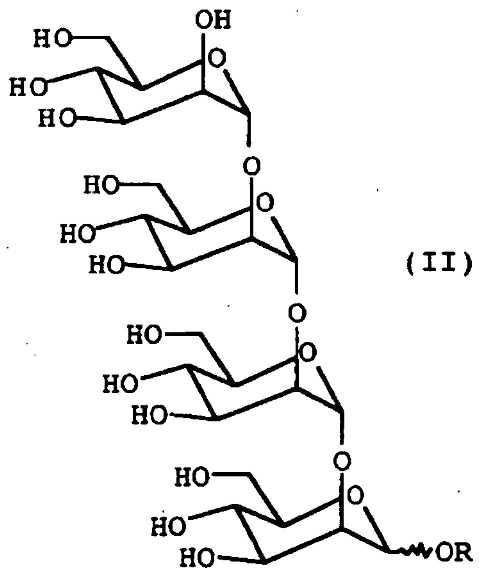

(II)

dans laquelle R à la même signification que dans la formule (I).

- D-Man α(1-3) D-Man α(1-2) D-Man α(1-2) D-Man α (1-2) de *S. cerevisiae,* répondant à la formule (III) suivante :

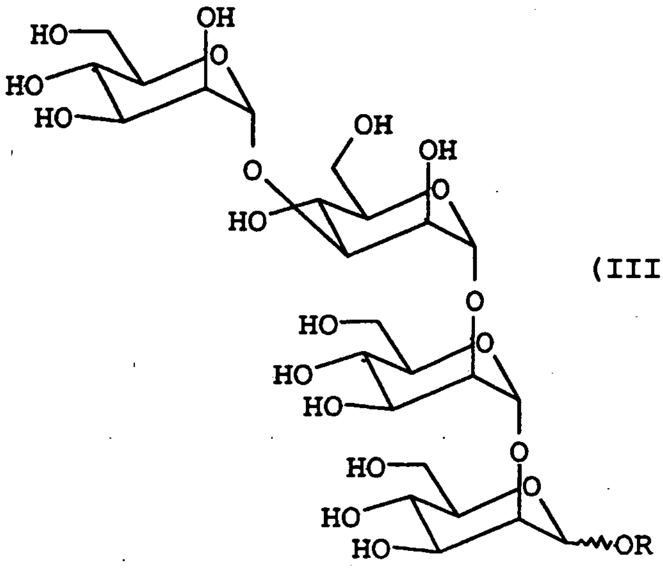

(III)

dans laquelle R à la même signification que dans la formule (I).

La présence d'un groupe R représentant un connecteur est utile pour la préparation de tests de détection de la présence chez un sujet d'anticorps spécifiques de certains oligomannosides pour le diagnostic d'une infection par *C. albicans* ou de maladies inflammatoires chroniques de l'intestin et notamment de la maladie de Crohn comme il sera décrit plus loin.

Le connecteur peut être tout groupe chimique permettant de coupler, de préférence par covalence mais aussi par d'autres liaisons par exemple de type hydrophobe, les oligomannosides de synthèse sur un support comme une plaque de microtitration. Le couplage covalent offre l'avantage d'utiliser une surface robuste et adaptée au test d'immunoanalyse, il permet une meilleure orientation des oligomannosides de synthèse et aussi de disposer d'une densité plus élevée d'épitopes et évite les problèmes de reconnaissance de l'anticorps car les sites antigéniques sont bien accessibles.

On préfère des connecteurs comportant une fonction acide carboxylique qui pourra être activée pour le couplage sur une surface elle-même activée pour réagir avec les oligomannosides de synthèse. A titre d'exemple de connecteur, on peut citer le connecteur de R. U. Lemieux (Lemieux, R.U. et al., 1975, J. Am. Chem. Soc. 97, 4076-4083), où R

représente un groupe de formule : $-CH_2-(CH_2)_7-CO_2H$.

Les connecteurs portant un groupe acide carboxylique, comme le connecteur de R. U. Lemieux peuvent être activés par un carbodiimide afin d'obtenir un ester activé qui permet la formation de liaisons amides avec des groupes amines primaires à la surface du support. La carbodiimide hydro-soluble (EDC : 1-ethyl-3-(3-dimethylaminopropyl)carbodiimide) est utilisée pour activer les groupements -COOH de l'oligomannoside de synthèse en présence de sulfo-N-hydroxy succinimide (sulfo-NHS : N-hydroxysuccinimidine). Le sulfo-NHS supprime de manière efficace l'hydrolyse du produit activé et permet la fixation de l'oligomannoside de synthèse à la surface de la plaque déjà sensibilisée par le groupement $-NH_2$.

Les oligomannosides de synthèse de l'invention peuvent être préparés par condensation de monosaccharides ou disaccharides protégés, de préférence, par condensation de dimannosides protégés selon une stratégie par dibloc.

Une forme de mise en oeuvre préférée d'une stratégie dibloc pour la préparation d'oligomannosides selon l'invention consiste à :

a) préparer des diblocs dont :

- l'un au moins des deux blocs est le bloc intermédiaire, où les fonctions hydroxyles libres de chaque monosaccharide sont substituées par un ou plusieurs groupes protecteurs identiques ou différents, sauf celle nécessaire à la condensation avec un autre dibloc qui est activée par un groupe partant,
- l'un des deux blocs est le bloc terminal, où les fonctions hydroxyles libres de chaque monosaccharide sont substituées par un ou plusieurs groupes protecteurs identiques ou différents, sauf celle nécessaire à la condensation avec un autre dibloc, et éventuellement celle substituée par un groupe de liaison pour la fixation de l'oligomannoside sur un support,

b) à condenser lesdits diblocs, puis à déproteger l'oligomannoside ainsi préparé.

Un exemple de dimannosides Manα(1-2)Man pour la mise en oeuvre du procédé ci-dessus répond à la formule (IV) suivante :

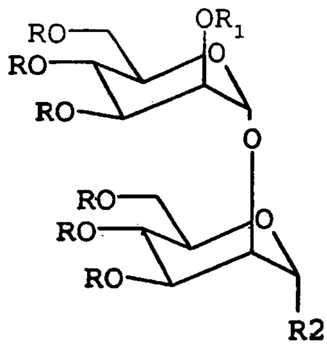

(IV)

dans laquelle :

- R est un groupe protecteur permanent. On entend par groupe protecteur permanent, un groupe protecteur qui est introduit en début de synthèse et retirer à la fin de la synthèse de l'oligamannoside. A titre d'exemple d'un tel groupe protecteur permanent, on peut citer un groupe benzyle.
- R1 est un groupe protecteur temporaire. On entend par groupe protecteur temporaire, un groupe protecteur qui est enlevé pour permettre la condensation. A titre d'exemple d'un tel groupe protecteur temporaire, on peut citer le groupe acétate.
- R2 est :
  - dans le cas d'un bloc intermédiaire, un groupe partant et dans ce cas le bloc peut être associé au reste du polymère en α ou β; à titre d'exemple d'un tel groupe partant, on peut citer $-O-C(NH)-CCl_3$ ou PhS,
  - dans le cas d'un bloc terminal, un groupe choisi parmi un groupe alkyle, benzyle, ou encore un connecteur, en α ou β.

Un exemple de dimannosides Manβ(1-2)Man pour la mise en oeuvre du procédé ci-dessus répond à la formule

(V) suivante :

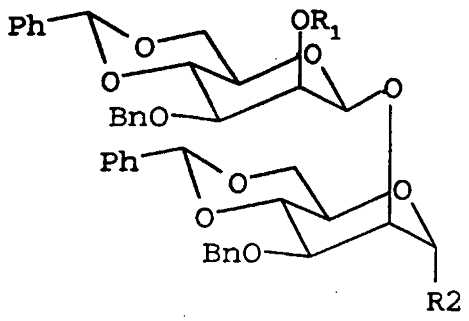

(V)

dans laquelle :

- R1 est un groupe protecteur temporaire; à titre d'exemple d'un tel groupe protecteur temporaire, on peut citer un groupe terbutyl diméthyl silyl
- R2 est :
  - dans le cas d'un bloc intermédiaire, un groupe partant et dans ce cas le bloc peut être associé au reste du polymère en α ou β; à titre d'exemple d'un tel groupe partant, on peut citer SPh ou SOPh;
  - dans le cas d'un bloc terminal, un groupe choisi parmi un groupe alkyle, benzyle, ou encore un connecteur, en α ou β.

Un exemple de dimannosides Manα(1-3)Man pour la mise en oeuvre du procédé ci-dessus répond à la formule (VI) suivante :

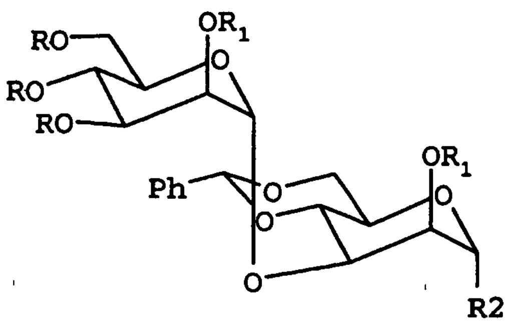

(VI

dans laquelle :

- R est un groupe protecteur permanent ; à titre d'exemple d'un tel groupe protecteur permanent, on peut citer le groupe benzyle,
- R1 est un groupe protecteur temporaire ; à titre d'exemple d'un tel groupe protecteur, on peut citer le groupe acétate,
- R2 est :
  - dans le cas d'un bloc intermédiaire, un groupe partant et dans ce cas le bloc peut être associé au reste du polymère en α; à titre d'exemple d'un tel groupe partant, on peut citer un groupe thiophényle (SPh),
  - dans le cas d'un bloc terminal, un groupe choisi parmi un groupe alkyle, benzyle, ou encore un connecteur, en α ou β.

Les dimannosides ci-dessus peuvent être utilisés pour la préparation de tetramannosides de l'invention.

Le tétramanoside D-Man β(1-2) D-Man β(1-2) D-Man β(1-2) D-Man de formule (I), peut être préparé à partir de deux blocs Manβ(1-2) de formule (V), dont l'un est un dibloc intermédiaire dans lequel R2 est un groupe partant, par exemple R2 = -SOPh, formant une liaison β, et l'autre un dibloc terminal dans lequel R2 par exemple un SPh.

Le tétramanoside D-Manα (1-2)D-Manα(1-2)D-Manα(1-2)D-Man, de formule (II) peut être préparé à partir de blocs Manα(1-2)Man de formule (IV), dont l'un est un dibloc intermédiaire dans lequel R2 est un groupe partant, par exemple R2 représente un groupe -C(NH)-$CCl_3$, et l'autre un dibloc terminal dans lequel R2 est -SPh.

Le tétramanoside D-Manα(1-3) D-Manα(1-2) D-Manα(1-2) D-Manα(1-2), de formule (III) peut être préparé à partir d'un dibloc Manα(1-3)Man de formule (VI) et d'un dibloc Manα(1-2)Man de formule (IV), le dibloc intermédiaire est Manα(1-3)Man de formule (VI) dans lequel R2 est un groupe partant, par exemple R2 est -SPh, et le dibloc terminal est Manα(1-2)Man dans lequel R2 représente le groupe R défini dans la formule (III).

Comme indiqué précédemment, les compositions comprenant au moins un oligomannoside de synthèse de l'invention sont utiles pour la détection *in vitro* sur un échantillon d'un patient de la présence d'une infection par un organisme infectieux ou pathogène, notamment une levure, un champignon, un virus, une bactérie dont l'enveloppe cellulaire contient des oligomannosides. Un tel procédé consiste à mettre en contact au moins une composition comprenant un oligomannoside de synthèse défini précédemment avantageusement préalablement fixé sur un support solide avec un échantillon biologique susceptible de contenir des anticorps dirigés contre l'organisme infectieux ou pathogène, puis la détection par tout moyen approprié d'un complexe antigène-anticorps. L'invention concerne plus particulièrement le diagnostic d'une infection par *C. albicans* et *S. cerevisiae,* ou dans le cas de la maladie de Crohn, la révélation d'anticorps anti- *S. cerevisiae.*

Ainsi, les inventeurs ont mis en évidence que les tetramannosides D-Manβ(1-2)D-Manβ(1-2)D-Manβ(1-2)D-Man de formule (I) et D-Manα(1-2)D-Man α(1-2)D-Manα(1-2)D-Man, de formule (II) permettent une détection spécifique d'une infection par *C. albicans,* donc le diagnostic des candidoses.

De même, les inventeurs ont mis en évidence que le tetramannoside D-Man α(1-3) D-Man α(1-2) D-Man α (1-2) D-Man α (1-2) de formule (III) permet une détection spécifique d'anticorps anti-*S. cerevisiae* et peut avantageusement mis en oeuvre dans des tests ASCA pour le diagnostic de la maladie de Crohn.

Les inventeurs ont encore montré que de façon surprenante le tetramannoside D-Man α(1-3) D-Man α(1-2) D-Man α(1-2) D-Man α (1-2) de formule (III) pouvait être utilisé dans le cadre des tests ASCA pour le diagnostic ou la prédiction des hépatites virales, de maladies autoimmunes ou encore de maladies inflammatoires.

L'invention a donc pour objet un procédé de détection d'anticorps anti-oligomannoside pour le diagnostic ou la prédiction d'une pathologie infectieuse et/ou inflammatoire, caractérisé en ce que l'on met en contact au moins un oligomannoside de synthèse décrit précédemment avec un échantillon biologique.

Plus particulièrement, l'invention se rapporte à un procédé de détection d'anticorps anti-*S. cerevisiae* pour le diagnostic de la maladie de Crohn ou d'une hépatite virale caractérisé en ce que l'on met en contact au moins un oligomannoside de synthèse décrit précédemment avec un échantillon biologique.

Avantageusement, l'oligomannoside de synthèse est le tetramannoside D-Man α(1-3) D-Man α(1-2) D-Man α (1-2) D-Man α (1-2) de formule (III).

La présente invention concerne également un procédé de préparation d'un support solide comprenant la synthèse d'un oligomannoside selon l'invention dans lequel R est un groupe connecteur, et le greffage dudit mannoside sur ledit support solide. La présente invention concerne également l'utilisation d'une composition pharmaceutique comprenant au moins un oligomannoside de synthèse présentant les mêmes motifs de mannose selon le même enchaînement de liaisons a ou b, (1-2), (1-3), (1-6) que les oligomannosides naturels des levures, et qui répond à la formule suivante :

[Manα(1-3)]p[Manα(1-2)]q[Manβ(1-2)]r(α ou β)Man-OR

dans laquelle :

- R représente un atome d'hydrogène, un alkyle en $C_1$ à $C_{20}$, un groupe connecteur, éventuellement marqué,
- p, q, et r sont des nombres entiers entre 0 et 19 et la somme de p+q+r est comprise entre 1 et 19,
- les trois parties du polymère [Manα (1-3)]p[Manα (1-2)]q[Manβ (1-2)]r peuvent être inversées ou répétées,

pour la préparation d'un médicament destiné à être utilisée pour inhiber la colonisation par les agents infectieux ou pathogènes dont les membranes contiennent des oligomannosides.

L'invention concerne encore un kit pour le diagnostic sur un échantillon biologique d'un patient d'une infection par un organisme infectieux ou pathogène, notamment une levure, un champignon, un virus, une bactérie dont l'enveloppe cellulaire continent des oligomannosides. Un tel kit comprend une composition comprenant au moins un oligomannoside de synthèse défini précédemment, avantageusement fixé sur un support solide, comme une plaque ELISA, des moyens pour détecter la formation de complexes antigène-anticorps, et éventuellement des réactif témoins.

Les travaux de recherche réalisés dans le cadre de l'invention ont permis de découvrir que les oligomannosides de synthèse définis précédemment peuvent être utilisés pour inhiber la colonisation par les agents infectieux ou pathogènes dont les membranes contiennent des oligomannosides. En effet, les levures du genre Candida utilisent des sucres de surface de leur paroi pour s'attacher aux cellules de leur hôte, cellules du vagin ou du tube digestif. Ainsi, les inventeurs sont parvenues à mettre en évidence que les sucres notamment de levures protègent des infections notamment celles déterminées par *C. albicans,* voire d'autres microbes exprimant les mêmes sucres par exemple LPS des *Salmonella, E. Coli)*

Plus particulièrement, les inventeurs ont montré, dans un modèle de candidose vaginale que le tetramannoside D-Manβ(1-2)D-Manβ(1-2)D-Manβ(1-2)D-Man de formule (I) réduisait la colonisation par *C. albicans* de manière très significative. De même le tetramannoside D-Manβ(1-2)D-Manβ(1-2)D-Manβ(1-2)D-Man de formule (I) s'est avéré très protecteur dans des modèles expérimentaux de colonisation digestive par *C. albicans.*

Des applications thérapeutiques des oligomannosides de synthèse décrits précédemment ou des conjugués formés d'un oligomannoside de synthèse couplé à une substance capable de rendre les sucres immunogènes, comme par exemple l'anatoxine tétanique, ainsi que les anticorps monoclonaux ou polyclonaux dirigés spécifiquement contre lesdits conjugués sont également décrites. Ces anticorps sont utiles comme outils de recherche mais aussi pour la mise au point d'outil de diagnostic.

L'invention concerne les compositions pharmaceutiques comprenant à titre d'agent actif au moins un oligomannoside de synthèse défini précédemment, conjugué ou non, associé dans la composition à un véhicule pharmaceutiquement acceptable. Ces compositions pharmaceutiques peuvent être appliquées localement ou généralement de façon à induire une inhibition de la colonisation ou une protection par immunisation locale ou générale selon le type d'infection observée.

L'invention envisage plus spécifiquement les conjugués formés à partir de D-Manβ(1-2)D-Manβ(1-2)D-Manβ (1-2)D-Man.

Ainsi, de façon surprenante, les inventeurs ont mis en évidence les propriétés inhibitrices des oligomannosides non conjugués, notamment dans les cas de colonisations intestinales par *C. albicans* et dans les cas candidoses vaginales.

D'autres avantages et caractéristiques de l'invention apparaîtront dans les exemples qui suivent concernant la préparation d'oligomannosides de l'invention et leur utilisation pour le diagnostic, la prévention et le traitement d'infections, et où il sera fait référence aux dessins en annexe dans lesquels:

La figure 1 représente le schéma réactionnel de la préparation de D-Man β(1-2) D-Man β(1-2) D-Man β(1-2) D-Man de formule (I).

La figure 2 représente le schéma réactionnel de la préparation de D-Man α(1-2) D-Man α(1-2) D-Man α(1-2) D-Man de formule (II).

La figure 3 représente le schéma réactionnel de la préparation de D-Man α(1-3) D-Man α(1-2) D-Man α(1-2) D-Man α (1-2) de formule (III).

La figure 4 représente le schéma réactionnel de la préparation de dimannosides Manβ(1-2)Man.

Les figures 5 et 6 rapportent les tests effectués respectivement avec le D-Man α(1-2) D-Man α(1-2) D-Man α (1-2) D-Man α (1-2) et le D-Man β(1-2) D-Man β(1-2) D-Man β(1-2) D-Man sur le facteur 5 spécifique des β-1,2 oligomannosides et le facteur 1 réagissant avec les α-1,2 oligomannosides vis à vis des mannotétraoses de synthèse d'anomérie α et β -1,2.

La figure 7 représente la réactivité du D-Man β (1-2) D-Man β (1-2) D-Man β (1-2) D-Man avec plusieurs anticorps monoclonaux.

La figure 8 représente la réactivité antigénique du mannotetraose de synthèse D-Man α(1-3) D-Man α(1-2) D-Man α(1-2) D-Man vis-à-vis du facteur antigénique 34.

La figure 9 représente la réactivité sérologique d'un pool de sérums de patients atteints de la maladie de Crohn vi-à-vis du mannotetraose de synthèse.

La figure 10 rapporte l'inhibition de la colonisation dans un modèle de candidose vaginale expérimentale de la rate par les tétramannosides de l'invention.

La figure 11 représente la comparaison de la colonisation digestive par la souche 10261 en fonction de l'administration préalable de sucre de synthèse. La colonisation digestive a été évaluée 7 jours après l'inoculation par la mesure des UFC/crotte pour des souriceaux ayant reçu de l'eau (Témoin), 50 μg (50β) ou 150 μg (150β) de βMan ou 150μg d'αMan (50α).

Exemple 1 : Préparation de D-Man β(1-2) D-Man β(1-2) D-Man β(1-2) D-Man de formule (I).

I - Schéma réactionnel.

La figure 1 en annexe représente le schéma réactionnel de la préparation de D-Man β(1-2) D-Man β(1-2) D-Man β(1-2) D-Man de formule (I).

- le composé de formule 1 (Stutz, A. et al., 1985, Carbohydr.Res. 137, 282-290) est préparé par réaction de PhCH$(OMe)_2$ sur le phényl 1-thio-α-D-mannopyranoside (Maity, S. K. et al., 1994, Tetrahedron, 50, 6965-6974) dans le DMF en présence de $HBF_4$ $Et_2O$. Une benzylation sélective donne le composé 2. Ce dernier est silylé et oxydé pour donner le composé 4.

La réaction (a) est réalisée dans les conditions suivantes : BnBr, $Bu_2SnO$, $NBu_4Br$, toluène, 110°C.
La réaction (b) est réalisée dans les conditions suivantes : TBDMSOTf, $Et_3N$, $CH_2Cl_2$.
La réaction (c) est réalisée dans les conditions suivantes : MCPBA, $CH_2Cl_2$, -40°C.
Les disaccharides et tétrasaccharides portant un thiophényl sont préparés tout d'abord en condensant les composés 2 et 4. La condensation entre 2 et 4 donne le disaccharide 5, celui ci peut être activé en sulfoxyde pour donner le composé 7 ou déprotéger en position 2' pour donner l'accepteur 6. La condensation des composés 6 et 7 donne le tétrasaccharide clé 8.
La réaction (d) est réalisée dans les conditions suivantes : $Tf_2O$, 2,6-diterbutyl-pyridine ou 2,6-diterbutyl-4-methyl pyridine (DBMP), $CH_2Cl_2$, -78°C.
La réaction (e) est réalisée dans les conditions suivantes : b) $NBu_4F.3H_2O$, THF.
La réaction (f) est réalisée dans les conditions suivantes : MCPBA, -40°C, $CH_2Cl_2$.
La réaction (g) est réalisée dans les conditions suivantes : $Tf_2O$, DBMP, $CH_2Cl_2$, -0°C.
Le 8-methoxycarbonyloctanol a été choisi comme connecteur. Ce dernier peut être préparé selon Lemieux (Lemieux, R. U. et al. 1975, J. Am. Chem. Soc., 97, 4076-4083) à partir de l'acide azélaique ou mieux par ozonolyse de l'oléate de méthyle et réduction in *situ* de l'aldéhyde formé par $NaBH_4$ (Gerlach, H. et al. 1978, Helv. Chim. Acta, 61, 1226-1231)
Ce composé 8 est ensuite transformé en 9 par élimination du silyle par NBu4NF, réaction avec l'eau en présence de NBS, puis en le tétramannoside de formule (I) dans laquelle R est H par déprotection des benzyles et benzylidènes.
La glycosylation du composé 8 avec le 8-methoxycarbonyloctanol donne le composé 10 qui donne après déprotection le tétramannoside de formule (I) dans laquelle R est $-(CH_2)_8-CO_2Me$.
La réaction (h) est réalisée dans les conditions suivantes : $NBu_4F.3H_2O$, THF; puis NBS, $H_2O$, acétone.
La réaction (i) est réalisée dans les conditions suivantes : NBS, TfOH, 8-methoxycarbonyloctanol, tamis moléculaire 4 Angstrom, $CH_2Cl_2$.
La réaction (j) est réalisée dans les conditions suivantes : $H_2$, Pd/C, MeOH, AcOEt.
La réaction (k) est réalisée dans les conditions suivantes : 1-$NBu_4NF.3H_2O$, THF; 2-$H_2$, Pd/C, MeOH, AcOEt, 3-NaOH, THF, $H_2O$.

II - Protocole expérimental.

1) Généralités.

Les points de fusion ont été mesurés sur un appareil capillaire Buchi 510 et n'ont pas été corrigés. Les pouvoirs rotatoires ont été mesurés à température ambiante à l'aide d'un polarimètre Perkin-Elmer 241. Les spectres de masse en mode d'ionisation chimique (ammoniac) ont été obtenus avec un spectromètre Nermag R10-10. Les analyses élémentaires ont été effectuées par le Service de Micro Analyse de l'Université Pierre et Marie Curie (Paris VI). Les spectres RMN du proton ont été enregistrés sur des appareil Bruker à 250 ou 400 MHz en solution dans le chloroforme deutérié. Les déplacements chimiques sont donnés en ppm par rapport au TMS et les abréviations utilisées sont les suivantes: s (singulet); se (singulet élargi); d (doublet); de (doublet élargi); t (triplet); q (quadruplet) et m (multiplet); by (benzylidène). Les chromatographies sur couches minces ont été réalisées sur plaques de silice Merck 60 $F_{254}$ et révélées par vaporisation d'une solution alcoolique d'acide sulfurique concentré (20% v/v) et chauffage. Les chromatographies sur colonne (flash) ont été faites sur gel de silice 60 (230-400 mesh, Merck).

1) Phenyl 3-O-benzyl-4,6-O-benzylidene-1-thio-α-D-mannopyranoside (2).

Ce composé est décrit dans la littérature: Z Szurmai, L Balatoni, A.Liptak, Carbohydr. Res. 254 (1994) 301-309 et T Oshitari, S Kobayashi, Tetrahedron Lett (1995) 1089-92. La description ci-après rapporte une synthèse utilisable

sur de grandes quantités (avec deux variantes correspondant à deux solvants différents).

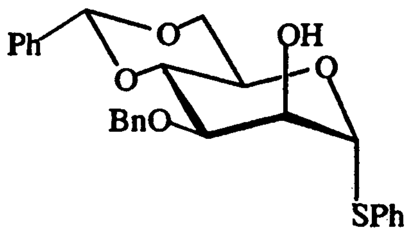

- Méthode 1 (le solvant est le toluène):

A une solution de 1 (composé connu : H Franzyk, M Medal, H Paulsen, K Bock J. Chem Soc. Perkin Trans I, (1995) 2883-98 avantageusement préparé selon R. Albert, K Dax, R. Pleschko, A Stütz, Carbohydr. Res. 137 (1985) 282-290) (10g, 28 mmol) dans 295 mL de toluène anhydre, on ajoute 7.59g d'oxyde de dibutylétain. Le mélange est porté à reflux du toluène avec un appareil de Dean Stark pendant une nuit. De l'iodure de tétrabutylammonium (6.59g), puis du bromure de benzyle (4.3mL) sont ajoutés. L'agitation est maintenue 3h sous reflux, puis on ramène à température ambiante (contrôle cyclohexane/acétate d'éthyle=3/1)) et concentre sous vide le mélange. On obtient un résidu, qui est purifié par chromatographie sur colonne (élution:cyclohexane/acétate d' éthyle=4/1) pour donner le produit 2 (11g, 89%).

- Méthode 2 (le solvant est l'acétonitrile):

On introduit dans un ballon, le composé 1 (9.05 g, 25.11 mmol) et 450 mL d'acétonitrile anhydre. On chauffe au reflux du $CH_3CN$ sous argon (le composé 1 se dissout dans l'acétonitrile à chaud). Quand la solution est limpide, on ajoute 23.53 g de tamis 4 Å en poudre, 7.51 g de $Bu_2SnO$. On laisse au reflux pendant 6 heures. On laisse revenir à température ambiante. On ajoute 8.10 g de $nBu_4N^+Br^-$ (1 éq), 6.3 mL de bromure de benzyle (2.3 éq) et agite pendant la nuit à 45°C. On filtre à travers un entonnoir fritté recouvert de célite, on rince les solides avec du dichlorométhane et concentre sous vide. On obtient un résidu, qui est purifié par chromatographie sur colonne (élution:cyclohexane/acétate d'éthyle= 3/1) pour donner le produit 2 (10.2 g, 90%)

$^1$H-RMN : (250 MHz, $CDCl_3$) : δ:5.55 (s, 1H, By); 5.52 (s, 1H, H-1); 4.82 (d, 1H, $J$gem=11.79 Hz, CHPh) ; 4.67 (d, 1H, $J_{gem}$=11.75 Hz, CHPh); 4.27 (ddd, 1H, H-5); 4.21 (d, 1H, $J_{2-3}$=3.54 Hz, H-2); 4.14 (dd, 1H, $J_{6a-6b}$=9.73 Hz et $J_{6a-5}$=4.80 Hz, H-6a); 4.11 (t, 1H, H-4); 3.89 (dd, 1H, $J_{3-4}$=9.50 Hz et $J_{3-2}$=3.37 Hz, H-3); 3.78 (t, 1H, $J_{6b-5}$=10.18 Hz et $J_{6b-6a}$=10.18 Hz, H-6b); 2.79 (s, 1H, O-H).

2) Phenyl 2-O-tertbutyldimethylsilyl-3-O-benzyl-4,6-benzylidene-1-thio-α-D- mannopyranoside (3).

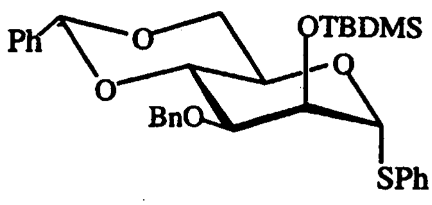

On introduit dans un ballon, sous atmosphère d'argon, le composé 2 (5.51 g, 12.23 mmol) et 55 mL de dichlorométhane anhydre. On ajoute sous agitation: 5.3 mL de triéthylamine (3.1 éq), 4.65 mL de triflate de terbutyldiméthylsilyle (1.65 éq). On agite pendant une nuit à température ambiante. On contrôle l'état d'avancement de la réaction par CCM (élution:cyclohexane/acétate d'éthyle=3/1). On neutralise avec une solution aqueuse de $NaHCO_3$, La phase organique est concentrée sous vide pour donner un résidu, qui est purifié par chromatographie sur colonne (élution:cyclohexane/acétate d'éthyle=96/4), pour donner le produit 3 (6.2 g, 90%). Rf:(cyclohexane/AcOEt= 96/4): 0.43. $[\alpha]_D$ = +122 (c 1,2; $CHCl_3$)

$^1$H-RMN (250 MHz,$CDCl_3$) :δ :5.55 (s, 1H, By) ; 5.24 (d, 1H, $J_{1-2}$=1.4 Hz, H-1); 4.69 (dd, 2H, CHPh); 4.26-4.08 (massif, 4H); 3.83-3.65 (massif, 2H); 0.81 (s, 9H, Si-tBu) ; 0.00 (s, 3H, Si-Me) ; -0.4 (s, 3H, Si-Me).

Analyse pour $C_{32}H_{40}O_5SSi$ (564.821) : calculée C:68.04 H:7.14 trouvée C:68.15 H:7.27

3) Phenyl (2-O-tertbutyldimethylsilyl- 3-O-benzyl- 4,6-O-benzylidene- α-D- mannopyranosyl) sulfoxide (4).

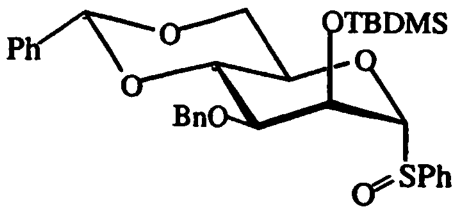

On introduit dans un ballon, sous atmosphère d'argon, le composé 3 (6.2 g, 10.97 mmol) et 125 mL de dichlorométhane anhydre. Dans le ballon refroidi à -78 °C, on ajoute de l'acide 3-chloro perbenzoique (12.06 mmol) en solution dans 26 mL de dichlorométhane. On maintient la température à -78° C pendant 15 minutes puis laisse revenir doucement à -30°C. On ajoute $(CH_3)_2S$ pour éliminer l'excès de peracide. On neutralise avec une solution saturée de $NaHCO_3$, lave avec une solution saturée de NaCl, extrait les phases aqueuses avec du dichlorométhane. La phase organique est séchée ($MgSO_4$), filtrée puis concentrée. On obtient un résidu, qui est purifié par chromatographie sur colonne (élution: cyclohexane/acétate d'éthyle=5/1), pour donner 4 (5.04g, 79% en diastéréoisomère majoritaire). Lors de la chromatographie on récupére l'autre diastéréoisomère en proportion très minoritaire(<5%).

Caracteristiques de l'isomère majoritaire : $[\alpha]_D$ = -67 (c 1,0; $CHCl_3$)

$^1$H-RMN :(400 MHz,$CDCl_3$) :δ7,70-7,20(m, 15 H, arom); 5.69 ( s, 1H, By); 4.93 et 4;82 ( 2d, 2H, CHPh); 4.73 (dd, 1H, $J_{1,2}$=1,2, $J_{2,3}$ 2.3Hz, H-2); 4.39 (d, 1H, H-1); 4.30 (dd, 1H, $J_{3,4}$=9,9; $J_{4,5}$ 9,9Hz; H-4); 4.28 (dd, 1H, H-3); 4.26 (dd, 1H, $J_{6a,6b}$ 10.3; $J_{6b,5}$=5,8Hz, H-6b); 4.14 ( ddd, 1H, J5,6a=120,1Hz, H-5); 3.79(dd, 1H, H-6a); 0.88 (s, 9H, tBu); 0.072 et -0.046 ( 2s, 6H, MeSi);

Spectre de masse : m/z 598 $(M+NH_4)^+$.

Analyse pour $C_{32}H_{40}O_6SSi$ (564.821) : calculée C: 66.17 H:6.94 trouvée C:66.30 H:7.06

4) Phényl 2-O-(3-O-benzyl- 4,6-O-benzylidène-2-O-tertbutyldiméthylsilyl-β-D-mannopyranosyl)- 3-O-benzyl-4,6-O-benzylidène-1-thio-α-D- mannopyranoside (5).

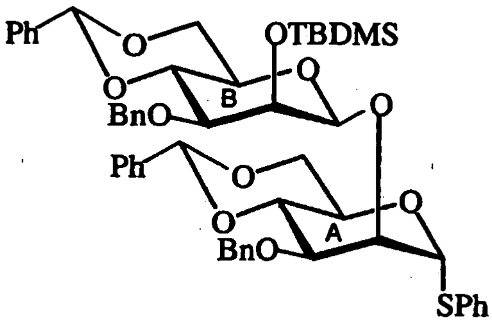

7.1 g (12.22 mmol, 1 éq) de 4 et 9.06 mL (40.3 mmol, 3.3 éq) de 2,6-ditertbutylpyridine sont dissous dans 200 mL de dichlorométhane anhydre et sont placés sous atmosphère d'argon. Après avoir refroidi la solution à -78°C, 2.27 mL (13.44 mmol, 1.1 éq) d'anhydride de l'acide trifluorométhanesulfonique sont ajoutés. Après 5 mn d'agitation à -78°C, 11 g (24.4 mmol, 2 éq) de 2 préalablement dilués dans 100mL de dichlorométhane anhydre sont additionnés goutte à goutte. La température est maintenue 1 heure à -78°C et laissée lentement remonter à 0°C. Le milieu reactionnel est alors neutralisé par une solution d'hydrogénocarbonate de sodium. La phase organique est ensuite lavée par une solution aqueuse de NaCl, séchée sur sulfate de magnésium, filtrée et évaporée sous vide. Une chromatographie sur gel de silice (élution:cyclohexane/acétate d'éthyle=96/4) du brut permet d'obtenir 5 (7.97 g, 72%) sous la forme d'une mousse blanche. pf : 81-83°C(hexane); $[\alpha]_D$ +12.5 (c 1.02, chloroforme)

$^1$H-R.M.N. (400MHz, $CDCl_3$) : δ 7.58-7.34m( 25H, arom.),5.66 et 5.65 (2s, 2H, by), 5.56 (d, 1H, $J_{1-2}$=1Hz, H-1A), 4.85 (2d, 2H, $J_{gem}$=12Hz, CHPh), 4.8 (d, 1H, $J_{gem}$=12Hz, CHPh), 4.75 (d, 1H, $J_{gem}$=12Hz, CHPh), 4.53 (dd, 1H,

$J_{2-1}$=1Hz et $J_{2-3}$=3 .3Hz, H-2A), 4.52 (se, 1H, H-1B), 4.45-4.4 (m, 1H, H-5A), 4.37 (t, 1H, $J_{4-3}$=$J_{4-5}$=9.5Hz, H-4A), 4.31 (dd, 1H, $J_{6b-6a}$=10Hz et $J_{6b-5}$=4.3Hz, H-6Ab), 4.26 (dd, 1H, $J_{6b-6a}$=10.3Hz et $J_{6b-5}$=4.8Hz, H-6Bb), 4.21 (t, 1H, $J_{4-3}$=$J_{4-5}$=9.5Hz, H-4B), 4.19 (de, 1H, $J_{2-3}$=2.7Hz, H-2B), 4 (dd, 1H, $J_{3-2}$=2.7Hz et $J_{3-4}$=9.5Hz, H-3A), 3.91 (t, 1H, $J_{6a-6b}$=$J_{6a-5}$=10Hz, H-6Aa), 3.84 (t, 1H, $J_{6a-6b}$=$J_{6a-5}$=10.2Hz, H-6Ba), 3.59 (dd, 1H, $J_{3-2}$=2.7Hz et $J_{3-4}$=9.5Hz, H-3B), 3.35 (dt, 1H, $J_{5-4}$=$J_{5-6a}$=10.2Hz et $J_{5-6}$=4.8Hz, H-5B), 1.0 (s, 9H, $SiC(CH_3)_3$), 0,30 et 0,23 (2s, 6H, $Si(CH_3)_2$).

$^{13}$C R.M.N. (100 MHz) : δ 138.55, 138.3, 137.5, 137.4, 133.6 (5 C arom.), 131.8-126.06 (25 CH arom), 101.6 (CH by), 101.4 (CH by), 99.8 ($^1J_{CH}$=157Hz, C-1B), 86.7 ($^1J_{CH}$=166Hz, C-1A), 78.7 (C-4B), 78.2 (C-4A), 77.5 (C-3B), 76.4 (C-2A), 74 (C-3A), 72.2 ($CH_2Ph$), 71.7 (C-2B), 70.7 ($CH_2Ph$), 68.5 (C-6B), 68.47 (C-6A), 67.7 (C-5B), 65.2 (C-5A), 26 ($C(CH_3)_3$), 18.5 ($C(CH_3)_3$), -4 ($SiCH_3$), -4.5 ($SiCH_3$).

Spectre de masse : m/z 922 (M+NH4)$^+$.

Analyse pour $C_{52}H_{60}O_{10}SSi$ (905.199) : calculée C:68.99 H:6.68 trouvée C:68.82 H:6.69

5) Phényl 2-O-(3-O-benzyl-4,6-O-benzylidène-β-D-mannopyranosyl)-3-O-benzyl-4,6-O-benzylidène-1-thio-α-D-mannopyranoside (6).

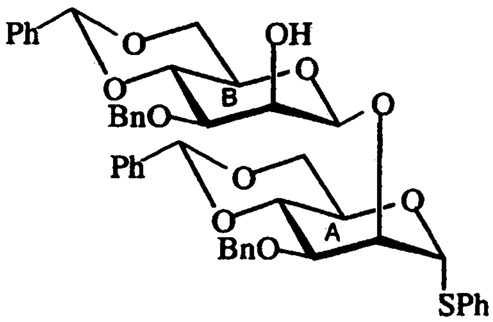

3.8 g (4.2 mmol, 1 éq) du produit 5 et 6.6 g (21 mmol, 5 éq) de fluorure de tetrabutylammonium trihydrate sont dissous dans 60 mL de tetrahydrofurane. Après 1 heure à température ambiante, le milieu est dilué par du dichlorométhane et la phase organique est lavé par de l'eau. La phase organique est séchée sur sulfate de magnésium, filtrée et évaporée sous pression réduite. Une chromatographie sur gel de silice (élution:cyclohexane/acétate d'éthyle=3/1) fournit 6 (3.0 g, 90%) sous forme d'une poudre blanche. pf : 79-81°C(hexane); $[\alpha]_D$ +30 (c 0.33, chloroforme)

$^1$H-R.M.N. (400MHz, $CDCl_3$) : δ 7.54-7.30m(, 25H, arom.), 5.59 et 5.51 (2s, 2H, by), 5.54 (d, 1H, $J_{1-2}$=1Hz, H-1A), 4.89 (d, 1H, $J_{gem}$=12.2Hz, CHPh), 4.86 (d, 1H, $J_{gem}$=11.8Hz, CHPh), 4.82 (d, 1H, $J_{gem}$=11.8Hz, CHPh), 4.8 (d, 1H, $J_{gem}$=12.2Hz, CHPh), 4.77 (d, 1H, $J_{1-2}$=0.8Hz, H-1B), 4.66 (dd, 1H, $J_{2-1}$=1Hz et $J_{2-3}$=3.3Hz, H-2A), 4.33 (dt, 1H, $J_{5-4}$=9.7Hz et $J_{5-6a}$=$J$ 5-6b=4-9Hz, H-5A), 4.32 (t, 1H, $J_{4-3}$= $J_{4-5}$=9.3Hz, H-4B), 4.26 (dd, 1H, $J_{6b-6a}$=10.5Hz et $J_{6b-5}$=4.9Hz, H-6Bb), 4.3 (dd, 1H, $J_{6b-6a}$=10Hz et $J_{6b-5}$=5Hz, H-6Ab), 4.24 (t, 1H, $J_{4-3}$=$J_{4-5}$=9.7Hz, H-4A), 4.19 (de, 1H, $J_{2-3}$=3.9Hz, H-2B), 4.04 (dd, 1H, $J_{3-2}$=3.3Hz et $J_{3-4}$=9.7Hz, H-3A), 3.8 (m, 2H, H-6Aa et H-6Ba), 3.72 (dd, 1H, $J_{3-2}$=3.9Hz et $J_{3-4}$=9.2Hz, H-3B), 3.43 (dt, 1H, $J$ 5-4=$J_{5-6a}$=9.5Hz et $J_{5-6b}$=4.9Hz, H-5B), 3.2 (br, 1H, OH).

$^{13}$C R.M.N. (100 MHz) : δ 138, 137.8, 137.3, 137.29, 131.7 (5 C arom.), 129.2-127.7 (25 CH arom), 101.4 (CH by), 101.2 (CH by), 97.4 ($^1J_{CH}$=163Hz, C-1B), 86.4 ($^1J_{CH}$=167Hz, C-1A) , 78.5 (C-4A) , 78.4 (C-4B) , 76.1 (C-3B) , 74.4 (C-3A), 74.35 (C-2A), 72.4 ($CH_2Ph$), 72.3 ($CH_2Ph$), 69.4 (C-2B), 68.5 (C-6B), 68.25 (C-6A), 66.8 (C-5B), 65.2 (C-5A) .

Spectre de masse : m/z 808 (M+NH4)$^+$.

Analyse pour $C_{46}H_{46}O_{10}S$ (790.93) : calculée C:69.85 H:5.86 trouvée C:69.76 H:6.01

6) Phénol [2-O-(3-O-benzyl-4,6-O-benzylidène-2-O-tertbutyldiméthylsilyl-β-D-mannopyranosyl)-3-O-benzyl-4,6-O-benzylidène α-D-mannopyranosyl] sulfoxyde (7).

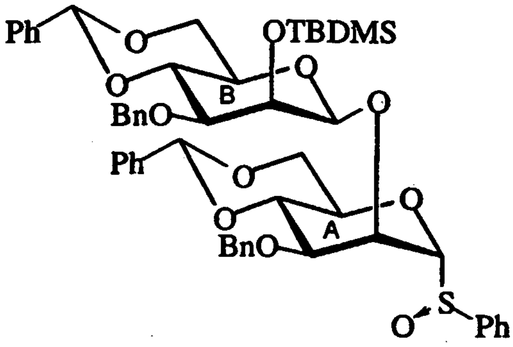

3.0 g (3.31mmol, 1 éq) de 5 sont dissous dans 40 mL de dichlorométhane anhydre. Après avoir refroidi le milieu reactionnel à -78°C, 0.97 g (5.5 mmol, 1.7 éq) d'acide 3-chloroperoxybenzoïque à 85 % préalablement dissous dans 15mL de dichlorométhane sont ajoutés à l'aide d'une canule. Après 15 mn d'agitation à -78°C, la température est laissée remonter à -30°C et quelques gouttes de diméthylsulfure sont additionnées. La phase organique est alors lavée par une solution d'hydrogénocarbonate de sodium, une solution aqueuse de NaCl, séchée ($MgSO_4$), filtrée et évaporée sous vide. Une chromatographie sur gel de silice (élution :cyclohexane/acétate d'éthyle=4/1, puis 3/1) du brut donne 2.8 g (91%) d'un premier diastéréomère 7 sous forme d'une mousse blanche et 71 mg (2%) d'un deuxième diastéréomère sous forme également d'une mousse blanche.

Caractéristiques du diastéréomère majoritaire pf : 89-91°C(hexane) ; $[\alpha]_D$ -99 (c 1, chloroforme)

$^1$H-R.M.N. (400MHz, $CDCl_3$) : δ 7.65-7.4m( 25H, arom.),5.63 et 5.62 (2s, 2H, by), 4.89 (1d, 1H, $J_{gem}$=12Hz, CHPh), 4.85 (d, 1H, $J_{gem}$=12.4Hz, CHPh), 4.81 (dd, 1H, $J_{2-1}$=1.1Hz et $J_{2-3}$=3.4Hz, H-2A), 4.78 (d, 1H, $J_{gem}$=12.4Hz, CHPh), 4.76 (1d, 1H, $J_{gem}$=12Hz, CHPh), 4.42 (d, 1H, $J_{1-2}$=1.1Hz, H-1A), 4.37 (t, 1H, $J_{4-3}$=$J_{4-5}$=10.1Hz, H-4A), 4.33 (dd, 1H, $J_{3-2}$=3.4Hz et $J_{3-4}$=10.1Hz, H-3A), 4.28 (dd, 1H, $J_{6b-6a}$=10.2Hz et $J_{6b-5}$=4.8Hz, H-6Ab), 4.26 (se, 1H, H-1B), 4.18-4.10 (m, 3H, H-5A, H-6Bb et H-4B), 4.08 (de, 1H, $J_{2-3}$=2.8Hz, H-2B), 3.75 (t, 1H, $J_{6a-6b}$=$J_{6a-5}$=10.1Hz, H-6Aa), 3.74 (dd, 1H, $J_{6b-6a}$=$J_{6b-5}$=10.2Hz, H-6Ba), 3.52 (dd, 1H, $J_{3-2}$=2.8Hz et $J_{3-4}$=9.7Hz, H-3B), 3.18 (dt, 1H, $J_{5-4}$=$J_{5-6a}$=9.8Hz et $J_{5-6}$=4.8Hz, H-5B), 1 (s, 9H, $SiC(CH_3)_3$), 0.22 et 0.17 (2s, 6H, $Si(CH_3)_2$).

$^{13}$C R.M.N. (100 MHz) : δ 141.3, 138.4, 138.3, 137.5, 137 (5 C arom.), 131.8-124.3 (25 CH arom), 101.7 (CH by), 101.4 (CH by), 99.8 ($^1J_{CH}$=156HZ, C-1B), 97.5 ($^1J_{CH}$=163Hz, C-1A) , 78.7 (C-4B) , 77.5 (C-3B) , 77.1 (C-4A), 74.2 (C-3A), 72.4 ($CH_2Ph$), 71.7 (C-2B), 71.4 (C-2A), 70.9 ($CH_2Ph$), 70.1 (C-5A), 68.4 (C-6B), 68.1 (C-6A), 67.5 (C-5B), 26 ($C(CH_3)_3$), 18.5 ($C(CH_3)_3$), -4 ($SiCH_3$), -4.5 ($SiCH_3$).

Analyse pour $C_{46}H_{46}O_{10}S$ (921.198) : calculée C:67.8 H:6.56 trouvée C:67.68 H:6.90

7) Phényl 2-O-(2-O-(2-O-(3-O-benzyl-4,6-O-benzylidène-2-O-tertbutyldiméthyl silyl-β-D-mannopyranosyl) -3-O-benzyl-4,6-O-benzvlidéne-β-D-mannopyranosyl)-3-O-benzyl-4,6-O-benzylidène-β-D-mannopyranosyl)-3-O-benzyl-4,6-O-benzylidène-1-thio-a-D-mannopyranoside (8).

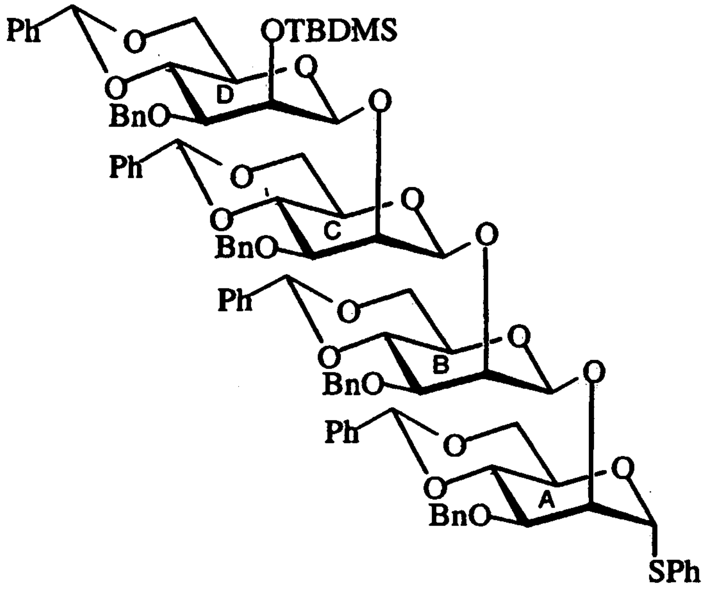

Le protocole expérimental est le même que celui de la synthèse du disaccharide 5. On obtient à partir de 6 (1.140g, 2éq) et 7 (0.664g, 1éq) le composé 8 (580 mg, 55%) d'une mousse blanche après une chromatographie sur gel de silice (élution:cyclohexane/acétate d'éthyle=85/15). pf : 111-114°C (hexane); $[\alpha]_D$ -53.5 (c 0.6, chloroforme)

$^1$H-R.M.N. (400MHz, $CDCl_3$) : δ 7.5-7.15m(45H, arom.),5.615*2, 5.61 et 5.6 (4s, 4H, by), 5.53 (d, 1H, $J_{1-2}$=1.1Hz, H-1A), 5.35 (se, 1H, H-1B), 5.03 (se, 1H, H-1C), 4.82 (d, 1H, $J_{gem}$=12.8Hz, CHPh) , 4.76 (s, 2H, $CH_2$Ph), 4.73 (d, 1H, $J_{gem}$=12.8Hz, CHPh), 4.72 (se, 1H, H-1D), 4.72 (d, 1H, $J_{gem}$=11.6Hz, CHPh) , 4.67 (d, 1H, $J_{gem}$=17.7Hz, CHPh) , 4.57 (d, 1H, $J_{gem}$=11.6Hz, CHPh), 4.58-4.57 (m, 1H, H-2A), 4.57 (de, 1H, $J_{2-3}$=3.2Hz, H-2B), 4.57 (2de, 2H, $J_{2-3}$=3.2Hz, H-2C et H-2D), 4.42-4.37 (m, 1H, H-5A), 4.39-4.35 (m, 2H, H-6Db et H-6Bb), 4.38 (d, 1H, $J_{gem}$=11.7Hz, CHPh) , 4.29 (dd, 1H, $J_{6b-6a}$=10.2 et $J_{6b-5}$=4.8Hz, H-6Ab), 4.24 (dd, 1H, $J_{4-3}$=10 et $J_{4-5}$=9.6Hz, H-4B), 4.16 (t, 1H, $J_{4-3}$=$J_{4-5}$=9.7Hz, H-4C), 4.13 (dd, 1H, $J_{6b-6a}$=10.2 et $J_{6b-5}$=4.7Hz, H-6Cb), 4.05 (t, 1H, $J_{4-3}$=$J_{4-5}$=10Hz, H-4A), 4.02 (t, 1H, $J_{4-3}$=$J_{4-5}$=10Hz, H-4D), 4.05-4.02 (m, 1H, H-3A), 4 (t, 1H, $J_{6a-6b}$=$J_{6a-5}$=10.3Hz, H-6Ba), 3.89 (t, 1H, $J_{6a-6b}$= $J_{6a-5}$=10.2Hz, H-6Da), 3.8 (t, 1H, $J_{6a-6b}$=$J_{6a-5}$=10.2Hz, H-6Aa), 3.79 (t, 1H, $J_{6a-6b}$=$J_{6a-5}$=10.2Hz, H-6Ca), 3.69 (dd, 1H, $J_{3-2}$=3.2Hz et $J_{3-4}$=9.9Hz, H-3D), 3.58 (dd, 1H, $J_{3-2}$=2.7Hz et $J_{3-4}$=9.7Hz, H-3C), 3.55 (dd, 1H, $J_{3-2}$=3.2Hz et $J_{3-4}$=10Hz, H-3B), 3.49 (ddd, 1H, $J_{5-4}$=9.6Hz et $J_{5-6b}$=10.3Hz et $J_{5-6a}$=4.8Hz, H-5B), 3.42 (ddd, 1H, $J_{5-4}$=9.8Hz et $J_{5-6b}$=4.8Hz et $J_{5-6a}$=10.2Hz, H-5D), 3.31 (ddd, 1H, $J_{5-4}$=9.7Hz et $J_{5-6b}$=4.75Hz et $J_{5-6a}$=10.1Hz, H-5D), 0.95 (s, 9H, $SiC(CH_3)_3$), 0.25 et 0.14 (2s, 6H, $Si(CH_3)_2$).

$^{13}$C R.M.N. (100 MHz) : δ 138.6, 138.4, 138.1, 138, 137.7, 137.4, 136.95, 136.93, 133.2 (9 C arom.), 131.65-126 (45 CH arom), 103.1 ($^1J_{CH}$=159Hz, C-1C), 102.05 (CH by), 102.02 (CH by), 101.6 (CH by), 101.2 (CH by), 101.19 ($^1J_{CH}$=158.5Hz, C-1B), 99 ($^1J_{CH}$=167Hz, C-1A), 85.4 ($^1J_{CH}$=159.2Hz, C-1D), 79.7 (C-3C), 79.05 (C-4C), 78.9 (C-4A), 78.5 (C-4D), 77.6 (C-4B), 76.9 (C-3B), 75.8 (C-3D), 75.3 (C-2B), 75.2 (C-2A), 74.4 (C-3A), 73.1 (C-2C ou C-2D), 72.8 ($CH_2$Ph) , 71.9 ($CH_2$Ph) , 71.2 (C-2C ou C-2D), 70.9 ($CH_2$Ph) , 70.1 ($CH_2$Ph) , 68. 8 (C-6A) , 68.7 (C-6D et C-6B), 68.5 (C-6C), 68.1 (C-5B), 67.8 (C-5C), 67.7 (C-5D), 65.1 (C-5A) , 26.1 ($C(CH_3)_3$), 18.5 ($C(CH_3)_3$), -3.7 ($SiCH_3$), -4.7 (SiCH3).

Spectre de masse : m/z 1602 (M+NH4)$^+$.

Analyse pour $C_{92}H_{100}O_{20}SSi$ (1585.956) : calculée C:69.67 H:6.35 trouvée C:69.57 H:6.41

8) 8-méthoxycarbonyloctyl 2-O-(2-O-(2-O-(3-O-benzyl-4,6-O-benzylidène-2-O-tertbutyldiméthyl silyl-β-D-mannopyranosyl) -3-O-benzyl-4,6-O-benzylidène-β-D-mannopyranosyl)-3-O-benzyl-4,6-O-benzylidéne-β-D-mannopyranosyl)-3-O-benzyl-4,5-O-benzylidène-D-mannopyranoside (10).

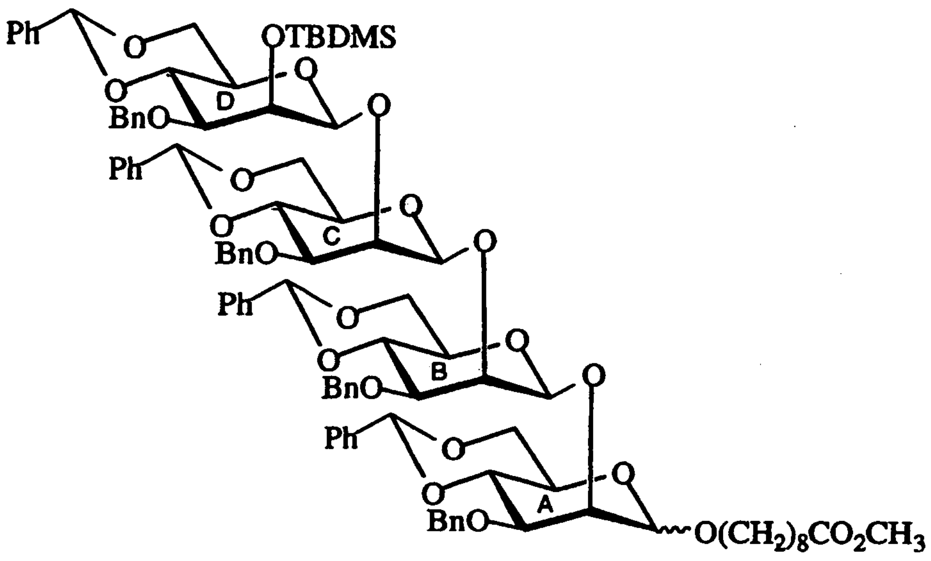

562 mg (354 μmol, 1 éq) de 8, 166 mg (885 μmol, 2.5 éq) du méthoxycarbonyloctan-1-ol (préparé selon H. Gerlach, P. Künzler, K. Oertle, Helv Chim. Acta, 61 (1978), 1226-1231.) et 700 mg de tamis moléculaire 4Å en poudre sont mis en suspension dans 10 mL de dichlorométhane anhydre. Le tout est mis sous atmosphère d'argon et agité durant 30mn. La solution est refroidie à -20°C et 126 mg (709 μmol, 2 éq) de N-bromosuccinimide ainsi que 6.3 μL (7.08 μmol, 0.2 éq) d'acide trifluorométhanesulfonique y sont ajoutés. Après 1 heure à -20°C, une solution d'hydrogénocarbonate de sodium est additionnée. Le tout est filtré sur un lit de célite. La phase organique est lavée par une solution de thiosulfate de sodium et par une solution aqueuse de NaCl. Celle-ci est ensuite séchée sur sulfate de magnésium, filtrée et évaporée sous vide. Le brut est chromatographié sur gel de silice (élution:cyclohexane/acétate d'éthyle=3.5/1) et fournit 412 mg (70%) du composé 10 (α/β=1/6) non séparables sous la forme d'une mousse blanche.

$^1$H-R.M.N. (400MHz, $CDCl_3$) du produit β-O-connecteur : δ 7.48-7.16 (m, 40H, arom.),5.64, 5.62 et 5.6, 5.34 (4s, 4H, by), 5.34 (s, 1H, H-1A), 5.16 (s, 1H, H-1B), 4.86 (d, 1H, $J_{gem}$=12.3Hz, CHPh), 4.84 (s, 1H, H-1C), 4.82 (d, 1H, $J_{gem}$=12Hz, CHPh) , 4.79 (d, 1H, $J_{gem}$=12.4Hz, CHPh), 4.74 (d, 1H, $J_{2-3}$=3.2Hz, H-2C), 4.73 (d, 1H, $J_{gem}$=12Hz, CHPh), 4.68 (d, 1H, $J_{gem}$=12.3Hz, CHPh), 4.67 (d, 1H, $J_{gem}$=11.8Hz, CHPh), 4.65 (d, 1H, $J$ 2-3=3.4Hz, H-2A), 4.63 (d, 1H, $J_{gem}$=12.4Hz, CHPh), 4.56 (d, 1H, $J_{gem}$=11.8Hz, CHPh), 4.51 (d, 1H, $J_{2-3}$=3.2Hz, H-2B), 4.47 (s, 1H, H-1D) , 4.41 (dd, 1H, $J_{6b-6a}$=10.4Hz et $J_{6b-5}$=4.8Hz, H-6Cb), 4.36 (dd, 1H, $J_{6b-6a}$=10.3Hz et $J_{6b-5}$=4.8Hz, H-6Ab), 4.32 (dd, 1H, $J_{6b-6a}$=10.3Hz et $J_{6b-5}$=4.5Hz, H-6Db), 4.28 (t, 1H, $J_{4-3}$=$J_{4-5}$=9.6Hz, H-4A), 4.23 (dd, 1H, $J_{6b-6a}$=10.4Hz et $J_{6b-5}$=4.6Hz, H-6Bb), 4.21 (d, 1H, $J_{2-3}$=3.15Hz, H-2D), 4.17 (t, 1H, $J_{4-3}$= $J_{4-55}$=9.3Hz, H-4B), 4.01 (t, 1H, $J_{6a-6b}$=$J_{6a-5}$=10.3Hz, H-6Aa), 3.99 (t, 1H, $J_{4-3}$= $J_{4-5}$=9.5Hz, H-4C), 3.91 (t, 1H, $J_{6-6b}$= $J_{6a-5}$=10.4Hz, H-6Ca), 3.95-3.88 (m, 1H, -O-CH-$CH_2$-), 3.88 (t, 1H, $J_{4-3}$=$J_{4-5}$=9.8Hz, H-4D), 3.85 (t, 1H, $J_{6a-6b}$= $J_{6a-5}$=10.4Hz, H-6Ba), 3.69 (s, 3H, -C:O-$OCH_3$), 3.64 (dd, 1H, $J_{3-2}$=3.2Hz et $J_{3-4}$=9.5Hz, H-3C), 3.6 (dd, 1H, $J_{3-2}$=3.4Hz et $J_{3-4}$=9.6Hz, H-3A), 3.59 (dd, 1H, $J_{3-2}$=3.15Hz et $J_{3-4}$=9.8Hz, H-3D), 3.60-3.57 (m, 1H, H-6Da) , 3.54 (dd, 1H, $J_{3-2}$=3.2Hz et $J_{3-4}$=9.3Hz, H-3B), 3.5-3.27 (m, 5H, H-5A, H-5B, H-5C, H-5D et -O-CH-$CH_2$-), 2.31 (t, 2H, $J_{3-2}$=7.5Hz, -$CH_2$C:O-$OCH_3$),1.64-1.58, 1.34-1.31 (m, 12H, -$CH_2$), 0.94 (s, 9H, $SiC(CH_3)_3$), 0.25 et 0.14 (2s, 6H, $Si(CH_3)_2$).

$^{13}$C R.M.N. (100 MHz) : 8 138.5, 138.4, 138.39, 138.2, 137.7, 137.5, 137.1, 137.08 (8 C arom.), 129-126 (40 CH arom), 103.9 (159.7C-1C), 102.7 ($^1J_{CH}$=160Hz, C-1D), 101.8 (CH by), 101.6 (CH by), 101.58 ($^1J_{CH}$=155Hz, C-1A), 101.5 (CH by), 101.35 ($^1J_{CH}$=158.5Hz, C-1B), 101.2 (CH by), 79.9 (C-3B), 78.97 (C-4B) , 78.46 (C-4D) , 78.4 (C-4C), 77.9 (C-4A), 77.6 (C-2D), 76.29 (C-3D), 76.27 (C-3C), 76.23 (C-3A), 75.22 (C-2A), 72.83 (C-2C), 72.8 ($CH_2$Ph), 71.9 ($CH_2$Ph), 71.16 (C-2B), 70.62 ($CH_2$Ph), 70.14 (-O-$CH_2$-), 69.8 ($CH_2$Ph), 68.87, 68.82*2, 68.5 (C-6A, C-6B, C-6C et C-6D), 67.88 (C-5B), 67.83 (C-5C), 67.7 (C-5A), 67.4 (C-5D), 34 (-$CH_2$-C:O-O-$CH_3$), 29.5, 29.2, 29.1, 29, 25.5, 24.8 (-$CH_2$-), 25.9 ($C(CH_3)_3$), 18.5 ($C(CH_3)_3$), -3.7 ($SiCH_3$), -4.7 ($SiCH_3$).

Spectre de masse : m/z 1663.7 $(M+H)^+$.

Analyse pour $C_{96}H_{114}O_{23}Si$ (1664.03) : calculée C:69.29 H:6.905 trouvée C:69.13 H:7.06

9) 8-carboxyloctyl 2-O-(2-O-(2-O-(β-D-mannopyranosyl)-β-D-mannopyranosyl)-β-D-mannopyranosyl)-D-mannopyranoside (I, R=connecteur).

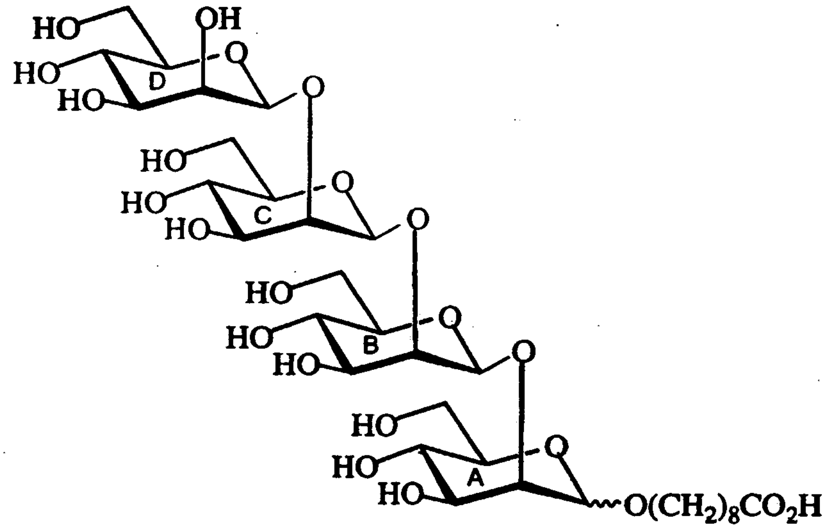

- Etape 1: désilylation

305 mg (183 μmol, 1 éq) du composé 10 et 405 mg (1.28 mmol, 7 éq) de fluorure de tetrabutylammonium trihydrate sont mis en solution dans 10 mL de tetrahydrofurane. Après 12 heures de chauffage à 60°C, le milieu reactionnel est dilué par du dichlorométhane et lavé par de l'eau. La phase organique est séchée sur sulfate de magnésium, filtrée et évaporée sous pression réduite. La chromatographie du brut sur gel de silice (élution:cyclohexane/acétate d'éthyle=2/1) fournit 230 mg (80%) du produit désilylé sous la forme d'une mousse blanche.

Etape 2 saponification

140 mg (85 μmol) du produit précédent sont dissous dans 5 mL de tetrahydrofurane. A cela sont ajoutés 4.5 mL (0.1N) d'hydroxyde de sodium. Le tout est chauffé à 60°C durant une nuit. La solution est alors acidifiée par une solution d'acide chlorhydrique (1M) et extraite 3 fois par du dichlorométhane. La phase organique est séchée sur $MgSO_4$, filtrée et évaporée sous vide. La chromatographie du brut sur gel de silice (élution :dichlorométhane / méthanol=50/1) fournit 115 mg (82%) du produit sous la forme d'une mousse blanche.

Etape 3 hydrogénolyse

66mg (42 μmol) du produit précédent sont dissous dans 2 mL de méthanol et agités sous atmosphère de dihydrogène (1.4bar) en présence de Pd/C (10 %) en quantité catalytique pour donner (I, R=connecteur) (29.3 mg, 83%), après lyophilisation, sous forme d'une poudre blanche amorphe.(mélange α/β 1/6 au niveau de l'aglycone)
$^1$H-R.M.N., (400MHz, $D_2O$) du produit β-O-connecteur : δ4.99 (se, 1H, H-1A), 4.89 (se, 1H, H-1B), 4.84 (se, 1H, H-1C) , 4.68 (se, 1H, H-1D) , 4.36 (d, 1H, $J_{2-3}$=3Hz, H-2A), 4.28 (d, 1H, $J_{2-3}$=3.2Hz, H-2C), 4.18 (d, 1H, $J_{2-3}$=3.19Hz, H-2D), 4.11 (d, 1H, $J_{2-3}$=3.2Hz, H-2B), 3.9-3.84 (m, 5H, H-6Ab, H-6Bb, H-6Cb, H-6Db et -O-CH-$CH_2$), 3.73-3.65 (m, 4H, H-6Aa, H-6Ba, H-6Ca, H-6Da), 3.63 (dd, 1H, $J_{3-2}$=3.19Hz et $J_{3-4}$=9.7Hz, H-3D), 3.62-3.57 (m, 2H, - O-CH-$CH_2$ et H-3C), 3.59 (dd, 1H, $J_{3-2}$=3Hz et $J_{3-4}$=10.3Hz, H-3A), 3.57 (dd, 1H, $J_{3-2}$=3.2Hz et $J_{3-4}$=9.7Hz, H-3B), 3.55 (t, 1H, $J_{4-3}$=$J_{4-5}$=10.3Hz, H-4A), 3.51 (t, 1H, $J_{4-3}$=$J_{4-5}$=9.7Hz, H-4B), 3.46 (t, 1H, $J_{4-3}$=$J_{4-5}$=9.7Hz, H-4C), 3.44 (t, 1H, $J_{4-3}$=$J_{4-5}$=9.7Hz, H-4D), 3.36-3.3 (m, 4H, H-5A, H-5B, H-5C et H-5D), 2.25 (t, 2H, $J_{CH2-CH2}$=7.4Hz,- $CH_2$-COOH), 1.6-1.52 et 1.3-1.28 (m, 12H, -$CH_2$-).
$^{13}$C R.M.N. (100 MHz) : 8 181.7 (COOH), 101.58, 101.46, 101.26, 100.32 (C-1A, C-1B, C-1C, C-1D), 79.5, 79.3, 78.7, 76.6, 76.5*3, 73.2, 72.6, 72.3*2, 70.7, 67.8, 67.4, 67.2, 67.1, 61.5, 61.06, 61.02, 60.9 (20 CH cycle), 70.4 (-O-$CH_2$-), 34 (-$CH_2$-$CO_2$H), 29, 28.7, 28.65, 28.6, 25.7, 25.5 (6 -$CH_2$-).
Spectre de masse (FAB) m/z calculé $C_{33}H_{58}O_{23}Na$ : 845.32. Trouvé 845.24

10) 2-O-(2-O-(2-O-(3-O-benzyl-4,6-O-benzylidène-β-D-mannopyranosyl)-3-O-benzyl-4, 6-O-benzylidène-β-D-manno pyranosyl)-3-O-benzyl-4,6-O-benzylidène-β-D-mannopyranosyl) -3-O-benzyl-4,6-O-benzylidène-D-mannopyranose (9).

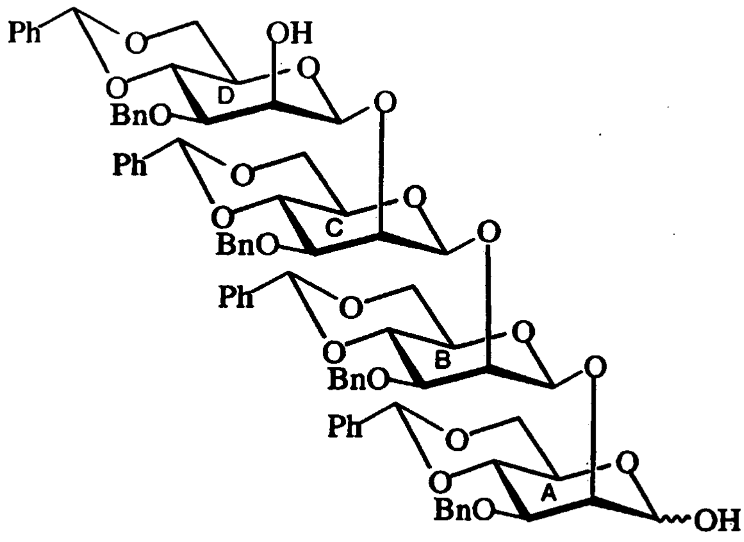

Etape 1: désilylation

Pour le protocole expérimental, voir l'étape 1 du composé I (R=connecteur).

8 (235 mg) traité par le fluorure de tetrabutylammonium donne du phénol 2-O-(2-O-(2-O-(3-O-benzyl-4,6-O-benzylidène β-D-mannopyranosyl)-3-O-benzyl-4,6-O-benzylidéne-β-D-mannopyranosyl)-3-O-benzyl-4,6-O-benzylidène-β-D-mannopyranosyl)-3-O-benzyl-4,6-O-benzylidène-1-thio-α-D-mannopyranoside (202 mg, 92%) sous la forme d'une mousse blanche, après une chromatographie sur gel de silice (élution:cyclohexane/acétate d'éthyle=2.5/1) et après une recristallisation dans du méthanol.
pf : 133-136°C(méthanol); $[\alpha]_D$ -42 (c 0.5, chloroforme)
$^1$H-R.M.N. (400MHz, $CDCl_3$) : δ 7.57-7.32m (45H, arom.), 5.73, 5.71, 5.67 et 5.66 (4s, 4H, by), 5.64 (s, 1H, H-1A), 5.36 (s, 1H, H-1B), 4.92 (s, 1H, H-1D), 4.86 (d, 1H, $J_{gem}$=12.1Hz, CHPh), 4.83 (d, 1H, $J_{gem}$=11.8Hz, CHPh), 4.79 (s, 1H, H-1C) , 4.78 (s, 2H, $CH_2$Ph) , 4.77 (2d, 2H, 2CHPh), 4.71 (d, 1H, $J_{2\text{-}3}$=3.2Hz, H-2A), 4.69 (d, 1H, $J_{gem}$=11.8Hz, CHPh), 4.64 (d, 1H, $J_{gem}$=11.8Hz, CHPh), 4.61 (d, 1H, $J_{2\text{-}3}$=3Hz, H-2B), 4.53 (d, 1H, $J_{2\text{-}3}$=3.4Hz, H-2C), 4.51-4.46 (m, 1H, H-5A), 4.47 (dd, 1H, $J_{6b\text{-}6a}$=10.3Hz et $J$ 6b-$_5$=4.8Hz, H-6Bb), 4.41-4.36 (m, 4H, H-6Ab, H-6Cb, H-6Db, H-2D), 4.36 (t, 1H, $J_{4\text{-}3}$=$J_{4\text{-}5}$=9.7Hz, H-4D), 4.32 (t, 1H, $J_{4\text{-}3}$=$J_{4\text{-}5}$=9.8Hz, H-4B), 4.21 (t, 1H, J $_{4\text{-}3}$=$J_{4\text{-}5}$=9.7Hz, H-4C), 4.13-4.11 (m, 2H, H-4A et H-3A), 4.08 (t, 1H, $J_{6a\text{-}6b}$=$J_{6a\text{-}5}$=10Hz, H-6Ba), 3.98 (dd, 1H, $J_{6a\text{-}6b}$=10.4 et $J_{6a\text{-}5}$=9.7Hz, H-6Ca), 3.95 (dd, 1H, $J_{6a\text{-}6b}$=10.1$J_{6a\text{-}5}$=9.7Hz, H-6Da), 3.91 (t, 1H, $J_{6a\text{-}6b}$=$J_{6a\text{-}5}$=10.2Hz, H-6Aa), 3.75 (dd, 1H, $J_{3\text{-}2}$=3.4Hz et $J_{3\text{-}4}$=9.7Hz, H-3C), 3.65 (dd, 1H, $J_{3\text{-}2}$=3Hz et $J_{3\text{-}4}$=9.8Hz, H-3B), 3.56 (ddd, 1H, $J_{5\text{-}4}$=9.8Hz et $J_{5\text{-}6b}$=4-8Hz et $J_{5\text{-}6a}$=9.8Hz, H-5B), 3.53 (dd, 1H, $J_{3\text{-}2}$=3.1Hz et $J_{3\text{-}4}$=9.6Hz, H-3D), 3.44 (ddd, 1H, $J_{5\text{-}4}$=9.7Hz et $J_{5\text{-}6b}$=9.7Hz et $J_{5\text{-}6a}$=4.7Hz, H-5C), 3.35 (ddd, 1H, $J_{5\text{-}4}$=9.7Hz et $J_{5\text{-}6b}$=9.7Hz et $J_{5\text{-}6a}$=4.9Hz, H-5D), 3.28 (se, 1H, OH).

$^{13}$C R.M.N. (100 MHz) : δ 138.2*2, 137.9, 137.8, 137.5, 137.2, 137.04, 136.8, 133.05 (9 C arom.), 131.5-125.9 (45 CH arom), 102.1 (CH by), 101.5 (CH by), 101.48 (C-1D), 101.26 (CH by), 101.15 (CH by), 101 (C-1B), 98.2 (C-1C), 85.14(C-1A), 78.98 (C-4A), 78.4 (C-4D), 78 (C-4B), 77.98 (C-4C), 77.7 (C-3B), 76.9 (C-3D), 75.3 (C-3C), 74.6 (C-3A), 74.4 (C-2A), 74.3 (C-2C), 74.1 (C-2B), 72.4 ($CH_2$Ph) , 71.8*2 ($CH_2$Ph), 71 ($CH_2$Ph) , 68.9 (C-2D), 68.6 (C-6D), 68.5 (C-6A) , 68.4 (C-6B), 68.25 (C-6C), 67.8 (C-5B et C-5C), 66.8 (C-5D), 64.9 (C-5A).

Spectre de masse : m/z 1488 $(M+NH4)^+$.

Analyse pour $C_{86}H_{86}O_{20}S.1CH_3OH$ (1503.67) : calculée C:69.49 H:6.03 trouvée C:69.28 H:5.92

Etape 2 hydrolyse du thiophényl

175 mg (119 μmol, 1 éq) du composé précédent sont dissous dans un mélange de solvants acétone/eau=4.5mL/0.5mL. Le tout est refroidi à 0°C. A cela sont ajoutés 106 mg (0.595 mmol, 5 éq) de N-bromosuccinimide. Une solution d'hydrogénocarbonate de sodium est versée dans le ballon après 30 mn d'agitation à 0°C. L'acétone est alors évaporée sous vide. Le résidu est repris par du dichlorométhane, lavé par une solution de thiosulfate de sodium et par une solution aqueuse de NaCl. La phase organique est ensuite séchée sur sulfate de magnésium, filtrée et évaporée. Le brut est chromatographié sur gel de silice (élution:cyclohexane/acétate d'éthyle=1.8/1 puis 1.5/1) et donne 138 mg (Rdt=84%) du composé 9 d'un mélange de configuration α/β (2.6/1) sous forme d'une mousse blanche.

$^1$H-R.M.N. (400MHz, $CDCl_3$) du produit α-OH: δ 5.52 (s, 1H, H-1B) , 5.22 (de, 1H, $J_{1\text{-}OH}$=3Hz, H-1A) , 4.78 (s, 1H, H-1D) , 4.63 (s, 1H, H-1C) .

$^{13}C$ R.M.N. (100 MHz) : δ 101.7 (C-1D), 101.1 (C-1B), 99.2 (C-1C), 92.09 (C-1A) et disparition du pic caractéristique à 85.14 ppm de C-1A possédant un thiophénol en tant qu'aglycone.

Spectre de masse : m/z 1396.8 $(M+NH4)^+$.

Analyse pour $C_{80}H_{82}O_{21}$ (1379.529) : calculée C:69.65 H:5.99 trouvée C:69.46 H:6.11

11) 2-O-(2-O-(2-O-(β-D-mannopyranosyl) -β-D-mannopyranosyl)-β-D-mannopyranosyl)-D-mannopyranose (I, R=H).

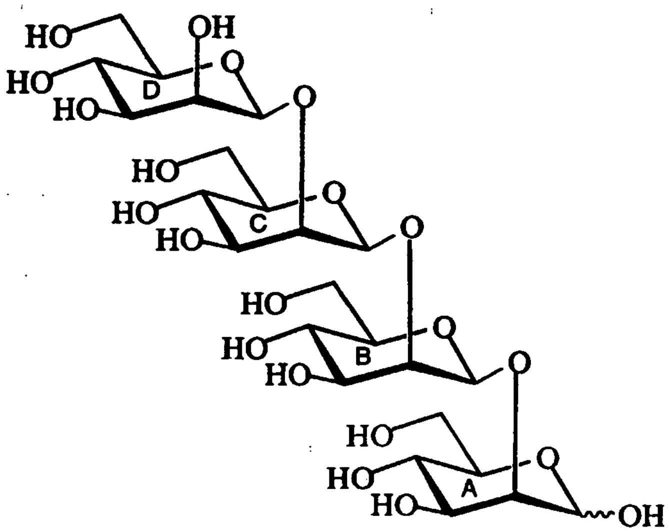

Pour le protocole expérimental, voir l'étape 3 du composé I (R=connecteur).

111.4 mg (80.7 μmol) du composé 9 donne 46 mg (85%) du composé I (R=H) après lyophilisation sous forme d'une poudre amorphe.

$^1H$-R.M.N. (400MHz, $D_2O$) du produit α-OH : δ 5.24 (d, 1H, $J_{1-2}$=1.64Hz, H-1A), 4.91 (s, 1H, H-1C), 4.89 (s, 1H, H-1D), 4.81 (se, 1H, H=1B), 4.39 (d, 1H, $J_{2-3}$=3.2Hz, H-2C), 4.22 (d, 1H, $J_{2-3}$=3.27Hz, H-2B), 4.11 (d, 1H, $J_{2-3}$=3.1Hz, H-2D), 4.08 (dd, 1H, $J_{2-1}$=1.64Hz et $J_{2-3}$=3.02Hz, H-2A).

$^{13}C$ R.M.N. (100 MHz) : δ 101.53, 101.29, 99.46, 92.4 (C-1A, C-1B, C-1C, C-1D), 79.7, 79, 78.7, 76.6, 76.5, 76.4, 73.2, 72.67, 72.5, 72.2, 70.7, 69.4, 67.7, 67.4, 67.26, 67.1, 61.5, 61.1, 60.84, 60.7 (20 CH cycle).

Spectre de masse (FAB) m/z : calculée $C_{24}H_{42}O_{21}Na$ : 689.21 obtenue : 689.32

Exemple 2: Préparation de D-Man α(1-2) D-Man α(1-2) D-Man α(1-2) D-Man, de formule (II).

I - Schéma réactionnel.

La figure 2 en annexe représente le schéma réactionnel de la préparation de D-Man α(1-2) D-Man α(1-2) D-Man α(1-2) D-Man de formule (II). Comme dans l'exemple 1 une stratégie par bloc a été utilisée. Un groupe thiophényl protège intermédiairement le carbone anomère. Le composé 11 conduit selon la réaction mise en oeuvre aux composés 12 ou 13, lesquels sont condensés en le disaccharide 14.

La réaction (a') est réalisée dans les conditions suivantes : $CH_3COOH$, $H_2O$ (80/20) puis $CCl_3CN$, DBU, $CH_2Cl_2$, 0°C.

La réaction (b') est réalisée dans les conditions suivantes : PhSH, $HgBr_2$, $CH_3CN$, 80°C puis $CH_30Na$, $CH_30H$.

La réaction (c') est réalisée dans les conditions suivantes : TMSOTf, tamis moléculaire 4 Angstrom, $CH_2Cl_2$.

Le disaccharide 14 est transformé en disaccharides 15 et 16 lesquels sont condensés pour obtenir le tetrasaccharide clé 17.

La réaction (d') est réalisée dans les conditions suivantes : NBS, eau, acétone, puis $CCl_3CN$, DBU, $CH_2Cl_2$.

La réaction (e') est réalisée dans les conditions suivantes : MeONa, MeOH.

La réaction (f') est réalisée dans les conditions suivantes : 1) $BF_3$. $Et_2O$, tamis moléculaire 4 Angstrom, $CH_2Cl_2$ ; 2) MeONa/MeOH.

Le tetrasaccharide clé 17 peut être ou non fonctionalisé en l'un des composés 18 ou 19 et donne après déprotection le tétramannoside de formule (II) dans laquelle R est H ou $-(CH_2)_8-Co_2Me$.

La réaction (g') est réalisée dans les conditions suivantes : NBS, TfOH, 8-methoxycarbonyloctanol, tamis moléculaire 4 Angstrom, $CH_2Cl_2$, -15°C.

La réaction (h') est réalisée dans les conditions suivantes : NBS, eau, acétone.

La réaction (i') est réalisée dans les conditions suivantes : 1- NaOH, THF, eau, 2-$H_2$, Pd/C, MeOH.

La réaction (j') est réalisée dans les conditions suivantes : $H_2$, Pd/C, MeOH.

II - Protocole expérimental.

Les composés 12 (F Yamazaki, S Sato, T Nukuda, Y. Ito, T Ogawa, Carbohydr. Res. 201 (1990) 31-50) et 13 (Y-M. Zhang, J.-M. Mallet, P. Sinaÿ.Carbohydr. Res. 236, (1992), 73-88.) sont préparés selon la littérature à partir de l'orthoester 11 (N. E. Franks R Montgomery, Carbohydr. Res. 6 (1968) 286-98)

1) Phényl 2-O-(2-O-acétyl-3,4,6-tri-O-benzyl-α-D-mannopyranosyl)-3,4,6-tri-O-benzyl-1-thio-α-D-mannopyranoside (14).

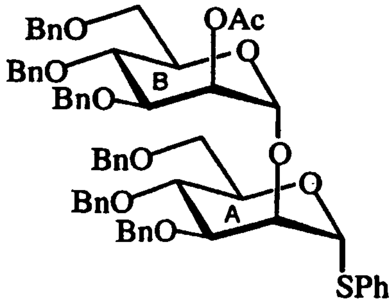

Un mélange de 12 (F Yamazaki, S Sato, T Nukuda, Y. Ito, T Ogawa, Carbohydr. Res. 201 (1990) 31-50) (9.7 g, 15.24 mmol, 1.2 éq), 13 (Y-M. Zhang, J.-M. Mallet, P. Sinaÿ.Carbohydr. Res. 236, (1992), 73-88) (6.88 g, 12.7 mmol, 1 éq) et 17 g de tamis moléculaire 4Å en poudre dans 100 mL de dichlorométhane anhydre est agité sous atmosphère d'argon. Après 30 mn d'agitation, le milieu reactionnel est refroidi à -10°C. Du trifluorométhanesulfonate de triméthylsilyle (0.44 mL, 1.9 mmol, 0.15 éq) sont alors additionnés. 30mn plus tard, la solution est neutralisée par une solution aqueuse d'hydrogénocarbonate de sodium. La phase organique est ensuite lavée par une solution de saumure, séchée sur sulfate de magnésium, filtrée et concentrée. La chromatographie du brut sur gel de silice (élution:cyclohexane/acétate d'éthyle=6/1) fournit 14 (9.3 g, 72%) sous la forme d'une sirop incolore. $[\alpha]_D$ +93 (c 0.55, chloroforme)
$^1$H-R.M.N. (400MHz, $CDCl_3$) : δ 7.48-7.25 m, (35H, arom.), 5.69 (d, 1H, $J_{1-2}$=1.62Hz, H-1A), 5.58 (dd, 1H, $J_{2-1}$=1.7Hz et $J_{2-3}$=3.3Hz, H-2B), 5.13 (d, 1H, $J_{1-2}$=1.7Hz, H-1B), 4.94 (d, 1H, $J_{gem}$=10.8Hz, CHPh), 4.87 (d, 1H, $J_{gem}$=10.8Hz, CHPh), 4.79 (d, 1H, $J_{gem}$=11.8Hz, CHPh), 4.75 (d, 1H, $J_{gem}$=11.8Hz, CHPh), 4.71 (d, 1H, $J_{gem}$=10.9Hz, CHPh), 4.69 (d, 1H, $J_{gem}$=12Hz, CHPh), 4.64 (d, 1H, $J_{gem}$=10.8Hz, CHPh), 4.6 (d, 1H, $J_{gem}$=12.3Hz, CHPh), 4.52 (d, 1H, $J_{gem}$=12Hz, CHPh), 4.47 (d, 1H, $J_{gem}$=10.8Hz, CHPh), 4.45 (d, 1H, $J_{gem}$=10.9Hz, CHPh), 4.43 (d, 1H, $J_{gem}$=12.3Hz, CHPh), 4.33 (ddd, 1H, $J_{5-4}$=9.3Hz, $J_{5-6a}$=1.7Hz et $J_{5-6b}$=5.2Hz, H-5A), 4.28 (dd, 1H, $J_{2-1}$=1.62Hz et $J_{2-3}$=2.8Hz, H-2A), 4.03 (dd, 1H, $J_{3-2}$=3.3Hz et $J_{3-4}$=9.4Hz, H-3B), 4.03-3.97 (m, 1H, H-5B), 3.99 (t, 1H, $J_{4-3}$=$J_{4-5}$=9.3Hz, H-4A), 3.94 (dd, 1H, $J_{3-2}$=2.8Hz et $J_{3-4}$=9.3Hz, H-3A), 3.88 (t, 1H, $J_{4-3}$=$J_{4-5}$=9.4Hz, H-4B), 3.88 (dd, 1H, $J_{6b-6a}$=11.1Hz et $J_{6b-5}$=5.2Hz, H-6Ab), 3.77 (dd, 1H, $J_{6b-6a}$=11.1Hz et $J_{6b-5}$=1.7Hz, H-6Aa), 3.74 (dd, 1H, $J_{6b-6}$a=10.6Hz et $J_{6b-5}$=4.5Hz, H-6Bb), 3.77 (dd, 1H, $J_{6b-6a}$=10.6Hz et $J_{6b-5}$=1.7Hz, H-6Ba), 2.19 (s, 3H, -C:O-$OCH_3$).
$^{13}$C R.M.N. (100 MHz) : δ 170.2 (-C:O-$OCH_3$), 138.4, 138.3, 138.27, 138, 137.98, 137.9, 131.6 (7 C arom.), 129-127.3 (35 CH arom), 99.7 ($^1J_{CH}$=169.2Hz, C-1B) , 87.1 ($^1J_{CH}$=168.8Hz, C-1A), 79.89 (C-3A), 78 (C-3B) , 76.6 (C-2A) , 75.2 ($CH_2$Ph), 75 ($CH_2$Ph), 74.7 (C-4A), 74.3 (C-4B), 73.1 (2 $CH_2$Ph), 72.8 (C-5A), 72.2 ($CH_2$Ph), 71.9 ($CH_2$Ph), 71.9 (C-5B), 69.1 (C-6A), 68.7 (C-2B), 68.5 (C-6B), 21.1 (-C:O-$OCH_3$).
Spectre de masse : m/z 1034.4 (M+NH4)$^+$.
Analyse pour $C_{62}H_{64}O_{11}S$ (1017.256) : calculée C:73.20 H:6.35 trouvée C:72.98 H:6.65

2) O-[2-O-(2-O-acétyl-3,4,6-tri-O-benzyl-α-D-mannopyranosyl)-3,4,6-tri-O-benzyl-α-D-mannopyranosyl et β-D-mannopyranosyl] trichloroacétimidate (15).

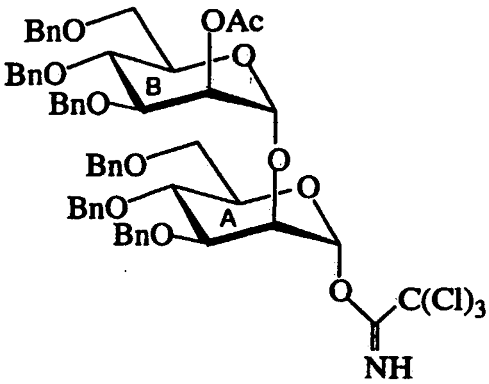

14 (3 g, 2.95 mmol, 1 éq) est dissous dans 150 mL d'un mélange acétone/eau:95/5. A cette solution sont ajoutés 2.6 g (11.8 mmol, 4 éq) de N-bromosuccinimide. Après 15 mn d'agitation, une solution d'hydrogénocarbonate de sodium est ajoutée et l'acétone est évaporée sous vide. Le résidu est repris par du dichlorométhane et lavé par une solution de saumure. Une flash chromatographie sur gel de silice du brut fournit 2.37 g (Rdt=87%) du produit OH libre en position réductrice sous forme d'une huile incolore. Cette huile ainsi obtenue est dissoute dans 10 mL de dichlorométhane anhydre. 3.08 mL (12 éq) de trichloroacétonitrile sont ajoutés, la solution est refroidie à 0°C et 115 μL (0.3 éq) de 1,8-diazabicyclo-[5.4.0]undec-7-ène sont alors additionnés au milieu. Après 20 mn, la solution est injectée directement sur un gel de silice (élution:cyclohexane/acétate d'éthyle=3/1) et 2.2 g (Rdt=81% sur les deux étapes) du composé 15α très majoritaire (α/β=92/8) sont ainsi obtenus sous la forme d'une huile incolore.

$^1$H-R.M.N. (400MHz, $CDCl_3$) du produit α-O-Imidate : 88.55 (s, 1H, C=NH) , 7.39-7.25 (m, 30H, arom.), 6.36 (d, 1H, $J_{1-2}$=1.95Hz, H-1A) , 5.67 (dd, 1H, $J_{2-1}$=1.6Hz et $J_{2-3}$=3.4Hz, H-2B), 5.2 (d, 1H, $J_{1-2}$=1.6Hz, H-1B), 4.95 (d, 1H, $J_{gem}$=10.6Hz, CHPh), 4.89 (d, 1H, $J_{gem}$=10.8Hz, CHPh), 4.81 (d, 1H, $J_{gem}$=11.9Hz, CHPh), 4.75 (d, 1H, $J_{gem}$=12Hz, CHPh), 4.73 (d, 1H, $J_{gem}$=11.9Hz, CHPh), 4.71 (d, 1H, $J_{gem}$=10.8Hz, CHPh), 4.7 (d, 1H, $J_{gem}$=12.6Hz, CHPh), 4.65 (d, 1H, $J_{gem}$=10.6Hz, CHPh), 4.55 (d, 1H, $J_{gem}$=12.6Hz, CHPh), 4.52 (d, 1H, $J_{gem}$=12Hz, CHPh), 4.51 (d, 1H, $J_{gem}$=10.8Hz, CHPh), 4.45 (d, 1H, $J_{gem}$=10.8Hz, CHPh), 4.13 (dd, 1H, $J_{2-1}$=1.95Hz et $J_{2-3}$=3.3Hz, H-2A), 4.09 (t, 1H, $J_{4-3}$=$J_{4-5}$=9.7Hz, H-4A), 4.08-3.97 (m, 5H, H-5A, H-5B, H-3A, H-3B et H-4B), 3.89 (dd, 1H, $J_{6b-6a}$=11.5Hz et $J_{6b-5}$=3.6Hz, H-6Ab), 3.86 (dd, 1H, $J_{6b-6a}$=11.6Hz et $J_{6b-5}$=4.1Hz, H-6Bb), 3.78 (dd, 1H, $J_{6b-6a}$=11.5Hz et $J_{6b-5}$=1.1Hz, H-6Aa), 3.75 (dd, 1H, $J_{6b-6a}$=11.6Hz et $J_{6b-5}$=1Hz, H-6Ba), 2.2 (s, 3H, -C:O-$OCH_3$).

$^{13}$C R.M.N. (100 MHz) : 8 170.25 (-C:O-$OCH_3$), 159.98 (-C=NH), 138.4, 138.3, 138.15, 138.07, 137.9, 137.88 (6 C arom.), 128.4-127.4 (30 CH arom), 99.5 (C-1B), 96.7 (C-1A), 78.5 (C-3A), 78.1 (C-3B), 75.4 ($CH_2$Ph), 75.1 ($CH_2$Ph), 74.7 (C-5B), 74.1 (C-4B), 73.8 (C-4A), 73.3 ($CH_2$Ph), 73.2 ($CH_2$Ph), 73 (C-2A), 72.4 ($CH_2$Ph), 71.97 ($CH_2$Ph), 71.9 (C-5A), 68.62 (C-2B), 68.58 (C-6B), 68.51 (C-6A), 21.15 (-C:O-OCH3).

Pas d'analyse élémentaire pour ces composés peu stables.

3) Phényl 2-O-(3,4,6-tri-O-benzyl-α-D-manno pyranosyl) - 3,4,6-tri-O-benzyl-1-thio-α-D-mannopyranoside (16).

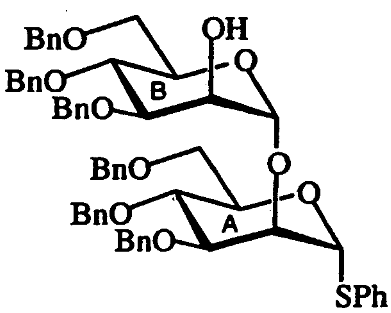

14 (6 g, 5.9mmol) est dissous dans 40 mL d'un mélange toluène/méthanol:1/1. A cela est ajouté du sodium (cat.). Après 15 mn, la solution est neutralisée par de la résine amberlite IR 120 (H+), filtrée et concentrée. Une chromatographie sur gel de silice (élution :cyclohexane / acétate d'éthyle=3/1) du brut fournit 16 (5.45 g, 95%) sous forme d'un sirop incolore.$[\alpha]_D$ +93.6 (c 0.51, chloroforme) $^1$H-R.M.N. (400MHz, $CDCl_3$) : δ 7.5-7.2 (m, 35H, arom.), 5.76 (d,

1H, $J_{1-2}$=1.6Hz, H-1A), 5.22 (d, 1H, $J_{1-2}$=1.6Hz, H-1B), 4.95 (d, 1H, $J_{gem}$=10.7Hz, CHPh), 4.85 (d, 1H, $J_{gem}$=10.9Hz, CHPh), 4.77 (s, 2H, $CH_2Ph$), 4.75 (d, 1H, $J_{gem}$=12Hz, CHPh), 4.66 (d, 1H, $J_{gem}$=10.7Hz, CHPh), 4.65 (d, 1H, $J_{gem}$=11.3Hz, CHPh), 4.6 (d, 1H, $J_{gem}$=11.3Hz, CHPh), 4.57 (d, 1H, $J_{gem}$=12.4Hz, CHPh), 4.55 (d, 1H, $J_{gem}$=12Hz, CHPh), 4.52 (d, 1H, $J_{gem}$=10.9Hz, CHPh), 4.45 (d, 1H, $J_{gem}$=12.4Hz, CHPh), 4.37-4.33 (m, 1H, H-5A), 4.34 (dd, 1H, $J_{2-1}$=1.6Hz et $J_{2-3}$=2.7Hz, H-2A), 4.21-4.18 (m, 1H, H-2B), 4.01 (t, 1H, $J_{4-3}$=$J_{4-5}$=9.3Hz, H-4A), 4.03-3.98 (m, 1H, H-5B), 3.96 (dd, 1H, $J_{3-2}$=2.7Hz et $J_{3-4}$=9.3Hz, H-3A), 3.95 (dd, 1H, $J_{6b-6a}$=11.2Hz et $J_{6b-5}$=4.6Hz, H-6Ab), 3.93 (dd, 1H, $J_{3-2}$=3.1Hz et $J_{3-4}$=9.4Hz, H-3B), 3.87 (t, 1H, $J_{4-3}$=$J_{4-5}$=9.4Hz, H-4B), 3.8 (dd, 1H, $J_{6b-6a}$=11.2Hz et $J_{6b-5}$=1.6Hz, H-6Aa), 3.71 (dd, 1H, $J_{6b-6a}$=10.6Hz et $J_{6b-5}$=4.9Hz, H-6Bb), 3.64 (dd, 1H, $J_{6b-6a}$=10.6Hz et $J_{6b-5}$=1.9Hz, H-6Ba), 2.53 (d, 1H, $J_{OH-2}$=1.6Hz, OH).

$^{13}C$ R.M.N. (100 MHz) : δ 138.5, 138.3, 138.2, 138, 137.9, 137.86, 131.5 (7 C arom.), 128.9-127.3 (35 CH arom), 101.2 (C-1B), 87.2 (C-1A), 79.99 (C-3A), 79.9 (C-3B), 76.5 (C-2A), 75.1 ($CH_2Ph$), 75 ($CH_2Ph$), 74.8 (C-4A), 74.2 (C-4B), 73.13 ($CH_2Ph$), 73.1 ($CH_2Ph$), 72.8 (C-5A), 72.3 ($CH_2Ph$), 72.1 ($CH_2Ph$), 71.6 (C-5B), 69.1 (C-6A), 68.5 (C-6B), 68.47 (C-2B) .

Spectre de masse : m/z 992.6 $(M+NH4)^+$.

Analyse pour $C_{60}H_{62}O_{10}S$ (975.218) : calculée C:73.89 H:6.408 trouvée C:74.15 H:6.90

4) Phényl 2-O-(2-O-(2-O-(3,4,6-tri-O-benzyl-α-D-mannopyranosyl)-3,4,6-tri-O-benzyl-α-D-mannopyranosyl)-3,4,6-tri-O-benzyl-α-D-mannopyranosyl)-3,4,6-tri-O-benzyl-1-thio-α-D-mannopyranoside (17).

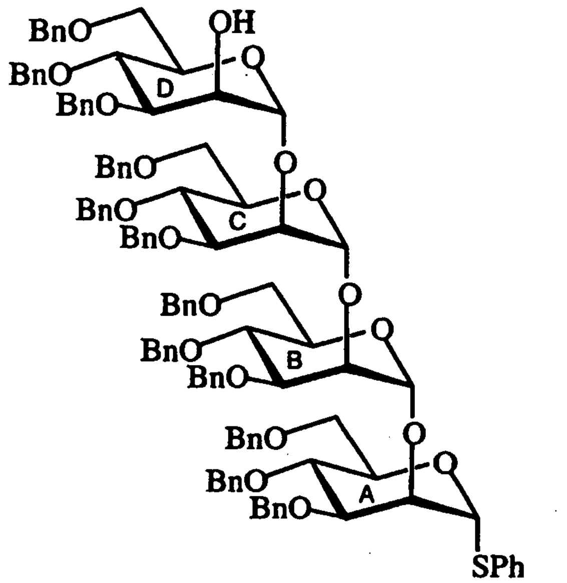

Un mélange de 16 (200 mg, 187 μmol, 1 éq), 15 (364 mg, 374 μmol, 2 éq), de 600 mg de tamis moléculaire 4 Å dans 6 mL de dichlorométhane anhydre est agité durant 30 mn sous atmosphère d'argon et refroidi à -10°C. Puis 71 μL (3 éq) d'éthérate de trifluorure de bore sont additionnés. Après 40 mn à -10°C, l'ensemble est neutralisé par une solution d'hydrogénocarbonate de sodium. Après une filtration sur un lit de célite, la phase organique est lavée par une solution de saumure, séchée sur sulfate de magnésium, filtrée et concentrée sous vide. Le résidu est chromatographié (élution:cyclohexane/acétate d'éthyle=6/1) et fournit 211 mg (Rdt=60%) d'une huile incolore. L'analyse par $^1H$-R.M.N montre la présence de deux produits, l'un de stéréochimie α au niveau de la liaison nouvellement créée et l'autre β. Ceux-ci sont malheureusement non séparables par chromatographie sur gel de silice. Le mélange est alors dissous dans un mélange toluène/méthanol et du sodium (cat.) y est ajouté. Après 15 mn, la solution est neutralisée par de la résine amberlite IR 120 (H+), filtrée et évaporée sous vide. Le résidu est chromatographié (élution:cyclohexane/acétate d'éthyle=5/1) et fournit le produit 17 (153 mg, 71%)

Caractéristiques de 17: $[\alpha]_D$ +48.7 (c 0.36, chloroforme)

$^1H$-R.M.N. (400MHz, $CDCl_3$) : δ 7.51-7.23 (m, 60H, arom.), 5.83 (d, 1H, $J_{1-2}$=1.4Hz, H-1A), 5.35 (d, 1H, $J_{1-2}$=1.4Hz, H-1B ou H-1C), 5.3 (d, 1H, $J_{1-2}$=1.6Hz, H-1B ou H-1C), 5.21 (d, 1H, $J_{1-2}$=1.5Hz, H-1D), 4.91 (d, 1H, $J_{gem}$=10.7Hz, CHPh), 4.9 (d, 1H, $J_{gem}$=10.9Hz, CHPh) , 4.89 (d, 1H, $J_{gem}$=10.6Hz, CHPh), 4.84 (d, 1H, $J_{gem}$=10.8Hz, CHPh), 4.71 (2d, 2H, $J_{gem}$=12.2Hz, 2 CHPh), 4.69 (d, 1H, $J_{gem}$=11.3Hz, CHPh), 4.67 (d, 1H, $J_{gem}$=11.3Hz, CHPh), 4.65 (s, 2H, $CH_2Ph$), 4.64 (d, 1H, $J_{gem}$=11.3Hz, CHPh), 4.61 (d, 1H, $J_{gem}$=10.9Hz, CHPh), 4.59 (d, 1H, $J_{gem}$=12.3Hz, CHPh), 4.56

(3d, 3H, 3 CHPh), 4.54 (d, 1H, $J_{gem}$=12.2Hz, CHPh), 4.53 (d, 1H, $J_{gem}$=11.3Hz, CHPh), 4.51 (d, 1H, $J_{gem}$=10.8Hz, CHPh), 4.49 (d, 1H, $J_{gem}$=11.9Hz, CHPh), 4.47 (d, 1H, $J_{gem}$=12.2Hz, CHPh), 4.46 (d, 1H, $J_{gem}$=12.8Hz, CHPh), 4.36 (d, 1H, $J_{gem}$=11.9Hz, CHPh), 4.36-4.34 (m, 2H, H-2A et H-5A, B, C ou D), 4.24 (d, 1H, $J_{gem}$=12.3Hz, CHPh), 4.21 (m, 1H, H-2D), 4.19 (dd, 1H, $J_{2-1}$=1.6Hz et $J_{2-3}$=2.4Hz, H-2B ou H-2C), 4.17 (dd, 1H, $J_{2-1}$=1.4Hz et $J_{2-3}$=2.4Hz, H-2B ou H-2C), 4.04-3.95 (m, 6H, H-3D, H-3B ou H-3C, H-4A, B, C ou D, 3*H-5A, B, C u D), 3.97-3.88 (m, 4H, H-3A, H-3B ou H-3C et 2*H-4A, B, C ou D), 3.83-3.56 (m, 5H, H-4A, B, C ou D, H-6A, H-6B, H-6C et H-6D), 2.47 (d, 1H, $J_{OH-2}$=2.25Hz, OH).

$^{13}$C R.M.N. (100 MHz) : δ 138.47*2, 138.44, 138.4*2, 138.32*2, 138.29, 138.2, 138.05, 138, 137.9, 134.2 (13 C arom.), 128.8-127.2 (60 CH arom), 101.4 (C-1B ou C-1C, $^1J_{CH}$=171.7Hz ou $^1J_{CH}$=170.9Hz), 101 (C-1B ou C-1C et C-1D, $^1J_{CH}$=171.7Hz ou $^1J_{CH}$=170.9Hz), 87.1 (C-1A, $^1J_{CH}$=169.1Hz), 80, 79.6, 79.4, 79 (4*C-3), 77.3 (C-2A), 75.5 (C-2B ou C-2C), 75.1 ($CH_2$Ph), 75.07 (C-2B ou C-2C), 74.94 ($CH_2$Ph), 74.85*2, 74.77, 72 (4*C-4), 73.26 ($CH_2$Ph), 73.18 ($CH_2$Ph), 73.1 ($CH_2$Ph), 72.8 ($CH_2$Ph), 72.6, 72.26, 72.22, 71.5 (4*C-5), 72.3 ($CH_2$Ph), 72 ($CH_2$Ph), 71.8 ($CH_2$Ph), 69.4, 69.2, 68.9, 68.7 (4*C-6), 68.4 (C-2D).

Spectre de masse : m/z 1861.9 $(M+Na)^+$.

Analyse pour $C_{114}H_{118}O_{20}S$ (1840.258) calculée C:74.4 H:6.463 trouvée C:74.3 H:6.54

5) 8-méthoxycarbonyloctyl 2-O-(2-O-(2-O-(3,4,6-tri- O- benzyl- α- D- mannopyranosyl)- 3,4,6-tri- O- benzyl- α- D- mannopyranosyl)- 3,4,6-tri- O- benzyl- α- D- mannopyranosyl)- 3,4,6-tri- O- benzyl- α- D- mannopyranoside (18a) et 8-méthoxycarbonyloctyl 2-O-(2-O-(2-O-(3,4,6-tri- O- benzyl- α- D- mannopyranosyl)- 3,4,6-tri- O- benzyl- α- D- mannopyranosyl)- 3,4,6-tri- O- benzyl- α- D- mannopyranosyl)- 3,4,6-tri- O- benzyl- β- D- mannopyranoside (18b).

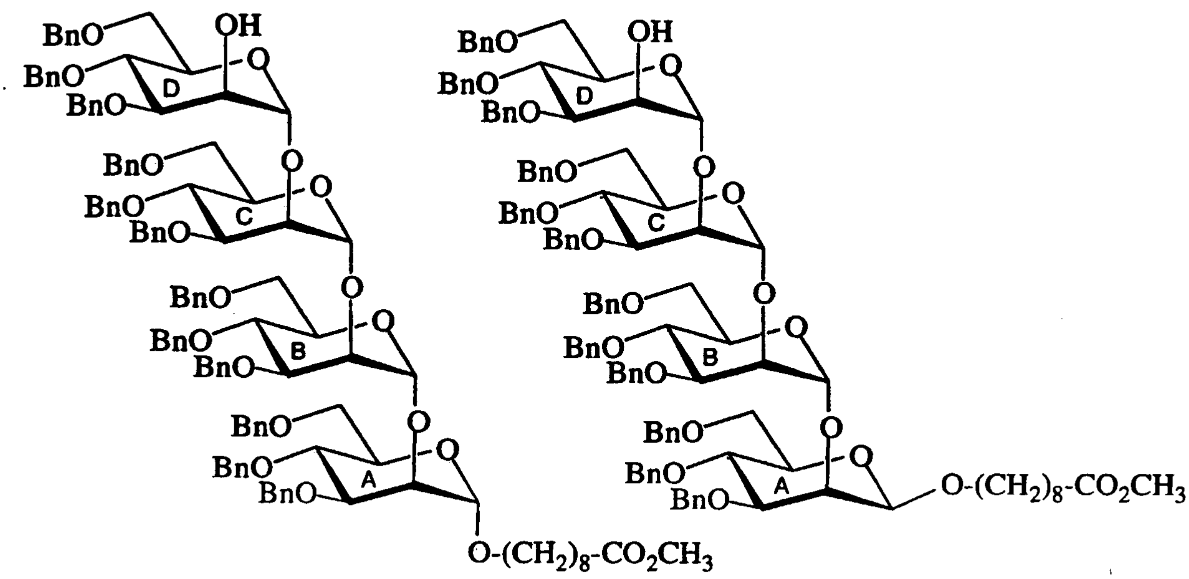

Pour le protocole expérimental, voir le composé 10.

A partir de 268 mg (146 μmol) du composé 17, les deux diastéréomères 18a α-O-connecteur (101 mg, 36%) et 18b β-O-connecteur (100 mg, 36%) sont obtenus après une chromatographie sur gel de silice (élution : cyclohexane / acétate d'éthyle=4/1) dans un rapport α/β=1/1.

Caractéristique de 18a α-O-connecteur:$[\alpha]_D$ +35 (c 0.2, chloroforme)

$^1$H-R.M.N. (400MHz, $CDCl_3$) : δ 7.36-7.23 (m, 55H, arom.), 5.3 (d, 1H, $J_{1-2}$=1.2Hz, H-1B ou H-1C), 5.27 (d, 1H, $J_{1-2}$=1.6Hz, H-1B ou H-1C), 5.18 (d, 1H, $J_{1-2}$=1.4Hz, H-1D), 4.99 (d, 1H, $J_{1-2}$=1.4Hz, H-1A), 4.89 (2d, 2H, $J_{gem}$=10.9Hz, 2 CHPh), 4.86 (d, 1H, $J_{gem}$=10.9Hz, CHPh) , 4.82 (d, 1H, $J_{gem}$=10.5Hz, CHPh), 4.72 (2d, 2H, 2 CHPh), 4.67 (d, 1H, $J_{gem}$=12.2Hz, CHPh), 4.63-4.58 (m, 8H, 8 CHPh), 4.56 (d, 1H, $J_{gem}$=12.3Hz, CHPh), 4.55 (d, 1H, $J_{gem}$=10.9Hz, CHPh), 4.54 (d, 1H, $J_{gem}$=10.9Hz, CHPh), 4.49 (d, 1H, $J_{gem}$=10.5Hz, CHPh), 4.48 (d, 1H, $J_{gem}$=11.5Hz, CHPh), 4.47 (d, 1H, $J_{gem}$=11.2Hz, CHPh), 4.4 (d, 1H, $J_{gem}$=12.2Hz, CHPh) , 4.35 (d, 1H, $J_{gem}$=11.5Hz, CHPh) , 4.21 (d, 1H, $J_{gem}$=12.3Hz, CHPh), 4.2-4.19 (m, 1H, H-2D), 4.185 (dd, 1H, $J_{2-1}$=1.6Hz et $J_{2-3}$=2.5Hz, H-2B ou H-2C), 4.15 (dd, 1H, $J_{2-1}$=1.2Hz et $J_{2-3}$=2.1Hz, H-2B ou H-2C), 4.03 (dd, 1H, $J_{2-1}$=1.4Hz et $J_{2-3}$=2.5Hz, H-2A) , 4.01-3.89 (m, 9H, 4*H-3, 3*H-5A, B, C ou D, 2*H-4A, B, C ou D), 3.84-3.72 (m, 5H, 2*H-4A, B, C ou D, 1*H-5A, B, C ou D, 2*H-6A, B, C ou D), 3.71 (s, 3H,

-C:O-$OCH_3$), 3.67-3.51 (m, 2H, 2*H-6A, B, C ou D), 3.60 (dt, 1H, $J_{CH-CH2}$=6.8Hz et $J_{gem}$=9.5Hz, - O-CH-$CH_2$-), 3.28 (dt, 1H, $J_{CH-CH2}$=6.6Hz et $J_{gem}$=9.5Hz, - O-CH-$CH_2$-), 2.44 (br, 1H, OH), 2.34 (t, 2H, $J_{3-2}$=7.6Hz, - $CH_2$C:O-$OCH_3$), 1.69-1.62 (m, 2H, -$CH_2$-), 1.55-1.48 (m, 2H, - $CH_2$-), 1.37-1.27 (m, 8H, -$(CH_2)_4$-).

$^{13}$C R.M.N. (100 MHz) : δ 174.2 (-C:O-$OCH_3$), 138.6, 138.55, 138.5*4, 138.44, 138.36*2, 138.3, 138.1, 138 (12 C arom.), 128.4-127.26 (55 CH arom), 101.1 (C-1B ou C-1C, $^1J_{CH}$=171.7Hz ou $^1J_{CH}$=172.9Hz), 100.9 (C-1B ou C-1C et C-1D, $^1J_{CH}$=171.7Hz ou $^1J_{CH}$=172.9Hz), 98.7 (C-1A, $^1J_{CH}$=168.5Hz), 80, 79.4*2, 79.1 (4*C-3), 75.8 (C-2A), 75.6 (C-2B ou C-2C), 75.13 (C-2B ou C-2C), 75.1 (2*$CH_2$Ph), 74.96 ($CH_2$Ph), 73.9*2, 74.8, 74.3 (4*C-4), 73.3 ($CH_2$Ph), 73.2 ($CH_2$Ph), 73.18 (2*$CH_2$Ph), 72.33 (2*$CH_2$Ph), 72.3, 72, 71.76, 71.73 (4*C-5), 72 ($CH_2$Ph), 71.76 ($CH_2$Ph), 71.73 ($CH_2$Ph), 69.6, 69.5, 63.35, 68.8 (4*C-6), 68.5 (C-2D), 67.65 (-O-$CH_2$-), 69.8 ($CH_2$Ph), 51.4 (-C:O-$OCH_3$), 34 (-$CH_2$-C:O-O-$CH_3$), 29.4, 29.2, 29.1, 29, 26, 24.9 (-$CH_2$-)

Spectre de masse : m/z 1934.8 (M+NH4)$^+$.

Analyse pour $C_{118}H_{132}O_{23}$ (1918.35) : calculée C:73.88 H:6.93 trouvée C:73.76 H:7.12

Caractéristique de 18b β-O-connecteur$[\alpha]_D$ +2 (c 0.2, chloroforme)

$^1$H-R.M.N. (400MHz, $CDCl_3$) : δ 7.38-7.25 (m, 55H, arom.), 5.39 (d, 1H, $J_{1-2}$=1.5Hz, H-1B ou H-1C), 5.34 (d, 1H, $J_{1-2}$=1.5Hz, H-1B ou H-1C), 5.23 (d, 1H, $J_{1-2}$=1.45Hz, H-1D), 4.9 (d, H, $J_{gem}$=10.6Hz, CHPh), 4.89 (d, 1H, $J_{gem}$=10.9Hz, CHPh), 4.87 (d, 1H, $J_{gem}$=10.6Hz, CHPh), 4.82 (d, H, $J_{gem}$=12.3Hz, CHPh), 4.81 (d, 1H, $J_{gem}$=10.1Hz, CHPh), 4.79 (d, 1H, $J_{gem}$=11.5Hz, CHPh), 4.69 (d, 1H, $J_{gem}$=12.2Hz, CHPh), 4.68-4.55 (m, 11H, 11 CHPh), 4.54 (d, 1H, $J_{gem}$=11.7Hz, CHPh), 4.52 (d, 1H, $J_{gem}$=10.6$_H$z, CHPh), 4.51 (d, 1H, $J_{gem}$=11.5Hz, CHPh), 4.48 (d, 1H, $J_{gem}$=10.1Hz, CHPh), 4.41-4.38 (m, 1H, H-5B ou H-5C), 4.38 (d, 1H, $J_{gem}$=12.2Hz, CHPh), 4.3 (s, 1H, H-1A), 4.27 (d, 1H, $J_{gem}$=12.4Hz, CHPh), 4.25-4.24 (m, 2H, H-2B et H-2C), 4.2-4.19 (m, 1H, H-2D), 4.13 (d, 1H, $J_{2-3}$=2.3Hz, H-2A), 4.09 (dd, 1H, $J_{3-2}$=2.8Hz et $J_{3-4}$=9.6Hz, H-3B ou H-3C), 4.05 (dd, 1H, $J_{3-2}$=2.8Hz et $J_{3-4}$=9.1Hz, H-3B ou H-3C), 4.047-3.88 (m, 7H, H-3D, 3*H-4B, B ou D, 3*H-5B, C ou D), 3.92-3.89 (m, 1H, -O-CH-$CH_2$-), 3.8-3.5 (m, 8H, H-4A, 4*H-6A, B, C et D, -C:O-$OCH_3$), 3.48 (dd, 1H, $J_{3-2}$=2.5Hz et $J_{3-4}$=9.4Hz, H-3A), 3.44-3.39 (m, 2H, H-5A et -O-CH-$CH_2$-), 2.44 (br, 1H, OH), 2.34 (t, 2H, $J$ 3-2=7.6Hz, -$CH_2$C:O-$OCH_3$), 1.67-1.57 (m, 4H, -$(CH_2)_2$-), 1.39-1.28 (m, 8H, -$(CH_2)_4$-).

$^{13}$C R.M.N. (100 MHz) : δ 174.2 (-C:O-$OCH_3$), 138.9, 138.77, 138.66, 138.65, 138.56*2, 138.37, 138.35, 138.12*2, 138.07, 137.9 (12 C arom.), 128.4-127.2 (55 CH arom), 100.8 (C-1D), 1.00.5 (C-1B ou C-1C), 99.9 (C-1A), 99.5 (C-1B ou C-1C), 82.3 (C-3A), 80 (C-3D) , 79.7 (C-3B ou C-3C) , 79.5 (C-3B ou C-3C), 75.6 (C-5A), 75.3 (C-2B ou C-2C), 75.02 ($CH_2$Ph), 75*2 ($CH_2$Ph), 74.93 (C-4A), 74.9 (C-2B ou C-2C), 74.88, 74.84, 74.2 (C-4B, C-4C, C-4D), 73.35 ($CH_2$Ph), 73.23 ($CH_2$Ph) , 73.21 ($CH_2$Ph), 73.17 ($CH_2$Ph), 73.12 ($CH_2$Ph), 72.5 (C-2A), 72.34 ($CH_2$Ph), 72.2, 71.6, 71.5 (C-5B, C-5C, C-5D), 72.1 ($CH_2$Ph), 72 ($CH_2$Ph), 71.8 ($CH_2$Ph), 69.7, 69.4, 63.37, 69.2 (4*C-6), 68.48 (-O-$CH_2$-), 68.46 (C-2D), 51.4 (-C:O-$OCH_3$), 34 (-$CH_2$-C:O-O-$CH_3$), 29.6, 29.2, 29.16, 29.1, 26, 24.9 (-CH2-)

Spectre de masse : m/z 1934.95 (M+NH4)$^+$.

Analyse pour $C_{118}H_{132}O_{23}$ (1918.35) : calculée C:73.88 H:6.93 trouvée C:73.84 H:7.1

6) 8-carboxyloctyl 2-O-(2-0-(2-O-(α-D-mannopyranosyl)-α-D-mannopyranosyl)-α-D-mannopyranosyl)-α-D-mannopyranoside et β-D-mannopyranoside (II, R= connecteur).

A partir des deux isomères 18a et 18b, deux composés (II R= connecteur) ont été préparés:

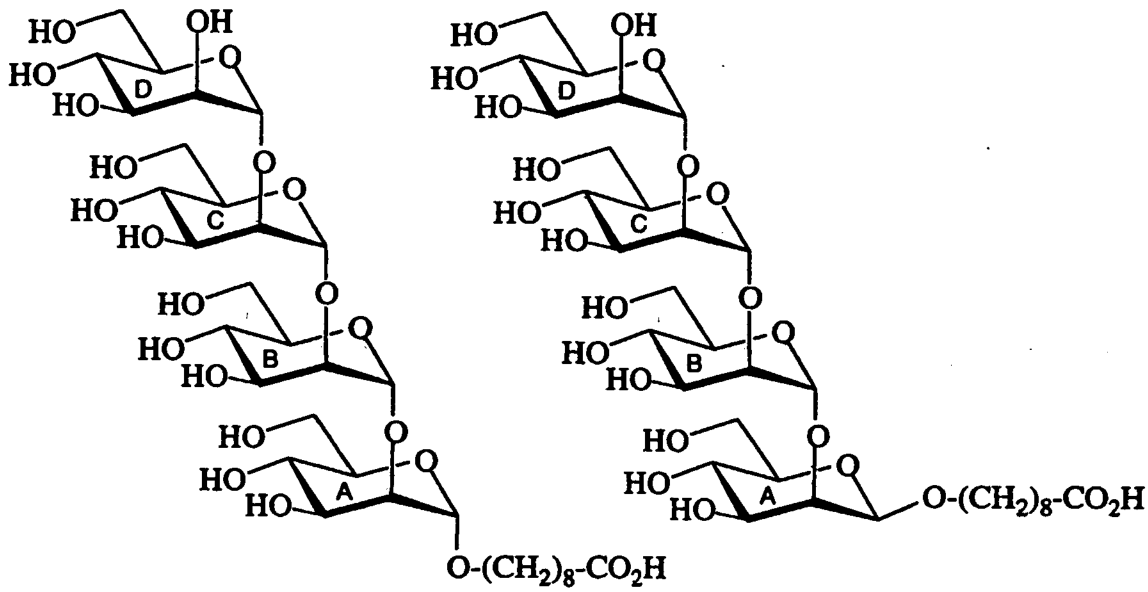

- <u>Composé α-aglycone</u>

Etape 1 : saponification

Pour le protocole expérimental, voir l'étape 2 de I (R=connecteur).

<u>18a</u> ( 90 mg, 47 μmol) , donne 70 mg (78%) de 8-carboxyloctyl 2-O-(2-O-(2-O-(3,4,6-tri-O-benzyl-_-D-mannopyranosyl)-3,4,6-tri-O-benzyl-_-D-mannopyranosyl)-3,4,6-tri-O-benzyl-_-D-mannopyranosyl)-3,4,6-tri-O-benzyl--D-mannopyranoside après une chromatographie sur gel de silice (élution:cyclohexane/acétate d'éthyle=2/1): [_]D +17 (c 1, chloroforme)
1H-R.M.N. (400MHz, CDCl3) : _7.36-7.23 (m, 55H, arom.), 5.3 (d, 1H, J 1-2=1.2Hz, H-1B ou H-1C) , 5.27 (d, 1H, J 1-2=1.6Hz, H-1B ou H-1C), 5.18 (d, 1H, J 1-2=1.4Hz, H-1D), 4.99 (d, 1H, J 1-2=1.4Hz, H-1A), Absence du pic caractéristique du méthyle de l'ester méthylique.
Spectre de masse : m/z 1920.86 (M+NH4+)+.
Analyse pour C117H130O23 (1904.32) : calculée C:73.79 H:6.88 trouvée C:73.36 H:7.12

Etape 2 : hydrogénolyse

Pour le protocole, voir étape 3 de I (R=connecteur).

A partir de 48 mg (25 _mol) du précurseur, 20 mg (Rdt=95%) sont obtenus pour le composé_-O-connecteur II (R= connecteur).
1H-R.M.N. (400MHz, D2O) du composé _-O-connecteur: _5.25 (d, 1H, J 1-2=1.6Hz, H-1B ou H-1C), 5.24 (d, 1H, J 1-2=1.5Hz, H-1B ou H-1C), 5.04 (d, 1H, J 1-2=1.3Hz, H-1D), 4.99 (d, 1H, J 1-2=2Hz, H-1A), 3.69-3.63 (m, 1H, -O-CH-CH2-), 3.48 (dt, 1H, J CH-CH2=6.2Hz et J gem=9.9Hz, -O-CH-CH2-), 2.22 (t, 2H, J 3-2=7.5Hz, -CH2COOH), 1.58-1.48 (m, 4H, -(CH2)2-), 1.3-1.24 (m, 8H, -(CH2)4-).
$^{13}C$ R.M.N. (100 MHz) : δ 102.5, 100.96, 100.91, 98.29 (4 C-1).
Spectre de masse (FAB) : calculée $C_{33}H_{58}O_{23}Na$ : m/z 845.32 obtenue : 845.37

- <u>Composé β- aglycone</u>

Etape 1 : saponification

Pour le protocole expérimental, voir l'étape 2 de I (R=connecteur).

<u>18b</u> (89 mg , 46.4 μmol) donne 64 mg (72%) de 8-carboxyloctyl 2-O-(2-O-(2-O-(3,4,6-tri-O-benzyl-α-D-mannopyranosyl)-3,4,6-tri-O-benzyl-α-D-mannopyranosyl)-3,4,6-tri-O-benzyl-α-D-mannopyranosyl)- 3,4,6-tri-O-benzyl-β-D-mannopyranoside : $[\alpha]_D$ +19 (c 1, chloroforme) $^1$H-R.M.N. (400MHz, $CDCl_3$) : δ 7.38-7.25 (m, 55H, arom.), 5.39 (d, 1H, $J_{1-2}$=l.SHz, H-1B ou H-1C), 5.34 (d, 1H, $J_{1-2}$=1.5Hz, H-1B ou H-1C), 5.23 (d, 1H, $J_{1-2}$=1.45Hz, H-1D), 4.3 (s, 1H,

H-1A), Absence du pic caractéristique du méthyle de l'ester méthylique.
Spectre de masse : m/z 1920.9 (M+NH4+)$^+$.
Analyse pour $C_{117}H_{130}O_{23}$ (1904.32) : calculée C:73.79 H:6.88 trouvée C:73.37 H:7.28

Etape 2 : hydrogénolyse

Pour le protocole, voir étape 3 de I (R=connecteur).

18 mg (92%) II (R= connecteur) β-O-connecteur sont obtenu à partir de 45 mg (23.6 μmol):
$^1$H-R.M.N. (400MHz, $D_2O$) du composé g-O-connecteur: δ5.33 (d, 1H, $J_{1-2}$=1.3Hz, H-1B ou H-1C), 5.24 (d, 1H, $J_{1-2}$=1.8Hz, H-1B ou H-1C), 4.98 (d, 1H, $J_{1-2}$=1. 9Hz, H-1D), 4.63 (s, 1H, H-1A), 3.84-3.79 (m, 1H, -O-CH-$CH_2$-), 3.53 (dt, 1H, $J_{CH-CH2}$=5.8Hz et $J_{gem}$=9.9Hz, -O-CH-$CH_2$-), 2.22 (t, 2H, $J_{3-2}$=7.5Hz, -$CH_2$COOH), 1.59-1.49 (m, 4H, -$(CH_2)_2$-), 1.3-1.24 (m, 8H, -$(CH_2)_4$-).
$^{13}$C R.M.N. (100 MHz) : δ 102.5, 100.95, 100.91, 99.99 (4 C-1).
Spectre de masse (FAB) : calculée $C_{33}H_{58}O_{23}Na$ : m/z 845.32 obtenue : 845.4

7) 2-O-(2-O-(2-O-(3,4,6-tri-O-benzyl-α-D-mannopyranosyl)-3,4,6-tri-O-benzyl-α-D-mannopyranosyl)-3,4,6-tri-O-benzyl-α-D-mannopyranosyl)-3,4,6-tri-O-benzyl-D-mannopyranose (19).

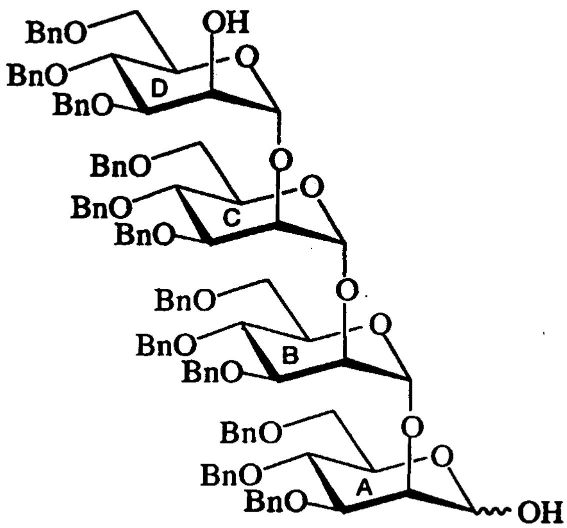

Pour le protocole expérimental, voir l'étape 2 de I (R=H). A partir de 105 mg (57 μmol) du composé 17, 84 mg (85%) du composé 19 (α/β=87/13) sont obtenus après une chromatographie sur gel de silice avec élution:cyclohexane/acétate d'éthyle=3/1 puis 2/1.
$^1$H-R.M.N. (400MHz, $CDCl_3$) du composé α : 87.4-7.27(m, 60H, arom.), 5.38-5.37 (m, 1H, H-1A), 5.34 (d, 1H, $J_{1-2}$=1.5Hz, H-1B ou H-1C), 5.32 (d, 1H, $J_{1-2}$=1.7Hz, H-1B ou H-1C), 5.23 (d, 1H, $J_{1-2}$=1.6Hz, H-1D), 2.83 (br, 1H, OH), 2.51 (br, 1H, OH).
$^{13}$C R.M.N. (100 MHz) : δ 100.94 (C-1D), 100.9 (C-1B ou C-1C), 100.85 (C-1B ou C-1C), 93.3 (C-1A).
Spectre de masse : m/z 1769.54 (M+Na)$^+$.
Analyse pour $C_{117}H_{130}O_{23}$ (1748.1) : calculée C:74.2 H:6.57 trouvée C:74.15 H:6.7

8) 2-O-(2-O-(2-O-(α-D-mannopyranosyl)-α-D-manno pyranosyl)-α-D-mannopyranosyl)-D-mannolpyranose II (R=H).

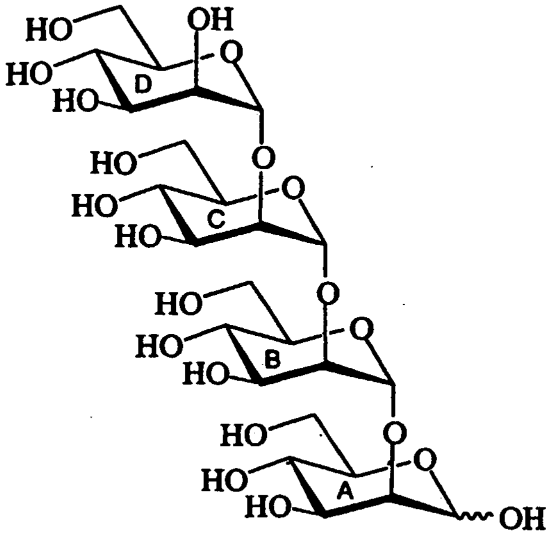

Pour le protocole expérimental, voir l'étape 3 du composé I (R=connecteur).

30 mg (18 μmol) du composé précédent donnent 18 mg (Rdt=95%) du composé II (R=H) après lyophilisation. $^1$H-R.M.N. (400MHz, $D_2O$) du composé α-OH: δ5.23(s, 1H, H-1A), 5.15 (se, 2H, H-1B et H-1C), 4.91 (d, 1H, $J_{1-2}$=1.6Hz, H-1D).
Spectre de masse : calculée $C_{24}H_{42}O_{21}Na$ : m/z 689.21 obtenue 689.41

Exemple 3 Préparation de D-Man α(1-3) D-Man α(1-2) D-Man α(1-2) D-Man α (1-2), de formule (III).

I - Schéma réactionnel.

La figure 3 en annexe représente le schéma réactionnel de la préparation de D-Man α(1-3) D-Man α(1-2) D-Man α(1-2) D-Man α (1-2) de formule (III). Celui-ci est préparé par condensation d'un bloc α(1-3) avec un groupe partant SPh et un bloc α(1-2) dont l'un avec connecteur conformément aux réactions (a''') à (d''') ci-dessous.

La réaction (a''') est réalisée dans les conditions suivantes : TfOTMS, tamis moléculaire 4 Angstrom, $CH_2Cl_2$, -20°C.

La réaction (b''') est réalisée dans les conditions suivantes : MeONa, MeOH.

La réaction (c''') est réalisée dans les conditions suivantes : NIS, TfOH, , tamis moléculaire 4 Angstrom, $CH_2Cl_2$, -20°C.

La réaction (d''') est réalisée dans les conditions suivantes : 1-NaOH, THF, eau, 2-$H_2$, Pd/C, MeOH, AcOEt.

II - Protocole expérimental.

Les composés 22 (Y-M. Zhang, J.-M. Mallet, P. Sinaÿ.Carbohydr. Res. 236, (1992), 73-88.) et 21 (P. J. Garegg, H. Hultberg, T Norberg, Carbohydr. Res. 96 (1981) 59-64) sont préparés selon les protocoles de la littérature.

1) 8-méthoxycarbonyloctyl 2-O-(2-O-benzoyl-3,4,6-tri-O-benzyl-α-D-mannopyranosyl)-3,4,6-tri-O-benzyl-α-D-mannopyranoside (23).

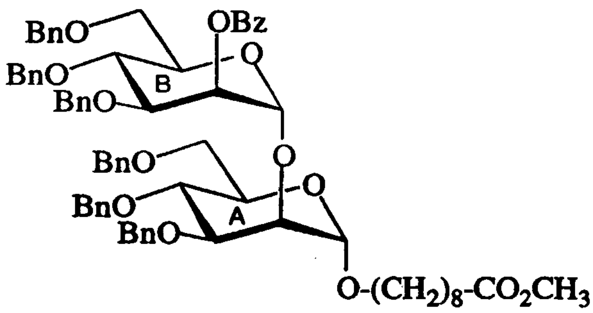

430 mg (665 µmol, 1 éq) de phényl 2-O-benzoyl-3,4,6-tri-O-benzyl-1-thio-a-D-mannopyranoside (22) (Y-M. Zhang, J.-M. Mallet, P. Sinaÿ.Carbohydr. Res. 236, (1992), 73-88.) , 412 mg (1 éq) de 8-méthoxycarbonyl 3,4,6-tri-O-benzyl-1-α-D-mannopyranoside (21) (P. J. Garegg, H. Hultberg, T Norberg, Carbohydr. Res. 96 (1981) 59-64) et 1 g de tamis moléculaire sont mis en suspension dans 9 mL de dichlorométhane anhydre et sous atmosphère d'argon. Après 30 mn d'agitation à température ambiante, la solution est refroidie à -20°C. 306 mg (2.2 éq) de N-iodosuccinimide et 11.7 µL (0.2 éq) d'acide trifluorométhanesulfonique sont alors successivement ajoutés. Après 30 mn à -20°C, le milieu reactionnel est neutralisé par une solution d'hydrogénocarbonate de sodium. Le tout est filtré sur un lit de célite, la phase organique est lavée par une solution de thiosulfate de sodium et par une solution aqueuse de NaCl. Celle-ci est ensuite séchée sur sulfate de magnésium, filtrée et concentrée sous pression réduite. Une chromatographie du brut (élution: cyclohexane/acétate d'éthyle=4/1) fournit 615 mg (80%) du produit 23 sous la forme d'une huile incolore. $[\alpha]_D$ +5.2 (c 0.56, chloroforme) $^1$H-R.M.N. (400MHz, $CDCl_3$) : δ 8.15-7.25 (m, 35H, arom.), 5.85 (dd, 1H, $J_{2-1}$=1.8Hz et $J_{2-3}$=3.3Hz, H-2B), 5.27 (d, 1H, $J_{1-2}$=1.8Hz, H-1B), 4.96 (d, 1H, $J_{1-2}$=1.66Hz, H-1A), 4.93 (d, 1H, $J_{gem}$=10.7Hz, CHPh), 4.92 (d, 1H, $J_{gem}$=10.9Hz, CHPh), 4.83 (d, 1H, $J_{gem}$=11.1Hz, CHPh), 4.78 (d, 1H, $J_{gem}$=12Hz, CHPh), 4.76 (s, 2H, $CH_2$ Ph), 4.74 (d, 1H, $J_{gem}$=12.2Hz, CHPh), 4.62 (d, 1H, $J_{gem}$=12.2Hz, CHPh), 4.62 (d, 1H, $J_{gem}$=10.7Hz, CHPh), 4.58 (d, 1H, $J_{gem}$=12Hz, CHPh), 4.57 (d, 1H, $J_{gem}$=10.9Hz, CHPh), 4.51 (d, 1H, $J_{gem}$=11.1Hz, CHPh), 4.19 (dd, 1H, $J_{3-2}$=3.3Hz et $J_{3-4}$=9Hz, H-3B), 4.12-4.09 (m, 2H, H-5B et H-4B), 4.07 (dd, 1H, $J_{2-1}$=1.66Hz et $J_{2-3}$=2.9Hz, H-2A), 4 (dd, 1H, $J_{3-2}$=2.9Hz et $J_{3-4}$=9.2Hz, H-3A), 3.94 (dd, 1H, $J_{6b-6a}$=10.5Hz et $J_{6b-5}$=3Hz, H-6Bb), 3.92 (t, 1H, $J_{4-3}$=$J_{4-5}$=9.2Hz, H-4A), 3.85-3.77 (m, 4H, H-6A, H-6Ba et H-5A), 3.71 (s, 3H, -C:O-$OCH_3$), 3.67 (dt, 1H, $J_{gem}$=9.5Hz et $J_{CH-CH2}$=6·7Hz, -O-CH-$CH_2$-), 3.35 (dt, 1H, $J_{gem}$=9.5Hz et $J_{CH-CH2}$=6.7Hz, -O-CH-$CH_2$-), 2.36 (t, 2H, $J_{CH2-CH2}$=7-SHz, -$CH_2$-$CO_2CH_3$), 1.69-1.65, 1.57-1.54 et 1.35-1.33 (m, 12H, -$CH_2$-).

$^{13}$C R.M.N. (100 MHz) : δ 174.2 (-C:O-$OCH_3$), 165.4 (-O-C:O), 138.5, 138.4, 138.32, 138.3, 138.26, 137.98, 129.9 (7 C arom.), 133-127.35 (35 CH arom), 99.5 (C-1B), 98.6 (C-1A), 79.73 (C-3A), 78.07 (C-3B), 75.2 (C-2A), 75.1 ($CH_2$Ph), 75 ($CH_2$Ph), 74.6 (C-4B), 74.3 (C-4A), 73.3 ($CH_2$Ph), 73.2 ($CH_2$Ph), 72.1 ($CH_2$Ph), 71.9 (C-5B), 71.7 (C-5A), 71.6 ($CH_2$Ph), 69.2 (C-6A), 69.15 (C-6B), 68.98 (C-2B), 67.64 (-O-$CH_2$-), 51.4 (-C:O-$OCH_3$), 34 (-$CH_2$-$COOCH_3$), 29.36, 29.17, 29.11, 29, 26, 24.9 (-CH2-).

Spectre de masse : m/z 1174.5 (M+NH4)$^+$.

Analyse pour $C_{71}H_{80}O_{14}$ (1157.40) : calculée C : 73.68 H:6.96 trouvée C:73.61 H:7.11

2) 8-méthoxycarbonyloctyl 2-O-(3,4,6-tri-o-benzyl-α-D-mannopyranosyl)-3,4,6-tri-O-benzyl-α-D-mannopyranoside (24).

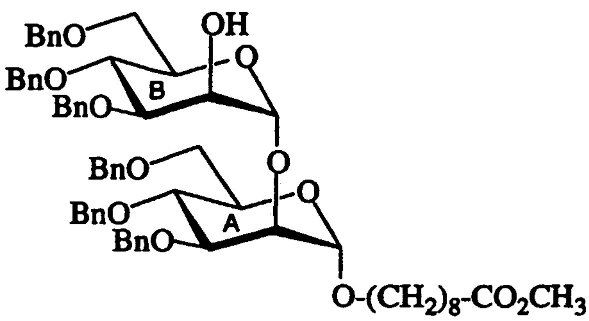

540 mg (467 µmol) du composé 23 sont dissous dans 5 mL d'un mélange de solvant méthanol/dichlorométhane

thane=1/1. Du sodium (cat.) est alors ajouté au mélange. Après 1 heure à température ambiante, la solution est neutralisée par de la résine amberlite IR 120 (H+). Le tout est filtré et concentré sous vide. Une chromatographie du brut (élution: cyclohexane/acétate d'éthyle=2.75/1) fournit 442 mg (Rdt=90%) du produit 24 sous la forme d'une huile incolore. $[\alpha]_D$ +34 (c 0.71, chloroforme)

$^1$H-R.M.N. (400MHz, $CDCl_3$) : δ 7.4-7.3 (m, 30H, arom.), 5.21 (d, 1H, $J_{1-2}$=1.45Hz, H-1B), 4.95 (d, 1H, $J_{1-2}$=1.75Hz, H-1A), 4.89 (d, 1H, $J_{gem}$=10.6Hz, CHPh), 4.87 (d, 1H, $J_{gem}$=10.9Hz, CHPh), 4.75 (d, 1H, $J_{gem}$=12.2Hz, CHPh), 4.75 (d, 1H, $J_{gem}$=11.6Hz, CHPh), 4.71 (d, 1H, $J_{gem}$=11.6Hz, CHPh), 4.69 (d, 1H, $J_{gem}$=12.1Hz, CHPh), 4.64 (d, 1H, $J_{gem}$=11.4Hz, CHPh), 4.59 (d, 1H, $J_{gem}$=10.6Hz, CHPh), 4.59 (d, 1H, $J_{gem}$=12.2Hz, CHPh), 4.58 (d, 1H, $J_{gem}$=11.4Hz, CHPh), 4.56 (d, 1H, $J_{gem}$=12.1Hz, CHPh), 4.54 (d, 1H, $J_{gem}$=10.9Hz, CHPh), 4.2-4.17 (m, 1H, H-2B), 4.08 (dd, 1H, $J_{2-1}$=1.75Hz et $J_{2-3}$=2.9Hz, H-2A), 4.03-3.99 (m, 1H, H-5B), 3.99 (dd, 1H, $J_{3-2}$=2.9Hz et $J_{3-4}$=9.3Hz, H-3A), 3.93 (dd, 1H, $J_{3-2}$=3.2Hz et $J_{3-4}$=9.1Hz, H-3B) , 3 .89 (t, 1H, $J_{4-3}$=$J_{4-5}$=9.3Hz, H-4A), 3.86(t, 1H, $J_{4-3}$= $J_{4-5}$=9.1Hz, H-4B), 3.86 (dd, 1H, $J_{6b-6a}$=11.1Hz et $J_{6b-5}$=4. 95Hz, H-6Ab), 3.82-3.75 (m, 4H, H-6Aa, H-6B et H-5A), 3.71 (s, 3H, -C:O-$OCH_3$), 3.65 (dt, 1H, $J_{gem}$=9.5Hz et $J_{CH-CH2}$=6.8Hz, -O-CH-$CH_2$-), 3.3 (dt, 1H, $J_{gem}$=9.5Hz et $J_{CH-CH2}$=6.8Hz, -O-CH-$CH_2$-), 2.51 (d, 1H, $J_{OH-2}$=1.9Hz, OH), 2.34 (t, 2H, $J_{CH2-CH2}$=7.6Hz, -CH2-$CO2CH_3$), 1.7-1.63, 1.56-1.49 et 1.37-1.27 (m, 12H, -$CH_2$-).

$^{13}$C R.M.N. (100 MHz) : δ 174.3 (-C:O-$OCH_3$), 138.5, 138.35, 138.3, 138.25, 138.1, 137.9 (6 C arom.), 128.4-127.3 (30 CH arom), 101 (C-1B), 98.7 (C-1A), 79.92 (C-3B), 79.79 (C-3A), 75.1 ($CH_2$Ph), 74.95 ($CH_2$Ph), 74.93 (C-2A), 74.76 (C-4A), 74.3 (C-4B) , 73.3 ($CH_2$Ph), 73.2 ($CH_2$Ph) , 72.8 ($CH_2$Ph), 72 ($CH_2$Ph), 71.7 (C-5B), 71.7 (C-5A), 69.2 (C-6A), 69 (C-6B), 68.45 (C-2B), 67.63 (-O-$CH_2$-), 51.4 (-C:O-$OCH_3$), 34 (-CH2-$COOCH_3$), 29.4, 29.2, 29.1, 29, 26, 24.9 (-CH2-).

Spectre de masse : m/z 1070.5 $(M+NH4)^+$.

Analyse pour $C_{64}H_{76}O_{13}$ (1053.30) : calculée C:72.98 H:7.27 trouvée C:72.89 H:7.43

3) Phenyl 2-O-acétyl-4,6-O-benzylidène-1-thio-α-D-mannopyranoside (25).

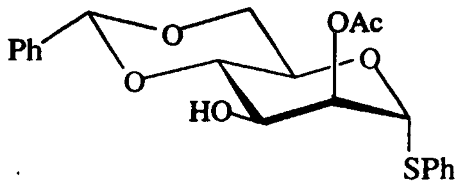

2 g (5.5 mmol, 1 éq) de phényl 4,6-O-benzylidène-1-thio-α-D-mannopyranoside 1 et 292 mg (1.2 mmol, 0.2 éq) d'acide 10-DL-camphorsulphonique sont dissous dans 10 mL de triéthyl orthoacétate. Après 30 mn à température ambiante, 14.4 mL d'acide acétique 80% sont ajoutés à la solution préalablement refroidie à 0°C. Après 1 heure en laissant remonter la température à l'ambiante, le tout est concentré et chromatographié sur gel de silice (élution:cyclohexane/acétate d'éthyle=2/1). 1.67 g (75%) du composé **25** sont obtenus, après évaporation des solvants, sous forme d'une poudre blanche. pf : 157-158°C; $[\alpha]_D$ +169 (c 1.05, chloroforme)

$^1$H-R.M.N. (400MHz, $CDCl_3$) : δ 7.56-7.3 (m, 10H, arom.), 5.66 (s, 1H, by), 5.52 (s, 1H, H-1), 5.51 (dd, 1H, $J_{2-1}$=1.3Hz et $J_{2-3}$=3.3Hz, H-2), 4.41 (ddd, 1H, $J_{5-4}$=9.7Hz, $J_{5-6a}$=10.3Hz et $J_{5-6b}$=4.9Hz, H-5), 4.29 (dd, 1H, $J_{6b-6a}$=10.3Hz et $J_{6b-5}$=4.9Hz, H-6b), 4.28-4.25 (m, 1H, H-3), 4.04(t, 1H, $J_{4-3}$=$J_{4-5}$=9.7Hz, H-4), 3.89 (t, 1H, $J_{6b-6a}$=$J_{6b-5}$=10.3Hz, H-6a), 2.65 (d, 1H, $J_{OH-3}$=3.5Hz, OH), 2.21 (s, 3H, O-C:O-$CH_3$).

$^{13}$C R.M.N. (100 MHz) : δ 170.3 (-O-C:O-$CH_3$), 136.9, 133 (2 C arom.), 132-126.2 (10 CH arom.), 102.2 (by), 86.8 (C-1), 79 (C-4), 73.5 (C-2), 68.3 (C-6), 67.7 (C-3), 64.5 (C-5A), 20.9 (-O-C:O-$CH_3$).

Spectre de masse : m/z 403.2 $(M+H)^+$.

Analyse pour $C_{21}H_{22}O_6S$ (402.46) calculée C:62.67 H:5.509 trouvée C:62.66 H:5.54

4) Phényl 3-O-(2-O-acétyl-3,4,6-tri-O-benzyl-α-D-mannopyranosyl)-2-O-acétyl-4,6-O-benzylidène-1-thio-α-D-mannopyranoside (26).

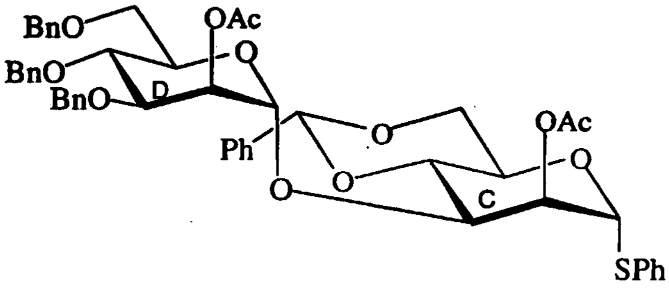

731 mg (1.15 mmol; 1.1 éq) de O-(2-O-acétyl-3,4,6-tri-O-benzyl-α-D-mannopyranose) trichloroacétimidate **(12),** 420 mg (1.044 mmol, 1 éq) du composé **25** et 1.3 g de tamis moléculaire sont mis en suspension dans 12 mL de dichlorométhane anhydre et sont maintenus sous atmosphère d'argon. Après 30 mn d'agitation à température ambiante, la solution est refroidie à -20°C et 22 μL (0.1 éq) de trifluorométhanesulfonate de triméthylsilyle sont injectés. Après 1 heure d'agitation, l'ensemble est neutralisé par une solution d'hydrogénocarbonate de sodium, filtré sur célite. la phase organique séparée est lavée par une solution aqueuse de NaCl, séchée sur sulfate de magnésium, filtrée et concentrée sous vide. Le brut est chromatographié sur gel de silice (élution:cyclohexane/acétate d'éthyle=4/1) et 626 mg (Rdt=68%) du composé **26** sont ainsi isolés sous la forme d'une mousse blanche.pf : 58-59°C; $[\alpha]_D$ +119 (c 0.55, chloroforme)
$^1$H-R.M.N. (400MHz, $CDCl_3$) : δ 7.47-7.25 (m, 25H, arom.), 5.69 (s, 1H, by), 5.55 (dd, 1H, $J_{2\text{-}1}$=1.8Hz et $J_{2\text{-}3}$=2 .7Hz, H-2D), 5.52 (dd, 1H, $J_{2\text{-}1}$=1.3Hz et $J_{2\text{-}3}$=3.4Hz, H-2C), 5.49 (d, 1H, $J$ 1-2=1.3Hz, H-1C), 5.34 (d, 1H, $J_{1\text{-}2}$=1.8Hz, H-1D), 4.89 (d, 1H, $J_{gem}$=10.9Hz, CHPh), 4.77 (d, 1H, $J_{gem}$=12.2Hz, CHPh), 4.74 (d, 1H, $J_{gem}$=11.4Hz, CHPh), 4.56 (d, 1H, $J_{gem}$=12.2Hz, CHPh), 4.56 (d, 1H, $J_{gem}$=11.4Hz, CHPh), 4.54 (d, 1H, $J_{gem}$=10.9Hz, CHPh), 4.42 (ddd, 1H, $J_{5\text{-}4}$=9.8Hz, $J_{5\text{-}6b}$=4.9Hz et $J_{5\text{-}6a}$=10.3Hz, H-5C), 4.4 (dd, 1H, $J_{3\text{-}2}$=3.4Hz et $J_{3\text{-}4}$=9.8Hz, H-3C), 4.3 (dd, 1H, $J_{6b\text{-}6a}$=10.3Hz et $J_{6b\text{-}5}$=4.9Hz, H-6Cb), 4.2 (t, 1H, $J_{4\text{-}3}$=$J_{4\text{-}5}$=9.8Hz, H-4C), 3.97-3.87 (m, 4H, H-5D, H-4D, H-3D et H-6Ca), 3.86 (dd, 1H, $J_{6b\text{-}6a}$=12Hz et $J_{6b\text{-}5}$=3-9Hz, H-6Db), 3.78 (dd, 1H, $J_{6a\text{-}6b}$=12Hz et $J_{6a\text{-}5}$=3.3Hz, H-6Da), 2.21 et 2.16 (2s, 6H, 2 O-C:O-$CH_3$).
$^{13}$C R.M.N. (100 MHz) : δ 170.1 et 169.7 (2 -O-C:O-$CH_3$), 138.4, 138.2, 137.9, 136.9, 133
(C arom.), 132-125.9 (25 CH arom.), 101.3 (by), 98.8 (C-1D), 86.8 (C-1C), 78.9 (C-4C), 75.6, 74.1 et 72.1 (C-3D, C-4D et C-5D), 74.9 ($CH_2$Ph), 73.4 ($CH_2$Ph), 73.1 (C-2C), 71.7 ($CH_2$Ph), 70.8 (C-3C), 68.6 (C-6D), 68.4 (C-2D), 68.2 (C-6C), 64.6 (C-5C), 21 et 20.8 (2 -O-C:O-$CH_3$).
Spectre de masse : m/z 894.3 $(M+NH_4)^+$.
Analyse pour $C_{50}H_{52}O_{12}S$ (877.02) : calculée C:68.47 H:5.976 trouvée C:68.36 H:6.15

5) 8-méthoxycarbonyloctyl 2-O-(2-O-(3-O-(2-O-acétyl-3,4,6-tri-O-benzyl-α-D-mannopyranosyl)-2-O-acétyl-4,6-O-benzylidène-α-D-mannopyranosyl)-3,4,6-tri-O-benzyl-α-D-mannopyranosyl)-3,4,6-tri-O-benzyl-α-D-mannopyranoside (27).

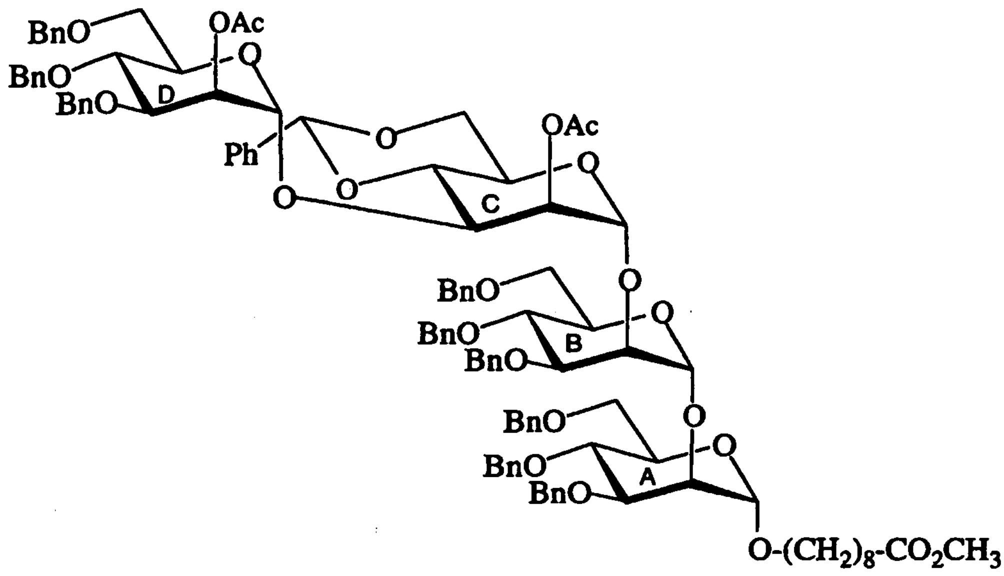

158 mg (180 μmol, 1 éq) du composé **26,** 186 mg (1 éq) du composé 24 et 500 mg de tamis moléculaire sont mis en solution dans 5 mL de dichlorométhane anhydre et sont maintenus sous atmosphère d'argon. Après 30 mn d'agitation, la solution est refroidie à -20°C et 81 mg (2 éq) de N-iodosuccinimide ainsi que 4 μL (0.3 éq) d'acide trifluorométhanesulfonique sont additionnés au milieu. Après 30 mn d'agitation, le tout est neutralisé par une solution d'hydrogénocarbonate de sodium et flitré sur un lit de célite. La phase organique est alors lavée par une solution de thiosulfate de sodium, par une solution aqueuse de NaCl, séchée sur sulfate de magnésium, filtrée et concentrée sous pression réduite. Le brut est chromatographié sur gel de silice (élution:cyclohexane/acétate d'éthyle=5/1) et 236 mg (Rdt=72%) du produit **27** sont ainsi isolés sous la forme d'une huile incolore.$[\alpha]_D$ +29 (c 0.85, chloroforme)
$^1$H-R.M.N. (400MHz, $CDCl_3$) : δ 7.38-7.25 (m, 50H, arom.), 5.66 (s, 1H, by), 5.56 (dd, 1H, $J_{2-1}$=1.5Hz et $J_{2-3}$=3Hz, H-2D), 5.44 (dd, 1H, $J_{2-1}$=1.3Hz et $J_{2-3}$=3.5Hz, H-2C), 5.32 (se, 1H, H-1D), 5.2 (se, 1H, H-1B), 5.04 (se, 1H, H-1C), 4.94 (se, 1H, H-1A), 4.88 (d, 1H, $J_{gem}$=10.6Hz, CHPh), 4.88 (d, 1H, $J_{gem}$=10.7Hz, CHPh), 4.87 (d, 1H, $J_{gem}$=10.9Hz, CHPh) , 4.75 (d, 1H, $J_{gem}$=12.4Hz, CHPh), 4.75 (d, 1H, $J_{gem}$=11.2Hz, CHPh), 4.72 (s, 2H, $CH_2$ Ph), 4.67 (d, 1H, *J*gem=12.3Hz, CHPh), 4.67 (d, 1H, J gem=12Hz, CHPh), 4.61 (d, 1H, J gem=12.4Hz, CHPh), 4.6 (d, 1H, J gem=10.6Hz, CHPh), 4.6 (d, 1H, J gem=12Hz, CHPh), 4.58 (s, 2H, CH2Ph), 4.57 (d, 1H, J gem=11.2Hz, CHPh), 4.53 (d, 1H, J gem=10.9Hz, CHPh), 4.52 (d, 1H, J gem=10.7Hz, CHPh), 4.33 (d, 1H, J gem=12.3Hz, CHPh), 4.43 (dd, 1H, J 3-2=3.5Hz et J 3-4=9.3Hz, H-3C), 4.21 (dd, 1H, J 6b-6a=10.4Hz et J 6b-5=4.5Hz, H-6Cb), 4.14-4.12 (m, 1H, H-2B), 4.09 (t, 1H, J 4-3=J 4-5=9.3Hz, H-4C), 4.07-4.04 (m, 1H, H-5C), 4.03 (t, 1H, J 4-3=J 4-5=9.9Hz, H-4D), 4.01-4 (m, 1H, H-2A), 3.98-3.96 (m, 1H, H-3B) , 3.93 (dd, 1H, J 3-2=3Hz et J 3-4=9.9Hz, H-3D), 3.88-3.85 (m, 1H, H-5D), 3.71 (s, 3H, -C:O-OCH3), 3.62-3.59 (m, 1H, H-6Da), 3.6-3.56 (m, 1H, -O-CH-CH2-), 3.25 (dt, 1H, J gem=9.4Hz et J CH-CH2=6.6Hz, -O-CH-CH2-), 2.34 (t, 2H, J CH2-CH2=7.5Hz, -CH2-CO2CH3), 2.14 et 2.13 (2s, 6H, 2 O-C:O-CH3), 1.7-1.62, 1.54-1.47 et 1.35-1.25 (m, 12H, -CH2-).
13C R.M.N. (100 MHz) : _ 174.3 (-C:O-OCH3), 170.1 et 169.5 (2 -O-C:O-CH3), 138.6, 138.55, 138.3*2, 138.29*2, 138.23, 138.2, 137.98, 137 (10 C arom.), 128.7-125.9 (50 CH arom.), 101.2 (by), 100.6 (C-1B, $^1J_{CH}$=171.3Hz), 99.8 (C-1C, $^1J_{CH}$=172.2Hz), 98.8 (C-1D, $^1J_{CH}$=174Hz), 98.6 (C-1A, $^1J_{CH}$=170.5Hz), 79.3 (C-3B), 78.9 (C-4C), 77.7 (C-3D), 75.56 (C-2A) , 75.4 (C-2B) , 75.13 ($CH_2$Ph) ; 75.06 ($CH_2$Ph), 74.87 ($CH_2$Ph), 73.9 (C-4D), 73.24 ($CH_2$Ph), 73.22 ($CH_2$Ph), 73.2 ($CH_2$Ph), 72.17 ($CH_2$Ph), 72.09 ($CH_2$Ph), 72 (C-5D), 71.7 ($CH_2$Ph), 71.4 (C-2C), 70.8 (C-3C), 68.05 (C-2D), 68.4 (C-6C), 67.63 (-O-$CH_2$-), 51.4(-C:O-$OCH_3$), 34 (-$CH_2$-$COOCH_3$), 29.4, 29.2, 29.1, 29, 26, 24.9 (-CH2-), 21 et 20.8 (2 -OC:O-CH3) .
Spectre de masse : m/z 1836.5 $(M+NH4)^+$.
Analyse pour $C_{108}H_{122}O_{25}$ (1820.026) : calculée C:71.27 H:6.75 trouvée C:71.08 H:6.94

6) 8-carboxyloctyl 2-O-(2-O-(3-O-(α-D-mannopyranosyl)-α-D-mannopyranosyl)-α-D-mannopyranosyl)-α-D-mannopyranoside III.

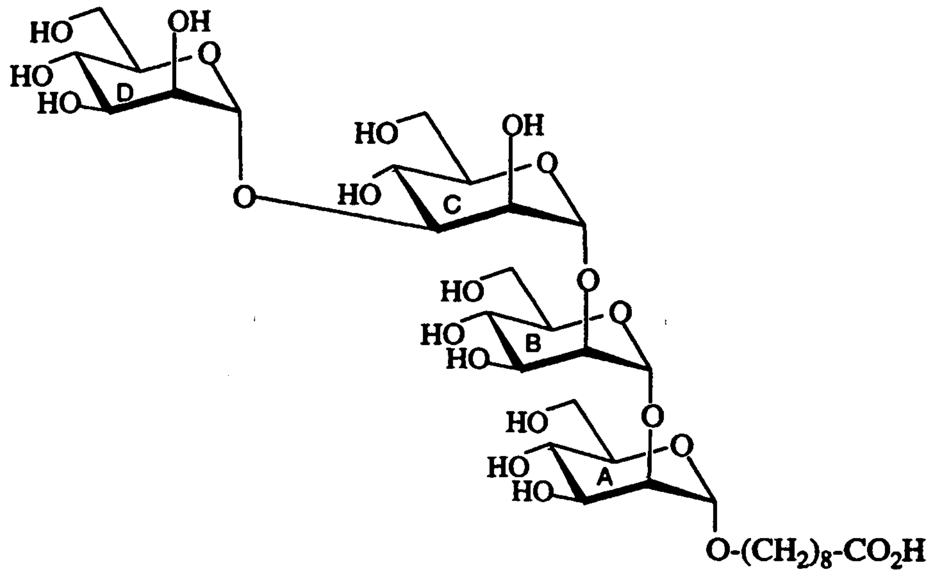

Etape 1 : saponification

200 mg (110 µmol, 1 éq) du précurseur 27 sont dissous dans 10 mL d'un mélange tétrahydrofurane/solution d'hydroxyde de sodium (0.1) (1/1). Après une nuit à reflux, l'ensemble est acidifié et extrait trois fois par du dichlorométhane.La phase organique est alors séchée sur sulfate de magnésium, filtrée et concentrée sous vide. Le produit brut est chromatographié sur gel de silice (élution:cyclohexane/acétate d'éthyle=1.5/1) et 155 mg (Rdt=82%) du composé sont ainsi isolés sous la forme d'une huile incolore.

8-carboxyloctyl 2-O-(2-O-(3-O-(3,4,6-tri-O-benzyl-α-D-mannopyranosyl)-4,6-O-benzylidène-α-D-mannopyranosyl)-3,4,6-tri-O-benzyl-α-D-mannopyranosyl)-3,4,6-tri-O-benzyl-α-D-mannopyranoside $[\alpha]_D$ +33.3 (c 0.52, chloroforme)

$^1$H-R.M.N. (400MHz, $CDCl_3$) : δ 7.5-7.25 (m, 50H, arom.), 5.6 (s, 1H, by), 5.21 (d, 1H, $J_{1-2}$=1.3Hz, H-1B), 5.16 (d, 1H, $J_{1-2}$=1.45Hz, H-1D), 5.12 (d, 1H, $J_{1-2}$=1.1Hz, H-1C), 4.97 (d, 1H, $J_{1-2}$=1.2Hz, H-1A), 4.42-4.41 (m, 1H, H-2C), 4.18-4.17 (m, 1H, H-2B), 4.08 (m, 1H, H-2D), 4 (dd, 1H, $J_{2-1}$=1.2Hz et $J_{2-3}$=3.1Hz, H-2A), 3.6 (dt, 1H, $J_{gem}$=9.5Hz et $J_{CH-CH2}$=6.5Hz, -O-CH-CH2-), 3.27 (dt, 1H, $J_{gem}$=9.5Hz et $J_{CH-CH2}$=6.5Hz, -O-CH-$CH_2$-), 2.35 (t, 2H, $J_{CH2-CH2}$=7.5Hz, -CH2-$CO_2$H), 1.7-1.6, 1.54-1.47 et 1.35-1.25 (m, 12H, -$CH_2$-). Absence des trois pics correspondant aux méthyles des esters.

$^{13}$C R.M.N. (100 MHz) : δ 178.5 (-$CO_2$H), 102.3 (C-1C), 101.7 (by), 100.9 (C-1B), 99.8 (C-1D), 98.5 (C-1A), 75.7 (C-2A), 74.8 (C-2B), 69.65 (C-2C), 68.5 (C-2D), 67.6 (-O-$CH_2$-), 33.8 (-$CH_2$-COOH), 29.3, 29, 28.9, 28.7, 25.9, 24.5 (-CH2-), 21 et 20.8 (2 -O-C:O-$CH_3$).

Spectre de masse : m/z 1738.9 $(M+NH4)^+$.

Analyse pour $C_{103}H_{116}O_{23}$ (1722.059) : calculée C:71.84 H:6.789 trouvée C:71.73 H:6.91

Etape 2

Pour le protocole expérimental, voir l'étape 3 du composé I (R=connecteur).

100 mg (58 µmol) du composé précédent donne 40 mg (Rdt=83%) du composé III sous la forme d'une poudre blanche amorphe après lyophilisation.

$^1$H-R.M.N. (400MHz, $D_2O$) : δ 5.25 (d, 1H, $J_{1-2}$=1.2Hz, H-1B), 5.1 (d, 1H, $J_{1-2}$=1.3Hz, H-1D), 5.05 (d, 1H, $J_{1-2}$=0.8Hz, H-1A), 4.99 (d, 1H, $J_{1-2}$=1.4Hz, H-1C), 4.19 (dd, 1H, $J_{2-1}$=1.42Hz et $J_{2-3}$=3Hz, H-2C), 4.07 (dd, 1H, $J_{2-1}$=1.2Hz et $J_{2-3}$=3.1Hz, H-2B), 4.03 (dd, 1H, $J_{2-1}$=1.3Hz et $J_{2-3}$=3.3Hz, H-2D), 3.9-3.88 (m, 1H, H-2A), 3.92 (dd, 1H, $J_{3-2}$=3.1Hz et $J_{3-4}$=9.6Hz, H-3B), 3.91 (dd, 1H, $J_{3-2}$=3Hz et $J_{3-4}$=9.4Hz, H-3C), 3.72-3.65 (m, 1H, -O-CH-$CH_2$-), 3.49 (dt, 1H, $J_{gem}$=10Hz et $J_{1a-CH2}$=6.2Hz-O-CH-$CH_2$-), 2.32 (t, 2H, $J_{CH2-CH2}$=7.4Hz, -$CH_2$-$CO_2$H), 1.6-1.5 et 1.33-1.26 -(m, 12H, -$CH_2$-).

$^{13}$C R.M.N. (100 MHz) : δ 178.3 (-$CO_2$H), 102.5 (C-1D), 102.49 (C-1C), 100.99 (C-1B), 98.3 (C-1A), 79.3 (C-2A), 78.9 (C-2B), 78.2 (C-3C), 73.66, 73.6*2, 73 (4*C-5), 70.63 (C-3D), 70.61 (C-3A), 70.34 (C-2D), 70.28 (C-3B), 69.9 (C-2C), 68.3 (-O-$CH_2$-), 67.4, 67.24, 67.2, 66.5 (4*C-4), 61.4*2, 61.3, 61.2 (4*C-6), 34.6 (-$CH_2$-COOH), 28.7, 28.56, 28.55, 28.48, 25.6, 24.6 (-CH2-).

Spectre de masse (FAB négatif) : calculée $C_{33}H_{57}O_{23}$ : m/z 821.32 obtenue 821.32

Exemple 4 : Synthèse des dimannosides VII.

La figure 4 en annexe représente le schéma réationnel du protocole décrit ci-dessous.

1) 2-O-(3-O-benzyl-4,6-O-benzylidène-β-D-manno pyranosyl)-3-O-benzyl-4,6-O-benzylidène-D-mannopyra (28).

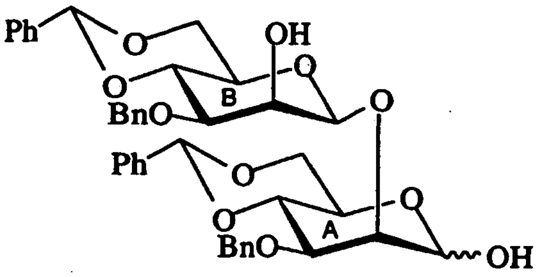

Pour le protocole expérimental, voir l'étape 2 du composé 9.

6 (200 mg , 252 μmol, 1 éq) donne 160 mg (Rdt=90%) du composé 28 (α/β=1/1) sous forme d'une mousse blanche après une chromatographie sur gel de silice (élution:cyclohexane/acétate d'éthyle=1.5/1).
$^1$H-R.M.N. (250MHz, $CDCl_3$) du mélange α-OH et β-OH: δ 7.46-7.16m(, 20H, arom.), 5.49*3 et 5.43 (2s, 4H, 4*by) , 5.21 (se, 1H, H-1) , 4.89-4.59 (m, 11H, 3*H-1 et 4*CH2Ph) , 4.35-3.55 (m, 20H, 4*H-2, 4*H-3, 4*H-4 et 4*H-6), 3.4-3.21 (m, 4H, 4*H-5).
Spectre de masse : m/z 716 $(M+NH4)^+$.
Analyse pour $C_{40}H_{42}O_{11}$ (698.77) : calculée C:68.75 H:6.058 trouvée C:68.50 H:6.26

1) 2 - O - (β -D-mannopyranosyl) -D-mannopyranose (IV, R=H).

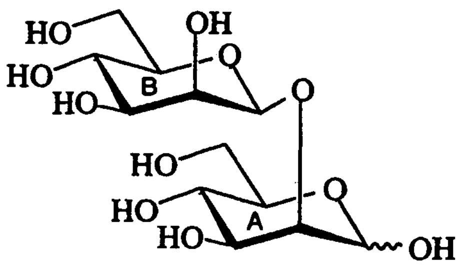

Pour le protocole expérimental, voir l'étape 3 du composé (I, R=connecteur).

28 ( 130 mg (35 μmol) donne 56.7 mg (Rdt=85%) de (IV, R=H) sous la forme d'une poudre amorphe blanche après lyophilisation.

$^1$H-R.M.N. (400MHz, $D_2O$) du composé α-OH : δ 5.27 (d, 1H, $J_{1-2}$=1.7Hz, H-1A), 4.76 (s, 1H, H-1B), 4.11 (dd, 1H, $J_{2-1}$=1.7Hz et $J_{2-3}$=3.3Hz, H-2A), 4.03 (d, 1H, $J_{2-3}$=3.1Hz, H-2B), 3.84 (dd, 1H, $J_{3-2}$=3.3Hz et $J_{3-4}$=9.7Hz, H-3A), 3.63 (dd, 1H, $J_{3-2}$=3.1Hz et $J_{3-4}$=9.5Hz, H-3B).

$^{13}$C R.M.N. (100 MHz) : δ 99.01 (C-1B), 92.25 (C-1A), 78.30 (C-2A), 71.13 (C-2B).

$^1$H-R.M.N. (400MHz, $D_2O$) du composé β-OH : 8 4.97 (s, 1H, H-1A), 4.81 (s, 1H, H-1A), 4.17 (d, 1H, $J_{2-3}$=3Hz, H-2A), 4.16 (d, 1H, $J_{2-3}$=3.3Hz, H-2B), 3.65-3.61 (m, 2H, H-3A et H-3B).

$^{13}$C R.M.N. (100 MHz) : δ 101.18 (C-1B), 93.99 (C-1A), 79.68 (C-2A), 70.63 (C-2B).
Spectre de masse HRMS : $(M-OH+NH_3)$
calculée: 342.1400 observée: 342.1391
$(M+NH_4)$+ calculée: 360.1506 observée: 360.1517

2) 8-méthoxycarbonyloctyl 2-O-(3-O-benzyl-4,6-O-benzylidène-β-D-mannopyranosyl)-3-O-benzyl-4,6-O-benzylidène-D-mannopyranoside (29) .

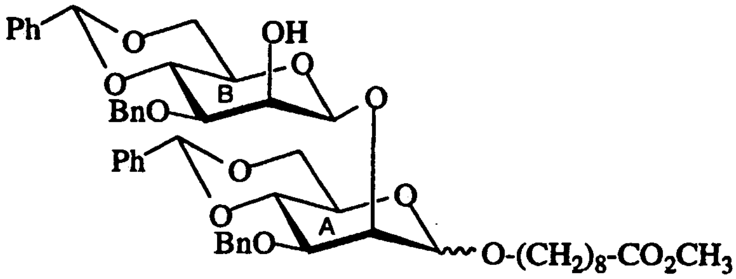

Pour le protocole expérimental, voir le composé 10.

6 (250 mg, 316 μmol) donne 190 mg (Rdt=70%) du composé 29 (mélange α/β=1/1, non séparables) , sous forme d'une huile incolore après chromatographie sur gel de silice (élution:cyclohexane/acétate d'éthyle=1.5/1)
$^1$H-R.M.N. (250MHz, $CDCl_3$) : δ 7.45-7.18m(, 20H, arom.),5.51*2, 5.49 et 5.43 (2s, 4H, 4*by), 4.86 (s, 1H, H-1), 4.80-4.67 (m, 10H, 2*H-1 et 4*CH2Ph), 4.41 (s, 1H, H-1), 4.31-3.53 (m, 28H, 4*H-2, 4*H-3, 4*H-4, 4*H-6, 2*-C:O-O-CH3 et 2*-O-CH2-), 3.42-3.21 (m, 6H, 4*H-5 est 2*-O-CH2-), 2.26-2.18 (m, 4H, 2*-CH2-CO2CH3), 1.54-1.18 (m, 24H, -O-CH2-(CH2) 6-CH2-C:O-)
Spectre de masse : m/z 886 $(M+NH4)^+$.
Analyse pour $C_{50}H_{60}O_{13}$ (869.028) :calculée C:69.10 H:6.959 trouvée C:68.55 H:7.41

3) 8-carboxyloctyl 2-0-(β-D-mannopyranosyl)-D-mannopyranoside (VII, R=connecteur).

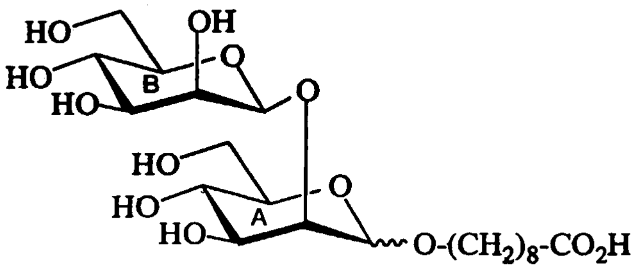

- Etape 1 : Pour le protocole expérimental, voir l'étape 2 du composé (I, R=connecteur).

60 mg (69 μmol) du précurseur 29 fournissent 42 mg (Rdt=72%) après chromatographie sur gel de silice avec élution: cyclohexane/acétate d'éthyl=1/1 sous la forme d'une poudre blanche.
$^1$H-R.M.N. (250MHz, $CDCl_3$) : δ 7.45-7.18m(, 20H, arom.),5.51*2, 5.49 et 5.43 (2s, 4H, 4*by). Absence du pic caractéristique du méthyle de l'ester méthylique.
Spectre de masse HRMS : m/z calculée: 855.3956 observée: 855.3958
Analyse pour $C_{40}H_{42}O_{11}$ (855.0008) : calculée C:68.83 H:6.837 trouvée C:68.64 H:7.10

Etape 2 : Pour le protocole expérimental, voir l'étape 3 du composé (I, R=connecteur).

A partir de 30 mg (35 μmol) de l'intermédiaire précédent, 15 mg (Rdt=92%) de (VII, R=connecteur) sous la forme d'une poudre amorphe sont récupérés après lyophilisation.

$^1$H-R.M.N. (400MHz, $D_2O$) du composé α-O-connecteur: δ 4.97 (s, 1H, H-1A), 4.75 (s, 1H, H-1B), 4.12 (d, 1H, J 2-3=3Hz, H-2A), 4.03 (d, 1H, $J_{2-3}$=2.9Hz, H-2B), 3.91-3.87 (m, 1H, -O-CH-), 3.81 (dd, 1H, $J_{3-2}$=3Hz et J 3-4=9.8Hz, H-3A), 3.65-3.61 (m, 1H, -O-CH-), 3.62-3.59 (m, 1H, H-3B), 2.3 (t, 2H, J $CH_2$-$CH_2$=7.4Hz, $-CH_2-CO_2H$), 1.63-1.56 (m, 4H, $-(CH_2)_2-$), 1.35-1.29 (m, 8H, $-(CH_2)_4-$).

$^{13}C$ R.M.N. (100 MHz) : δ 98.94 (C-1B, $^1J_C$-H=155Hz), 97.83 (C-1A, ˡJC-H=170Hz), 77.58 (C-2A), 71.1 (C-2B).
$^1H$-R.M.N. (400MHz, $D_2O$) du composé β-O-connecteur : δ 4.83 (s, 1H, H-1B), 4.72 (s, 1H, H-1A), 4.24 (d, 1H, J $_{2-3}$=2.9Hz, H-2A); 4.11 (d, 1H, J $_{2-3}$=2.9Hz, H-2B), 3.77-3.72 (m, 1H, -O-CH-), 3.66-3.61 (m, 2H, H-3A et H-3B), 3.57-3.51 (m, 1H, -O-CH-), 2.3 (t, 2H, J $CH_2$-$CH_2$=7.4Hz, -$CH_2$-$CO_2H$), 1.63-1.56 (m, 4H, -$(CH_2)_2$-), 1.35-1.29 (m, 8H, -(CH2)4-).
$^{13}C$ R.M.N. (100 MHz) : δ 101.04 (C-1B, $^1J_{C-H}$=161.8Hz), 100.45 (C-1A, $^1J_{C-H}$=157.5Hz), 78.4 (C-2A), 70.7 (C-2B).
Spectre de masse HRMS: (M-OH+NH3) calculée: 498.2551 observée: 498.2531
(M +NH4)+ calculée: 516.2656 observée: 516.2671

Exemple 5 Procédures de couplage covalent des oligomannosides de synthèse de l'invention.

Ce couplage présente les avantages suivante

- surface robuste et adaptée au tests d'immunoanalyse
- meilleure orientation de la biomolécule
- densité plus élevée en épitopes
- moins de problème de reconnaissance de l'anticorps : site antigénique accessible

Utilisation de la carbodiimide :

Cette méthode consiste à activer les groupements acide carboxylique des biomolécules elles-mêmes pour du carbodiimide, qui conduit à un ester activé puis à la formation de liaisons amides avec les groupes amine primaire de la surface (Nunc TechNote Vol. 4 N° 27-28). La carbodiimide hydro-soluble (etc : 1-ethyl-3-(3-dimethylaminopropyl) carbodiimide) est utilisée pour activer les groupements -COOH de la biomolécule en présence du sulfo-N-hydroxy succinimide (sulfo-NHS : N-hydroxysuccinimide) (Timkovich, R., 1977, Anal. Biochem. 79 : 139-143 ; Staros, J. V., et al., 1986, Anal. Biochem. 156 : 220-222 ; Rasmussen, S. E., 1990, Ann. Biol. Clin. 48 : 647-50). Le sulfo-NHS supprime de manière efficace l'hydrolyse du produit activé et permet la fixation du sucre de synthèse à la surface de la plaque déjà sensibilisée par le groupement NH2.

1) Matériel :

Dissoudre tous les réactifs de couplage dans l'eau distillée. Le reste des réactifs est reconstitué dans l'eau déminéralisée.

- Covalink NH2 C8 (Cat. Polylabo. N° N70897).
- Sucres de synthèse tetramannose βlié à une chaine de 8 carbones (bras Lemieux): 200 mg.
- Sulfo-NHS : sulfo-N-hydroxysuccinimide, PIERCE cat. N° 24510.
- EDC : 1-ethyl-3-(3-dimethylaminopropyl)-carbodiimide, Novabiochem, PIERCEN° 01-62-0011.
- PBS 0.15 M Na+, pH7.2, Sigma.
- PBS/Tween PBS, 0.05% ; Tween 20, Sigma.
- PBS/Tween/BSA : PBS/Tween, 0.5% BSA
- Lait écrémé.

2) Protocole :

Le tétramannoside de synthèse est utilisé sur deux plaques différentes.
A partir d'une solution mère de sucre à 0,8 mg/ml on réalise 6 dilutions de raison 2 pour obtenir une gamme de concentration de 40 à 1,25 μg/ml dans l'eau distillée.
On prépare de manière extemporanée une solution de :

NHS 3,48 mg/ml
EDC 3,07 mg/ml

On prévoit plusieurs puits pour le blanc et les contrôles (sucres sans réactifs de couplage, un témoin pour le conjugué)

Activation et couplage :

- On dépose successivement la solution de sucres à raison de 100 µl par puits :

  NHS 50 µl/puits
  EDC 50 µl/puits

- On couvre les plaques à l'aide d'un film plastique
- On incube les plaques pendant une nuit à température ambiante et sous agitation douce.

Lavages et Saturation :

Trois lavages avec de l'eau (300 µl/puits).
Saturation avec 300 µl/puits d'une solution de lait écrémé à 5% dans le tampon PBS.

Incubation 1 heure à température ambiante

5 lavages par le PBS-Tween 20 (0,05%) à raison de 300 µl/puits

Détection par methode ELISA :

- Les sucres ainsi couplés peuvent être détectés par l'anticoprs monoclonal 5B2 dilué au 250$^{ème}$ dans une solution PBS-Tween-BSA puis déposer 100 µl/puits.
- 5 lavages avec une solution PBS-Tween

Révélation :

- Déposer 100 µl/puits de conjugué (exemple : anti-IgM de rat dilué au 500$^{ème}$ pour révéler le 5B2) dans une solution PBS-Tween-BSA puis incuber une heure à température ambiante
- 5 Lavages PBS/Tween (300 µl/puits).
- Révélation par l'addition d'un substrat chromogène à base du tetraméthylbenzidine (TMB) :
- 200 µl de TMB (Kit de révélation , Sanofi Diagnostics Pasteur, Marnes-La-Coquette)
- Incuber les plaques à température ambiante pendant une demi heure
- Arrêter la réaction par 100 µl/puits par une solution de $H_2SO_4$ 2N.

Lecture :

- Filtre de lecture 450 nm
- Filtre de référence 620 nm

Exemple 6 : Réactivité des oligomannosides de synthèse vis à vis d'anticorps produits contre des oligomannosides naturels.

1) Oligomannosides de synthèse mimant les activités antigéniques des oligomannosides naturels de levures du genre Candida.

a) Anticorps polyclonaux.

Les anticorps polyclonaux monospécifiques commercialisés par la firme Iatron sont préparés par immunisation de lapins avec des levures entières puis adsorbés par des levures d'espèces hétérologues. Ils permettent, utilisés en

batterie, une identification par agglutination des principales espèces de Candida impliquées en pathologie humaine. Chacun de ces " facteurs " réagit avec des épitopes spécifiques qui ont été identifiés par toute une série d'études immunochimiques conduites au Japon. Les résultats de ces études sont synthétisés dans la publication de S. Suzuki (Suzuki, S. et al., 1997, Curr. Top. Med. Mycol. 8 :57-70).

Les figures 5 et 6 rapportent les tests effectués sur le facteur 5 (✿), spécifique des β-1,2 oligomannosides et le facteur 1 (✿) réagissant avec les α-1,2 oligomannosides vis à vis des mannotétraoses de synthèse d'anomérie α et β -1,2.

Les figures 5 et 6 attestent de la spécificité des réactions observées.

b) <u>Anticorps monoclonaux.</u>

Plusieurs anticorps monoclonaux ont été décrits dans la littérature pour réagir avec des β-1,2 oligomannosides de *C. albicans.* La figure 7 rapporte la réactivité du D-Man β(1-2) D-Man β(1-2) D-Man β(1-2) D-Man avec plusieurs de ces anticorps monoclonaux.

L'anticorps 5B2 (Cailliez, J. C. et al. 1988, Ann. Inst. Pasteur. Microbiol. 139 :171-88 ; Hopwood, V. et al. 1986, Infect. Immun. 54 : 222-227 ; Poulain, D. et al. 1991, Mycoses 34 : 221-226 ; Trinel, P.A., 1992, Infect. Immun. 60 : 3845-3851) généré par notre équipe (Dr. Poulain). Cet anticorps détecte des oligomannnosides circulants dans les sérums d'animaux et de patients infectés par *C. albicans.*

L'anticorps AF1 généré par A. Cassone (De Bernardis, F. et al., 1993, J. Clin. Microbiol. 31: 3142-3146 ; De Bernardis, F. et al. 1994, Infect. Immun. 62 : 509-519 ; Girmenia, C. et al., 1997, J. Clin. Microbiol. 35 : 903-906 ; Torosantucci, A. et al., 1990, J. Gen. Microbiol. 136 : 2155-2263). Cet anticorps protège les rates dans des modèles expérimentaux de candidose vaginale.

Les anticorps 10G et B6.1 générés par J. Cutler (Li, R. et al . , 1991, J. Gen. Microbiol. 137 : :455-464 ; Li, R. et al., 1993, J. Biol. Chem. 268 : 18293-8; Kanbe, T. et al., 1994, Infect. Immun. 62 . 1662-8 ; Han, Y. et al., 1995, Infect. Immun. 63 : 2714-9 ; Han, Y. et al., 1997, J. Infect. Dis. 175 : 1169- 75 ; Han, Y. et al., 1997, Infect. Immun. 65 : 4100-7 ; s Han, Y. et al., 1998, Infect. Immun. 66 : 5771-6 ; Zhang, M. X. et al., 1998, Infect. Immun. 66 : 6027-9). Ces anticorps réagissent contre des adhésines de *C. albicans* et protègent les souris dans des modèles expérimentaux de candidose disséminée.

A titre de témoin il a été utilisé un anticorps monoclonal dénommé CA1, produit par Sanofi-Diagnostic Pasteur et qui reconnaît des séquences oc-1,2 mannose (Jacquinot, P. M. et al., 1998, FEMS Microbiol. Lett. 169 : 131-8 ; Poulain, D. et al. 1991, Mycoses 34 : 221-226 ; Trinel, P.A., 1992, Infect. Immun. 60 : 3845-3851).

Les résultats de la figure 7 montrent que les sucres de synthèse réagissent de manière attendue avec ces anticorps monoclonaux et miment donc les antigènes qui présentent tous un grand intérêt en physiopathologie.

2) <u>Oligomannosides de synthèse mimant les activités antigéniques des oligomannosides naturels de levures du genre *Saccharomyces.*</u>

Il a été montré qu'un des épitopes majeurs vis à vis desquels était dirigée la réponse ASCA des patients atteints de la maladie de Crohn était un mannotétraose de formule Man α-1,3 Man α-1,2 Man α-1,2 Man (Sendid, B. et al., 1996, Clin. Diagn. Lab. Immunol. 3:219-26 ; Young, M. et al., 1998, Glycoconj. J. 15 : 815-22)

a) <u>Anticorps polyclonaux animaux.</u>

Selon les travaux immunochimiques ayant porté sur les sérums Iatron cette structure devrait réagir avec le Facteur 34 (Suzuki, S. et al., 1997, Curr. Top. Med. Mycol. 8 :57-70). La figure 8 montre que les résultats observés correspondent aux résultats attendus.

b) <u>Anticorps de patients.</u>

La figure 9 rapporte que l'utilisation du pool de sérums de patients, qui sert à standardiser le test ASCA, montre que ces anticorps se fixent à selon une réaction dépendante de la dose d'antigène de synthèse utilisée pour sensibiliser les plaques.

<u>Exemple 7 : Inhibition de la colonisation dans un modèle de candidose vaginale expérimentale de la rate (</u>F. de Bernardis et al. Infection and Immunity 1998, 66 3317-3325<u>).</u>

Des rates ovarectomisées sont maintenues en pseudo-oestrus par injection sous-cutanée de 0,5 mg de benzoate d'oestradiol tous les 2 jours pendant toute la durée des expériences.

Les animaux sont inoculés avec 0,1 mL d'une solution à $10^7$ levures. La cinétique de l'infection est suivie en

déterminant le nombre de levures présentes dans les sécrétions vaginales. 1 μL de sécrétions sont prélevées à l'aide d'une anse en plastique calibrée, étalés sur boite de sabouraud gélosé plus antibiotiques, les unités formant colonies sont numérées après 48h d'incubation à 28°C.

Le graphe de la figure 10 rapporte les résultats observés sur 4 séries de 5 rates chacune. 1 : le β-1,2 mamnotétraose (100pg) a été administré 1 heure avant inoculation. 2 : 1 heure après inoculation. 3 :1h, 24h et 48h après inoculation.

Exemple 8 Inhibition des colonisations intestinales par *C. albicans* par des β-1,2 oligomannosides de synthèse.

L'incidence croissante des candidoses systémiques est un problème médical et économique majeur en milieu hospitalier. Ces candidoses sont principalement déterminées par des levures dont l'habitat naturel est le tube digestif humain. On estime que 40 à 80% des individus sont porteurs de *C. albicans* ou de *C. glabrata,* les deux espèces les plus pathogènes du genre. Lors des candidoses systémiques c'est généralement la souche hébergée par le patient qui dissémine. Chez les patients de réanimation ou d'hématologie clinique l'intensité de la colonisation a été établie comme un facteur de risque indépendant de développement de candidose systémique.

La nature des systèmes ligand-récepteur, responsables de la colonisation intestinale par *C. albicans* est inconnue.

Les inventeurs ont pu montrer que des sucres particuliers de la surface pariétale de *C. albicans,* les β-1,2 oligomannosides se fixaient aux cellules macrophagiques par l'intermédiaire de la galectine-3. Les β-1,2 oligomannosides sont massivement exprimés à la surface de la paroi associés à plusieurs types de molécules, le mannane, les mannoprotéines et un glycolipide, le PLM. La galectine-3 est surtout connue comme une lectine exprimée sur les cellules épithéliales polarisées. Ces résultats laissent donc supposer que la galectine-3 pourrait être responsable de la fixation de *C. albicans* à l'intestin via les β-1,2 oligomannosides.

Parallèlement, il a été établi indirectement que les β-1,2 oligomannosides présentaient vraisemblablement un rôle dans la pathogénèse des candidoses. En effet des anticorps spécifiques de ces résidus se sont montrés protecteurs contrairement aux anticorps dirigés contre les résidus mannose liés en α, beaucoup plus ubiquitaires. Les mécanismes à la base de la protection ne sont pas élucidés. Aucune étude n'a concerné à ces jour, l'analyse de corrélations entre expression phénotypique de surface de β-1,2 oligomannosides et pathogénicité des souches de *C. albicans.* Cependant, des travaux anciens utilisant une anticorps monoclonal anti-glycoprotéine de *C.* albicans avaient montré que cet anticorps réagissait davantage avec les souches isolées en position pathogène que celles isolées en position saprophyte. Or, il a été montré ultérieurement que cet anticorps réagissait spécifiquement avec des épitopes β-1,2 oligomannosidiques.

Sur ces bases, le modèle expérimental qui a été développé pour explorer le rôle des β-1,2 oligomannosides dans la colonisation intestinale a visé tout d'abord à impliquer des souches de *C. albicans* sélectionnées pour présenter différents niveaux d'expression phénotypique de β-1,2 oligomannosides, puis à explorer l'effet de l'administration de β-1,2 oligomannosides sur la colonisation intestinale.

Les souches ont été choisies sur la base de travaux qui avaient montré des différences de pathogénicité reproductibles entre souches de *C. albicans* dans deux modèles expérimentaux d'infection systémique, chez le rat et la souris. La co-agglutination des levures avec des particules de latex sensibilisées par plusieurs anticorps monoclonaux anti-β-1,2 oligomannosides a permis de montrer que les souches les plus virulentes exprimaient davantage de foi-1,2 oligomannosides à leur surface. Le modèle de colonisation intestinale utilisé fut celui du souriceau nouveau né développé par Cole et al.. Deux souches y ont été impliquées, l'une «virulente », exprimant davantage de β-1,2 oligomannosides en surface qu'une souche « avirulente ». Sur la base des examens de selles, la souche virulente s'est montrée capable de maintenir une colonisation digestive plus intense et plus persistante que la souche exprimant moins de β-1,2 oligomannosides. il a alors été entrepris de tenter d'inhiber cette colonisation intense. Dans ce but, des β-1,2 oligomannosides obtenus par synthèse chimique selon l'invention ont été administrés aux souriceaux, préalablement à l'inoculation de la souche. Cette procédure permet de disposer de grandes quantités de produits purs en s'affranchissant des longues procédures de purification de matériels biologiques. L'administration de β-1,2 tétramannosides de synthèse à titre prophylactique a permis une réduction drastique et dose dépendante du nombre de levures retrouvées dans les selles par rapport aux animaux non traités ou traités par des α-1,2 tétramannosides de synthèse utilisés à titre de témoin. Ces résultats confortent les informations acquises sur l'importance physiopathologique des β-1,2 oligomannosides présentés spécifiquement par *C. albicans* aux cellules et aux lectines endogènes ses hôtes naturels. Ils laissent également entrevoir le développement de mesures prophylactiques à base d'analogues de produits naturels de *C. albicans* et visant à décontaminer le tube digestif des patients à risque.

I - Matériel et Méthodes.

1) Souches de *C. albicans.*

Il a été utilisé 7 souches de *C.* albicans de sérotype A pour lesquelles des expériences précédentes avaient permis de mettre en évidence des différences de pathogénicité dans des modèles de candidoses systémiques du rat et de la souris [Schmidt, 1996 #2553]. Il s'agit de 3 souches avirulentes ATCC44831, ATCC18804, et ATCC10231, de 3 souches virulentes : ATCC44505, ATCC62342, ATCC10261 et d'une souche de virulence intermédiaire : ATCC32354.

2) Agglutination par particules de latex sensibilisées par des anticorps monoclonaux anti-P-1, 2 oligomannosides.

Deux anticorps monoclonaux ont été utilisés pour sensibiliser des particules de latex. L'anticorps monoclonal AF1, fourni par le Pr. A Cassone [Cassone, 1988 #3333]et l'anticorps monoclonal DF9-3, fourni par le Dr. M. Borg-von-Zepelin [Borg-von-Zepelin, 1993 #283]. Ces anticorps monoclonaux sont spécifiques d'épitopes β-1,2 oligomannosidiques. Ils réagissent spécifiquement d'une part avec des β-1,2 oligomannosides dérivés du mannane convertis en néoglycolpides et utilisés pour sensibiliser des plaques de microtitration et d'autre part avec le PLM de *C. albicans* qui n'exprime que ce type d'épitopes. [Trinel, 1992 #3603; Trinel, 1993 #3292; Trinel, 1999 #3692].

Les particules de latex, sensibilisées selon la méthode précédemment décrite sont du type bichro-latex®. Elles sont colorées et mises en suspension dans un tampon de couleur complémentaire facilitant la lecture et contenant une solution qui inhibe l'agglutination spontanée de levures [Quindos, 1997 #3321]. Lors de la pratique du test, 15μL d'une suspension $10^6$ levures par mL (échelle de Mc farland) obtenue après 48h de culture à 24°C sur milieu de Sabouraud est mise en présence de 15 μL de Bichro-latex et agitée sur agitateur de Kline. Les scores d'agglutination sont appréciés à l'oeil nu après 1 min, 2 min et 3 min d'agitation selon une échelle de 0, 5 à 2 et cumulés. Cette méthode a fait preuve d'une excellente reproductibilité. Les agglutinations ont été pratiquées à l'aveugle sur les souches fournies par Schmidt avec des numéros arbitraire et les résultats ont été décryptés ultérieurement.

3) Animaux.

Les mères et leurs portées (10 à 15 souriceaux/portée) ont été livrées au $4^{ème}$ jour de vie (lignée CD1® (Crl : CD-1®(ICR) BR, Charles River, Saint Aubin les Elbeuf, France). Les animaux ont été inoculés au $6^{ème}$ jour de vie. Une séparation de la mère pendant 3 heures avant gavage et une attente d'une heure après l'inoculation ont toujours été observées.

4) Souches.

Deux souches de *C. albicans* ont été utilisées, la souche 4276 exprimant peu de β1-2 oligomannosides à sa surface, et la souche 4277 en exprimant beaucoup. Avant chaque utilisation, les souches ont été repiquées sur gélose de Sabouraud-chloramphénicol (SC) à partir d'une culture stockée à -80°C dans du PBS-glycérol 40%. Après incubation de 24 heures à 35°C, les levures ont été lavées 3 fois en eau physiologique stérile (NaCl 0,9%, eau (φ) et la suspension ajustée à 2 x $10^9$/ml. La viabilité de l'inoculum a été vérifiée à chaque expérience par ensemencement de dilutions appropriées des suspensions de levures sur gélose SC en boite de Pétri.

5) Traitement par les sucres de synthèse.

Pour certaines expériences, des sucres de synthèse (β1-2 oligomannoside (βMan) et α 1-2 oligomannosides (αMan) ont été administrés par gavage 1 heure avant inoculation. Les sucres ont dilués dans l'eau stérile de façon à obtenir la concentration désirée (voir résultats) dans un volume de 50 μl. Deux expériences indépendantes ont été entreprises. Dans la première, 4 portées infectées avec la souche 4277 ont été comparées après administration respective d'eau (portée 1), de 50 μg de βMan (portée 2), 150 μg de βMan (portée 3), ou de 150 μg de αMan (portée 4). Dans la deuxième expérience, quatre portées ont été infectées soit avec la souche 4276 (portées 1 et 2) soit avec la souche 4277 (portées 3 et 4), après traitement avec de l'eau (portée 1 et 3) ou 50 μg de liman (portées 2 et 4).

6) Surveillance de l'infection.

- Mortalité : les cages ont été surveillées une fois par jour et le nombre de souriceaux répertorié
- Colonisation digestive : de façon séquentielle à partir de J7 après l'inoculation, une crotte a été recueillie pour

chaque souriceau, broyée dans 100μl d'eau stérile à l'aide d'un petit piston adapté aux tubes Eppendorf de 1,5 ml, et le volume total a été ensemencé sur gélose SC en boite de Pétri. Le nombre d'unités formant colonies (UFC) a été compté après 24 heures d'incubation à 30°C. Lorsque la densité des UFC empêchait leur numération, un score a été attribué selon l'aspect de la culture pour permettre les comparaisons statistiques : 2 ou 1000 UFC, 3 ou 10,000 UFC, 4 ou 100,000 UFC.

7) Analyse statistique.

Les colonisations digestives selon la souche infectante et selon le traitement précédent l'inoculation ont été comparées par un test non paramétrique (test de Mann-Whitney ou Kruskall-Wallis, selon le nombre de groupe) grâce au logiciel Statview 4.5 pour Macintosh. Le pourcentage de cultures positives ou de morts à J1 a été comparé avec le Fisher exact test.

II - Résultats.

1) Expression de surface des β-1,2 oligomannosides selon la virulence de souches de sérotype A de *C. albicans.*

Les scores d'agglutination observés sur les 7 souches de sérotype A par des particules de bichro-latex sensibilisées par des anticorps monoclonaux anti-β-1,2 oligomannosides sont reportés sur le tableau 1, en fonction de leur virulence observée dans les modèles expérimentaux de candidose systémique. Toutes les souches avirulentes ont présenté un score d'agglutination inférieur aux souches virulentes ou d'une virulence intermédiaire.

Le tableau 1 ci-dessous présente les scores d'agglutination en utilisant des particules de bichro-latex sensibilisées par les anticorps DF9-3 et AF1 en fonction de la virulence des souches de C. albicans.

Tableau 1

| | DF9-3 | | | | AF1 | | | | | |
|---|---|---|---|---|---|---|---|---|---|---|
| Souches | 1 min | 2 min | 3 min | Cumulé | 1 min | 2 min | 3 min | Cumulé | Total | Statut |
| ATCC 44505 | 1 | 1,5 | 1,5 | 4 | 0,5 | 1 | 1 | 2 , 5 | 6,5 | Virulent |
| ATCC 44831 | 0,5 | 1 | 1 | 2,5 | 1 | 1 | 1 | 3 | 5,5 | Avirulent |
| ATCC 62342 | 1 | 1,5 | 2 | 4,5 | 1 | 1,5 | 1,5 | 4 | 8,5 | Virulent |
| ATCC 32354 | 1 | 1,5 | 2 | 4,5 | 0,5 | 1 | 1 | 2 ,5 | 7 | Intermédiaire |
| ATCC 18804 | 1 | 1 | 1 | 3 | 0,5 | 1 | 1 | 2 ,5 | 5,5 | Avirulent |
| ATCC 10261 | 1 | 1,5 | 2 | 4,5 | 1 | 1,5 | 2 | 4,5 | 9 | Virulent |
| ATCC 10231 | 1 | 1 | 1,5 | 3,5 | 0,5 | 0,5 | 1 | 2 | 5,5 | Avirulent |

2) Différences de mortalité et de colonisation digestive observées selon l'inoculation à la souris d'une souche virulente exprimant davantage de β-1, 2 oligomannosides qu'une souche non virulente.

a) Mortalité.

En cumulant les données obtenues dans 3 expériences indépendantes, la mortalité précoce (J1) après inoculation de la souche 10261 était supérieure à celle observée après inoculation de la souche 10231 (3/63 vs. 1/62, p=0,059 Fisher exact test).

b) Colonisation digestive.

Le nombre d'UFC présentes était significativement plus grand lorsque les souriceaux avaient été inoculés avec la souche 4277 que lorsqu'ils l'avaient été avec la souche 4276, et ce à tous les temps de la surveillance (Tableau 1). Par ailleurs, la colonisation durait significativement plus longtemps (à J33 après infection, 11/11 souriceaux infectés avec la souche 10261 avaient encore des crottes infectées contre 5/11 souriceaux seulement pour ceux qui avaient été inoculés avec la souche 10231, p=0,006 Fisher exact test).

3) Effet prophylactique de tetramannosides de synthèse d'anomérie a ou b-1,2 sur la colinsation digestive.

L'administration de tetramannosides de synthèse préalablement à l'inoculation de la souche pathogène 4277 a abouti à des résultats différents selon l'anomérie de liaison des sucres utilisés. Les résultats sont rapportés sur le tableau 2 et la figure 11.

Le tableau 2 ci-dessous rapporte la cinétique du tube digestif en fonction de la souche infectante.

Tableau 2

| | UFC/crotte en moyenne ± déviation standard (n) | | |
|---|---|---|---|
| Jour après infection | ATCC10231 | ATCC10261 | P (Mann-Whitney) |
| J12 | 154 ± 143 (n=19) | 609 ± 340 (n=18) | <0,0001 |
| J15 | 393 ± 419 (n=12) | 5777 ± 4316 (n=11) | 0,0003 |
| J19 | 58 ± 62 (n=11) | 795 ± 354 (n=11) | <0,0001 |
| J26 | 94 ± 285 (n=11) | 581 ± 416 (n=11) | 0,0006 |
| J33 | 2 ± 3 (n=11) | 1515 ± 3008 (n=11) | <0,0001 |

L'administration d'αMan avant l'inoculation par la souche 10261 n'a pas modifié le degré de colonisation digestive (médiane des UFC= 258 vs. 196), tandis que celle de βMan l'a diminué significativement et de façon d'autant plus importante que la dose était plus grande (médiane des UFC = 48,5 pour 50 μg, p<0,02 vs. témoin et 5 pour 150 μg, p<0,0001 vs. témoin) (Figure 1).

III - Discussion.

La clonisation digestive est reconnue comme une source difficilement maîtrisable d'infection systémique candidosique chez les patients hospitalisés. Malgré l'importance biologique et médicale de la colonisation naturelle de l'homme par *C. albicans* ses mécanismes moléculaires intimes restent inconnus. La découverte récente et inattendue que la galectine-3 était une lectine endogène humaine servant de récepteur spécifique pour les β-1,2 oligomannosides a permis d'ouvrir des perspectives nouvelles dans ce domaine car la galectine-3 est une lectine majoritairement exprimée sur les entérocytes. Parallèlement, bien que les β-1,2 oligomannosides apparaissent de plus en plus comme des molécules impliquées dans la pathogénèse des candidoses aucune étude n'avait concerné l'analyse de leur expression phénotypique en relation avec la pathogénicité des souches. Dans ce travail il est mainetant démontré que les différences de virulence observées entre souche de *C. albicans* dans des modèles de candidose systémique sont liées au niveau d'expression de surface de β-1,2 oligomannosides. Dans un second temps, il a été inoculé per os aux souriceaux nouveau nés des quantités identiques de levures provenant deux souches de virulence et de niveau d'expression d'oligomannosides différents ; La souche virulente exprimant davantage de β-1,2 oligomannosides a entraîné une mortalité plus importante des souriceaux ainsi qu'une colonisation plus intense et plus persistante. Ces résultats suggèrent que la virulence et/ou la quantité de β-1,2 oligomannosides présents à la surface des cellules des souches de levures sont liés à leur aptitude à entraîner le décès des souriceaux et à les coloniser plus intensément et plus longtemps. De façon à vérifier l'hypothèse du rôle des β-1,2 oligomannosides dans la colonisation intestinale il a été administré ces résidus à titre prophylactique avant d'inoculer la souche la plus virulente. Le gavage des souriceaux par les β-1,2 oligomannosides préalable à l'inoculation par *C. albicans* a entrainé une réduction de la colonisation dépendante de la dose administrée. La réduction quasi totale observée avec une dose de 150μg de β-1,2 tétramannoside ne fut pas observée lorsqu'un α-1,2 tétramannoside a été administré dans les mêmes conditions, démontrant ainsi le rôle spécifique des β-1,2 oligomannosides dans la colonisation intestinale par *C. albicans.* Ces résultats permettent de concevoir des mesures prophylactiques visant à décoloniser le tube digestif des patients risquant de développer une candidose systémique au décours de leur hospitalisation. Il s'agit des patients devant être soumis à des protocoles de chimiothérapie intensive, de greffe de moelle, d'organes solides, des patients de chirurgie digestive ou des patients de réanimation médicale capables de recevoir une alimentation entérale. Jusqu'à présent les méthodes prohylactiques ayant pour cible la décolonisation digestive ont été de plusieurs ordres. L'administration per os d'amphotéricine B, qui ne passe pas la barrière digestive n'a pas fourni de résultats véritablement probants. Plus récemment, l'administration d'immunoglobulines anti-*C. albicans* a été également proposée sans que l'on ne connaisse la nature des épitopes reconnus ni l'efficacité véritable de cette procédure. L'administration de *S. boulardii (S. cerevisiae)* correspond à des procédures connues de longue date sur l'utilisation de probiotiques susceptibles de rentrer en compétition avec *C. albicans* dans la niche écologique intestinale. Les données sur son efficacité sont contradictoires et quelques cas de septicémie déterminée

par *S. boulardii* -au demeurant rares en regard du nombre de patients traités- risquent de remettre cette prophylaxie en question. L'utilisation de molécules de levures ayant un rôle dans l'adhésion comme les β-1,2 oligomannosides rentre dans le cadre du traitement probiotique. Ces β-1,2 oligomannosides sont des molécules qui avec les levures qui les expriment naturellement à leur surface sont normalement présentes dans l'intestin et sont donc normalement tolérés par l'oganisme. Jusqu'à présent les études sur le rôle biologique de ces résidus ont été effectuées en utilisant du matériel préparé biochimiquement à partir de levures selon des procédures longues à réaliser et à standardiser avec tous les risque inhérents à la préparation voire la contamination de matériels biologiques. Une des originalités de cette étude repose sur l'utilisation de sucres de synthèse de structure parfaitement analogue à celle des sucres de *C. albicans.* La procédure suivie pour cette synthèse est facilement transposable à l'obtention en grandes quantités. Nombre de questions se posent concernant les mécanismes intimes de l'inhibition de la colonisation par *C. albicans* par les β-1,2 oligomannosides de synthèse ainsi que sur l'optimisation des doses des constructions chimiques. Il n'en reste pas moins que ce travail conforte sur le plan biologique les informations acquises sur l'importance physiopathologique des β-1,2 oligomannosides présentés spécifiquement par *C. albicans* aux cellules et aux lectines endogènes ses hôtes naturels. Il laisse également entrevoir le développement de mesures prophylactiques innovantes à base d'analogues de produits naturels de *C. albicans* et visant à décontaminer le tube digestif des patients à risque. Cette stratégie pourrait en complément d'autres déjà en oeuvre aider à la résolution d'un problème infectieux majeur lié à la présence naturelle dans le tube digestif des patients hospitalisés d'un agent opportuniste dont les capacités infectieuses croissent parallèlement aux progrès des techniques médico-chirurgicales affectant l'homéostasie des patients..

Exemple 9 : Effets thérapeutiques des β-1,2 oligomannosides de synthèse sur les candidoses vaginales.

Les candidoses vaginales sont des infections opportunistes provoquées par principalement par *C. albicans,* une levure dans la règle inoffensive. Son habitat naturel est le tube digestif humain (environ 50% des individus) ainsi que le vagin chez environ 10% des femmes. Les levures se multiplient dans le vagin sans aucune manifestation pathologique. Leur présence est cependant est en relation avec l'état physiologique de la femme. Ainsi, durant la grossesse, le pourcentage de femmes dont le vagin est colonisé par *C. albicans* croît pour atteindre 30%.
En dehors de ces relations de simple portage en surface *C. albicans* peut envahir la sphère vaginale en provoquant des infections très gènantes avec démangeaisons, brûlures, pertes blanches. Les causes de leur survenue ne sont pas élucidées. Leur mécanisme est complexe et les symptômes de l'infection sont très certainement majorés par les réactions de défense.
On sait actuellement que 75% des femmes souffriront d'un épisode de candidose vaginale au moins une fois dans leur existence. Plus de 20% d'entre elles présenteront des récidives difficiles à soigner malgré des antifongiques en théorie très efficaces. Ces récidives ont un impact physique et psychologique sur une part insoupçonnée de la population féminine. Bien qu'il ne s'agisse pas d'une maladie sexuellement transmissible au sens propre, elle perturbe la vie de millions de femmes et de couples.
Les inventeurs ont étudié les mécanismes mis en jeu lors de la transformation de la levure entre sa forme saprophyte, inoffensive et sa forme pathogène. Les principales propriétés biologiques de *C. albicans* impliquées dans la pathogénie sont celles qui lui permettent d'adhérer aux cellules de l'hôte, de pénétrer les tissus, d'éviter les réactions de défense. En se fondant sur l'analyse des variations de *C. albicans* en relation avec sa pathogénicité, les invenetrus pu identifier des molécules par lesquelles *C. albicans* adhère aux cellules et qui sont capables de moduler la réponse immunitaire. Ces molécules ont comme point commun d'exprimer des sucres particuliers à certaines espèces de *Candida* et plus particulièrement à *C. albicans :* les β-1,2 oligomannosides.

Plusieurs travaux utilisant ces sucres purifiés à partir de *C. albicans* ont montré qu'ils se fixaient aux cellules humaines et que cette fixation, s'opérant par un récepteur spécifique entraînait une réponse de la cellule cible.

L'importance des β-1,2 oligomannosides dans la pathogénie des candidoses d'une part et les difficultés inhérentes à leur obtention en grande quantité à partir de la levure d'autre part, ont conduit à préparer et utiliser les homologues obtenus par synthèse chimique selon la présente invention.

Des travaux de recherches ont permis de montrer que la présence de β-1,2 oligomannosides dans les sécrétions vaginales était un excellent marqueurs du comportement pathogène de *C. albicans.*

Ainsi, il a été montré que des anticorps anti-mannane et qu'un anticorps monoclonal dénommé AF1 reconnaissant des β-1,2 oligomannosides protégeaient la rate contre une candidose vaginale.

Ces résultats permettent de considérer que la production de β-1,2 oligomannosides par *Candida albicans* est un élément important dans les processus de pathogénèse des candidoses. Il a donc été considéré que l'administration de β-1,2 oligomannosides de synthèse (en tant que leurres) pouvait saturer les ligands des cellules vaginales avec lequels *C. albicans* interagit et ainsi priver la levure de sites d'interaction spécifique.

Un modèle de candidose vaginale reconnu par la communauté internationale et avec lequel les expérimentations animales les plus pertinentes à ce jour ont été conduites a été utilisé.

Les résultats obtenus lors de deux séries expérimentales sont parfaitement concordants. Ils démontrent une

relation dose-dépendante d'inhibition de la colonisation/infection par administration de β-1, 2 oligomannosides de synthèse 1h, 24h, 48h après l'inoculation. Même à la dose maximale, les α-1,2 oligomannosides administrés de la même façon à titre de témoin n'ont aucun effet comme montré dans le tableau 3 ci-après. Ainsi les animaux auxquels ont été administrés les β-1,2 oligomannosides voient leur colonisation diminuer de près de moitié dès le premier jour d'administration et sont les seuls à ne plus être porteurs de *C. albicans* à la fin des expériences.

Tableau 3

| Jours | SA-40 CONTROL | SA-40 +βM4 (150γ) | SA-40 +βM4 (100γ) | SA-40 +βM4 (50 γ) | SA-40+αMAN (100 γ) | SA-40+αMAN (50γ) |
|---|---|---|---|---|---|---|
| 0 | >100 | >100 | >100 | >100 | >100 | >100 |
| 1 | >100 | 62 ± 7 | 87 ± 6 | >100 | >100 | >100 |
| 2 | >100 | 54 ± 9 | 83 ± 8 | 88 ± 6 | 93 ± 3 | 95 ± 2 |
| 5 | 89 ± 3 | 27 ± 3 | 40 ± 6 | 62 ± 4 | 83 ± 5 | 86 ± 4 |
| 7 | 67 ± 5 | 28 ± 4 | 54 ± 8 | 57 ± 3 | 71 ± 6 | 69 ± 6 |
| 14 | 49 ± 9 | 5,6 ± 3 | 45 ± 14 | 23 ± 5 | 495 | 48 ± 4 |
| 21 | 20,8 ± 1,7 | <1 | <1 | <1 | 22,4 ± 3,4 | 21 ± 4 |

Il apparaît donc possible d'inhiber l'infection par *C. albicans* avec des sucres inoffensifs, totalement analogues aux naturels, par exemple contenus dans des ovules gynécologiques. Les implications de ces résultats sont importantes sur le plan médical et économique, par exemple comme un adjuvant aux traitements antifongiques classiques.

## Revendications

1. Composition pharmaceutique comprenant au moins un oligomannoside de synthèse présentant les mêmes motifs de mannose selon le même enchaînement de liaisons ou β, (1-2), (1-3), (1-6) que les oligomannosides naturels des levures, et qui répond à la formule suivante :

$$[Man\alpha(1\text{-}3)]p[Man\alpha(1\text{-}2)]q[Man\beta(1\text{-}2)]r(\alpha \text{ ou } \beta)Man\text{-}OR$$

dans laquelle :

- R représente un alkyle en $C_{15}$ à $C_{20}$, un groupe connecteur, éventuellement marqué,
- p, q, et r sont des nombres entiers entre 0 et 19 et la somme de p+q+r est comprise entre 1 et 19, - les trois parties du polymère [Manα(1-3)]p[Manα(1-2)]q[Manβ(1-2)]r peuvent être inversées ou répétées.

2. Composition pharmaceutique selon la revendication 1, dans laquelle p, q, et r sont des nombres entiers entre 0 et 11 et la somme de p+q+r est comprise entre 1 et 11.

3. Composition pharmaceutique selon la revendication 1 ou 2, dans laquelle le connecteur est un groupe chimique permettant de coupler par covalence ou par liaison hydrophobe un oligomannoside de synthèse sur un support, comme une plaque de microtitration.

4. Composition pharmaceutique selon l'une quelconque des revendications 1 à 3, dans laquelle le connecteur est du type comportant une fonction acide carboxylique qui peut être activée pour le couplage sur une surface elle-même activée.

5. Composition pharmaceutique selon l'une quelconque des revendications 1 à 4, dans laquelle l'oligomannoside de synthèse est homologue à un oligomannoside de la paroi d'une levure, d'un champignon, d'un virus, d'une bactérie dont l'enveloppe cellulaire contient des oligomannosides.

6. Composition pharmaceutique selon l'une quelconque des revendications 1 à 5, dans laquelle l'oligomannoside de synthèse est homologue à un oligomannoside de l'enveloppe cellulaire de *Candida albicans* ou *Saccharomyces cerevisiae.*

7. Composition pharmaceutique selon l'une quelconque des revendications précédentes, dans laquelle l'oligomannoside de synthèse répond à la formule (I) suivante :

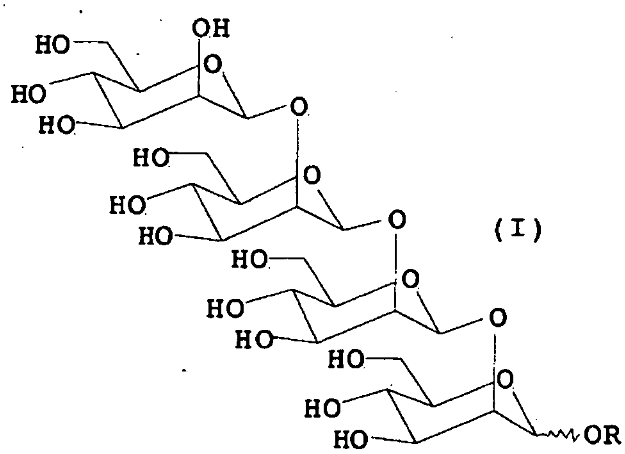

(I)

dans laquelle R représente un alkyle en $C_{15}$ à $C_{20}$, ou un groupe connecteur, éventuellement marqué, ou un dérivé de celui-ci dont un ou plusieurs des groupes hydroxyles sont substitués.

8. Composition pharmaceutique selon l'une quelconque des revendications précédentes, dans laquelle l'oligomannoside de synthèse répond à la formule (II) suivante :

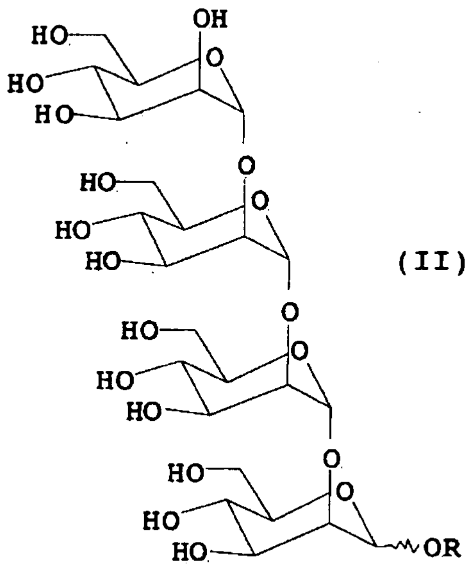

(II)

dans laquelle R à la même signification que dans la formule (I).

9. Composition pharmaceutique selon l'une quelconque des revendications précédentes, dans laquelle l'oligomannoside de synthèse répond à la formule (III) suivante :

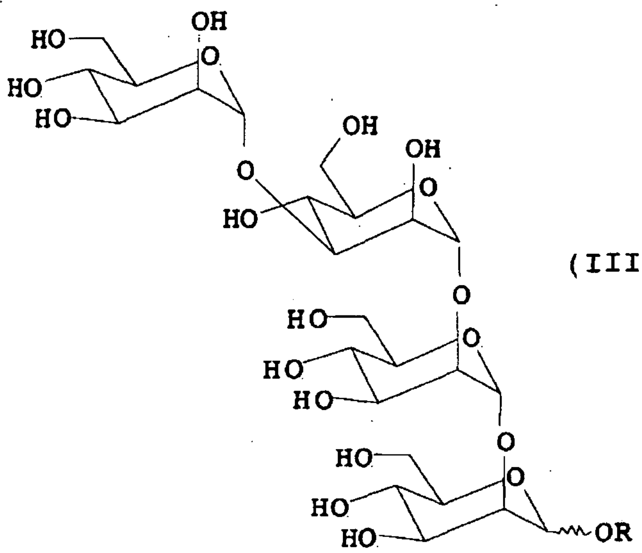

(III)

dans laquelle R à la même signification que dans la formule (I).

**10.** Utilisation d'une composition pharmaceutique comprenant au moins un oligomannoside de synthèse présentant les mêmes motifs de mannose selon le même enchaînement de liaisons α ou β, (1-2), (1-3), (1-6) que les oligomannosides naturels des levures, et qui répond à la formule suivante :

[Manα(1-3)]p[Manα(1-2)]q[Manβ(1-2)]r(α ou β)Man-OR

dans laquelle :

- R représente un atome d'hydrogène, un alkyle en $C_1$ à $C_{20}$, un groupe connecteur, éventuellement marqué,
- p, q, et r sont des nombres entiers entre 0 et 19 et la somme de p+q+r est comprise entre 1 et 19,
- les trois parties du polymère [Manα(1-3)]p[Manα(1-2)]q[Manβ(1-2)]r peuvent être inversées ou répétées,

pour la préparation d'un médicament destiné à être utilisé pour inhiber la colonisation par les agents infectieux ou pathogènes dont les membranes contiennent des oligomannosides.

**11.** Utilisation d'une composition pharmaceutique selon la revendication 10, dans laquelle R est un alkyle en $C_{15}$ à $C_{20}$,

**12.** Utilisation d'une composition pharmaceutique selon la revendication 10 ou 11 dans laquelle p, q, et r sont des nombres entiers entre 0 et 11 et la somme de p+q+r est comprise entre 1 et 11.

**13.** Procédé de préparation d'un oligomannoside de synthèse défini dans l'une quelconque des revendications 1 à 9, **caractérisé en ce qu'**il consiste à condenser des monosaccharides ou disaccharides protégés.

**14.** Procédé de préparation d'un oligomannoside de synthèse selon la revendication 13, dans lequel la condensation de dimannosides protégés est réalisée selon une stratégie par dibloc.

**15.** Procédé selon la revendication 13 ou 14 comprenant:

a) la préparation de diblocs dont :

- l'un au moins des deux blocs est le bloc intermédiaire, où les fonctions hydroxyles libres de chaque monosaccharide sont substituées par un ou plusieurs groupes protecteurs identiques ou, différents, sauf celle nécessaire à la condensation avec un autre dibloc qui est activée par un groupe partant,

- l'un des deux blocs est le bloc terminal, où les fonctions hydroxyles libres de chaque monosaccharide sont substituées par un ou plusieurs groupes protecteurs identiques ou différents, sauf celle nécessaire à la condensation avec un autre dibloc, et éventuellement celle substituée par un groupe de liaison pour la fixation de l'oligomannoside sur un support,

b) à condenser lesdits diblocs, puis à déproteger l'oligomannoside ainsi préparé.

**16.** Procédé selon la revendication 15, dans lequel à l'étape (a), on prépare au moins un dimannoside Man$\alpha$(1-2)Man de formule (IV) suivante :

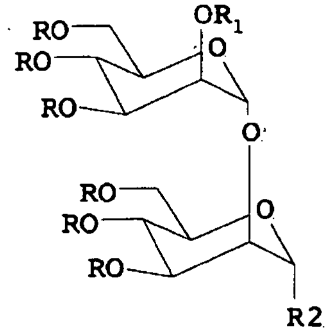

(IV)

dans laquelle :

- R est un groupe protecteur permanent.
- R1 est un groupe protecteur temporaire.
- R2 est :

. dans le cas d'un bloc intermédiaire, un groupe partant et dans ce cas le bloc peut être associé au reste du polymère en $\alpha$ ou $\beta$;
. dans le cas d'un bloc terminal, un groupe choisi parmi un groupe alkyle, benzyle, ou encore un connecteur, en $\alpha$ ou $\beta$.

**17.** Procédé selon la revendication 15, dans lequel l'étape (a), on prépare au moins un dimannoside Man$\beta$(1-2)Man de formule (V) suivante :

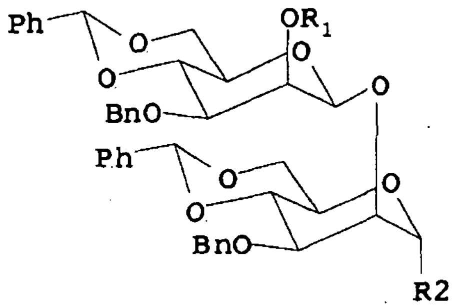

(V)

dans laquelle :

- R1 est un groupe protecteur temporaire;
- R2 est :

. dans le cas d'un bloc intermédiaire, un groupe partant et dans ce cas le bloc peut être associé au reste du polymère en $\alpha$ ou $\beta$;
. dans le cas d'un bloc terminal, un groupe choisi parmi un groupe alkyle, benzyle, ou encore un connecteur,

en α ou β.

**18.** Procédé selon la revendication 15, dans lequel à l'étape (a), on prépare au moins un dimannoside Manα(1-3)Man de formule (VI) suivante :

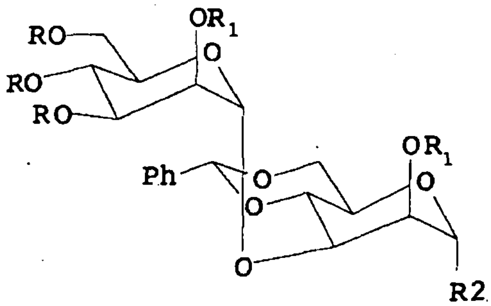

(VI

dans laquelle :

- R est un groupe protecteur permanent,
- R1 est un groupe protecteur temporaire,
- R2 est :

  . dans le cas d'un bloc intermédiaire, un groupe partant et dans ce cas le bloc peut être associé au reste du polymère en α,
  . dans le cas d'un bloc terminal, un groupe choisi parmi un groupe alkyle, benzyle, ou encore un connecteur, en α ou β.

**19.** Procédé de préparation d'un tétramanoside D-Man β(1-2) D-Man β(1-2) D-Man β(1-2) D-Man de formule (I) selon la revendication 15, dans lequel on condense deux blocs Manβ(1-2) de formule (V), dont l'un est un dibloc intermédiaire dans lequel R2 est un groupe partant, formant une liaison β, et l'autre un dibloc terminal dans lequel R2 est un groupe -SPh.

**20.** Procédé de préparation d'un tétramanoside D-Manα(1-2)D-Mana(1-2)D-Manα(1-2)D-Man, de formule (II) selon la revendication 15, dans lequel on condense deux blocs Manα(1-2)Man de formule (IV), dont l'un est un dibloc intermédiaire dans lequel R2 est un groupe partant, et l'autre un dibloc terminal dans lequel R2 est un groupe -SPh.

**21.** Procédé de préparation d'un tétramanoside D-Manα(1-3) D-Manα(1-2) D-Manα(1-2) D-Manα(1-2), de formule (III) selon la revendication 15, dans lequel on condense un dibloc Manα(1-3)Man de formule (VI) et d'un dibloc Manα (1-2)Man de formule (IV), le dibloc intermédiaire étant Manα(1-3)Man de formule (VI) dans lequel R2 est un groupe partant, et le dibloc terminal étant Mana(1-2)Man dans lequel R2 représente le groupe R défini dans la formule (III).

**22.** Procédé de préparation d'un support solide comprenant les étapes suivantes :

- synthèse d'un oligomannoside présentant les mêmes motifs de mannose selon le même enchaînement de liaisons α ou β, (1-2), (1-3), (1-6) que les oligomannosides naturels des levures, et qui répond à la formule suivante :

[Manα(1-3)]p[Manα(1-2)]q[Manβ(1-2)]r(α ou β)Man-OR

dans laquelle :

- R représente un groupe connecteur, éventuellement marqué,
- p, q, et r sont des nombres entiers entre 0 et 19 et la somme de p+q+r est comprise entre 1 et 19,
- les trois parties du polymère [Manα(1-3)]p[Manα(1-2)]q[Manβ(1-2)]r peuvent être inversées ou répétées, et

- le greffage dudit oligomannoside sur ledit support solide.

**23.** Procédé de préparation d'un support solide selon la revendication 22, dans lequel p, q, et r sont des nombres entiers entre 0 et 11 et la somme de p+q+r est comprise entre 1 et 11.

**24.** Procédé de détection *in vitro* sur un échantillon d'un patient de la présence d'une infection par un organisme infectieux ou pathogène, **caractérisé en ce que** l'on met en contact une composition selon l'une quelconque des revendications 1 à 9 avec un échantillon biologique susceptible de contenir des anticorps dirigés contre l'organisme infectieux ou pathogène, puis l'on détecte la formation d'un complexe antigène-anticorps.

**25.** Procédé de détection spécifique d'une infection par *C. albicans* selon la revendication 24, dans lequel une composition selon l'une quelconque des revendications 1 à 9 est préalablement fixé sur un support solide.

**26.** Procédé de détection spécifique d'une infection par *C. albicans* selon la revendication 24 ou 25, dans lequel on met en contact au moins une composition comprenant l'un au moins des tetramannosides D-Manβ(1-2)D-Manβ(1-2)D-Manβ(1-2)D-Man de formule (I) et D-Manα(1-2)D-Man α(1-2)D-Manα(1-2)D-Man de formule (II) avec un échantillon biologique.

**27.** Procédé de détection d'anticorps anti-oligomannosides de synthèse tels que définis selon l'une quelconque des revendications 1 à 9 pour le diagnostic ou la prédiction d'une pathologie infectieuse et/ou inflammatoire **caractérisé en ce qu'**on met en contact au moins un oligomannoside de synthèse avec un échantillon biologique.

**28.** Procédé de détection d'anticorps anti-S cerevisiae pour le diagnostic de la maladie de Crohn ou d'une hépatite virale, **caractérisé en ce que** l'on met en contact au moins un oligomannoside de synthèse tel que défini selon l'une quelconque des revendications 1 à 9 avec un échantillon biologique.

**29.** Procédé de détection spécifique d'une infection par *S. cerevisiae* selon la revendication 24 dans lequel on met en contact une composition comprenant le tetramannoside D-Man α(1-3) D-Man α(1-2) D-Man α(1-2) D-Man α (1-2) de formule (III) avec un échantillon biologique.

**30.** Un kit pour la mise en oeuvre d'un procédé selon l'une quelconque des revendications 24 à 29, **caractérisé en ce qu'**il comprend au moins une composition comprenant au moins un oligomannoside de synthèse selon l'une quelconque des revendications 1 à 9, des moyens pour détecter la formation de complexes antigène-anticorps, et éventuellement des réactif témoins.

**31.** Utilisation d'un oligomannoside tel que défini selon l'une quelconque des revendications 1 à 9 pour la préparation d'un médicament destiné à traiter ou à prévenir les candidoses.

**32.** Kit selon la revendication 30, dans lequel l'oligomannoside de synthèse est fixé sur un support solide.

## Claims

**1.** Pharmaceutical composition comprising at least one synthetic oligomannoside having the same mannose motifs according the same α or β bond linking, (1-2), (1-3), (1-6) as the natural oligomannosides of yeasts, and responding to the following formula :

$$[\text{Man}\alpha(1\text{-}3)]p[\text{Man}\alpha(1\text{-}2)]q[\text{Man}\beta(1\text{-}2)]r(\alpha \text{ or } \beta)\text{Man-OR}$$

wherein :

- R represents a $C_{15}$ to $C_{20}$ alkyl, a connector group, optionally labelled,
- p, q, and r are whole numbers between 0 and 19 and the sum p+q+r is comprise between 1 and 19,
- the three parts of the polymer [Manα(1-3)]p[Manα(1-2)]q[Manβ(1-2)]r can be inverted or repeated.

**2.** Pharmaceutical composition according to claim 1 wherein p, q, and r are whole numbers between 0 and 11 and the sum p+q+r is comprise between 1 and 11.

**3.** Pharmaceutical composition according to claim 1 or 2, wherein the connector is a chemical group enabling coupling by covalence or by hybrophobic bind a synthetic oligomannoside onto a support such as a microtitration plate.

**4.** Pharmaceutical composition according to any of claim 1 to 3 wherein the connector group is of the type of having a carboxylic acid group which can be activated for the coupling onto a surface which is itself activated.

**5.** Pharmaceutical composition according to any of claim 1 to 4 wherein the synthetic oligomannoside is homologous to a wall oligomannoside of a yeast, a fungus, a virus or a bacteria whose cellular envelope contains oligomannosides.

**6.** Pharmaceutical composition according to any of claim 1 to 5 wherein the synthetic oligomannoside is homologous to an oligomannoside of cellular envelope of *Candida albicans* or *Saccharomyces cerevisiae.*

**7.** Pharmaceutical composition according to any of preceding claim wherein, the synthetic oligomannoside respond to the following formula (I):

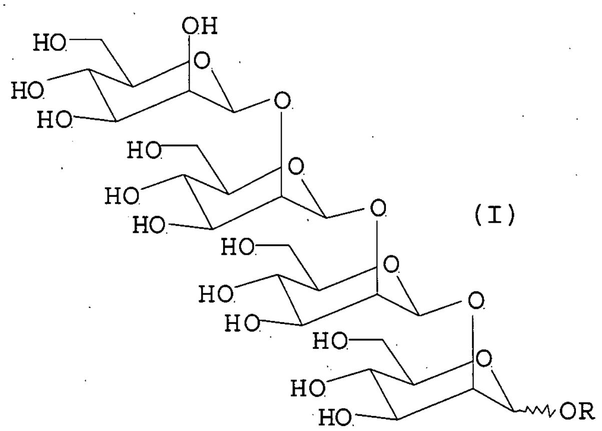

(I)

wherein R represents a $C_{15}$ to $C_{20}$ alkyl, or a connector group, optionally labelled , or a derivative in which one or more of the hydroxyl groups are substituted.

**8.** Pharmaceutical composition according to any of preceding claim wherein, the synthetic oligomannoside respond to the following formula (II):
wherein R has the same meaning as in formula

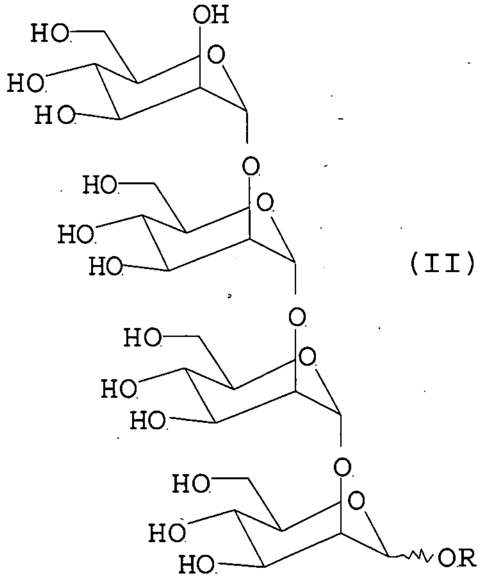

(II)

(I).

**9.** Pharmaceutical composition according to any of preceding claim wherein, the synthetic oligomannoside respond to the following formula (III):

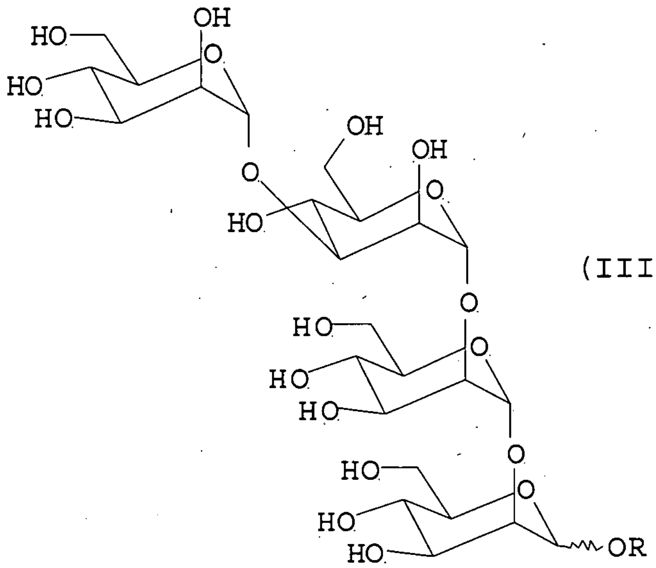

(III)

wherein R has the same meaning as in formula (I).

**10.** Use of a pharmaceutical composition comprising at least one synthetic oligomannoside having the same mannose motifs according the same α or β bond linking, (1-2), (1-3), (1-6) as the natural oligomannosides of yeasts, and responding to the following formula :

[Manα(1-3)]p[Manα(1-2)]q[Manβ(1-2)]r(α or β)Man-OR

wherein:

- R represents a hydrogen atom, a $C_{15}$ to $C_{20}$ alkyl, a connector group, optionally labelled,
- p, q, and r are whole numbers between 0 and 19 and the sum p+q+r is comprise between 1 and 19,
- the three parts of the polymer [Manα(1-3)]p[Manα(1-2)]q[Manβ(1-2)]r can be inverted or repeated,

for the manufacture of a medicament to be used for inhibiting colonization by infectious or pathogen organisms which have membrane containing oligomannosids.

**11.** Use of a pharmaceutical composition according to claim 10 wherein R is a $C_{15}$ à $C_{20}$ alkyl.

**12.** Use of a pharmaceutical composition according to claim 10 or 11 wherein p, q, and r whole numbers between 0 and 11 and the sum p+q+r is comprise between 1 and 11.

**13.** A process for preparing a synthetic oligomannoside as defined in any of claim 1 to 9 **characterised in that** it consists to condense protected monosaccharides or disaccharides.

**14.** A process for preparing a synthetic oligomannoside according to claim 13 wherein the condensation of protected dimannosides is realized according to a diblock strategy.

**15.** A process according to claim 13 or 14 comprising:

a) preparing diblock in which :

- at least one of the two blocks is the intermediary block in which the free hydroyl functionnal group of each monosaccharides are substituted by one or more, identical or different protector groups, except for the group necessary for the condensation with another diblock which is activated by a leaving group,
- one of the two blocks is the terminal block in which the free hydroxyl functions of each monosaccharide are substituted by one or more, identical or different protector groups except for the group necessary for the condensation with another diblock and eventually the group substituted by a binding group for the attachement of the oligomannoside on a support,

b) condensing said diblocks, then deprotect the oligomannoside prepared in this manner.

**16.** A process according to claim 15, wherein in step (a), at least one dimannoside Mana(1-2)Man of following formula (IV) is prepared:

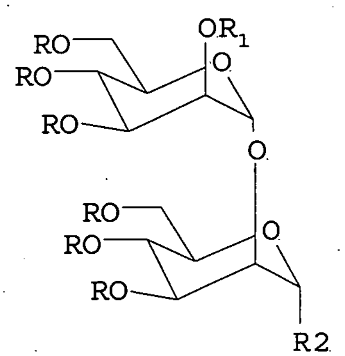

(IV)

wherein:

- R is a permanent protector group.
- R1 is a temporary protector group.
- R2 is :

. in the case of an intermediary block; a leaving group and in this case the block can be associated to the rest of the polymer at α or β;
. in the case of a terminal block, a group selected from an alkyl group, benzyl group, or a connector, in α or β.

**17.** A process according to claim 15, wherein in step (a), at least one dimannoside Manβ (1-2) Man of following formula (V) is prepared:

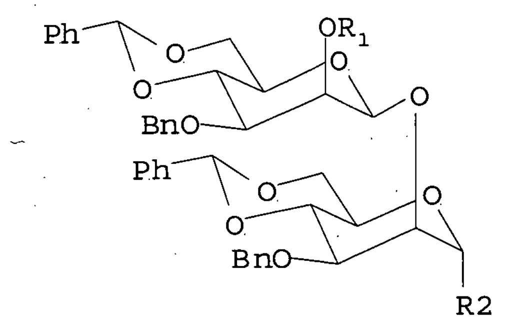

(V)

wherein :

- R1 is a temporary protector group.
- R2 is :

. in the case of an intermediary block, a leaving group and in this case the block can be associated to the rest of the polymer at α or β;

. in the case of a terminal block, a group selected from an alkyl group, benzyl group, or a connector, in α or β.

**18.** A process according to claim 15, wherein in step (a), at least one dimannoside Manα(1-3)Man of following formula (VI) is prepared:

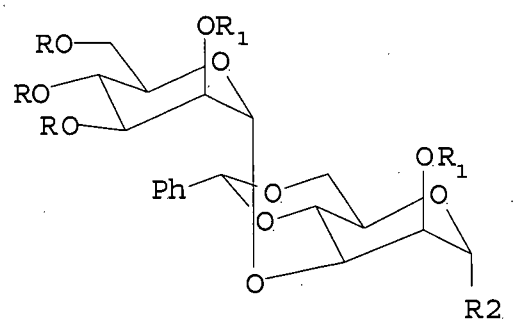

(VI

wherein :

- R is a permanent protector group.
- R1 is a temporary protector group.
- R2 is :

. in the case of an intermediary block, a leaving group and in this case the block can be associated to the rest of the polymer at α;

. in the case of a terminal block, a group selected from an alkyl group, benzyl group, or a connector, in α or β.

**19.** A process for preparing a tetramannoside D-Man β(1-2) D-Man β(1-2) D-Man β(1-2) D-Man of formula (I) according to claim 15, wherein two blocks Manβ(1-2) of formula (V) are condensate, in which one is an intermediary block wherein R2 is a leaving group, forming a bond β, and the other a terminal diblock wherein R2 is a -SPh group.

**20.** A process for preparing a tetramannoside D-Manα(1-2)D-Manα(1-2)D-Manα(1-2)D-Man, of formula (II) according to claim 15, wherein two blocks Manα(1-2)Man of formula (IV) are condensate, in which one is an intermediary block wherein R2 is leaving group, and the other a terminal diblock wherein R2 is a -SPh group.

**21.** A process for preparing a tetramannoside D-Manα(1-3) D-Manα(1-2) D-Manα(1-2) D-Manα(1-2), of formula (III) according to claim 15, wherein a diblock Manα(1-3)Man of formula (VI) and a diblock Manα(1-2)Man of formula (IV) are condensate, the intermediary diblock being Manα(1-3)Man of formula (VI) in which R2 is a leaving group, and the terminal diblock being Manα(1-2)Man in which R2 represents R group defined in formula (III).

**22.** A process for preparing a solid support comprising the following steps:

- synthesis of a oligomannoside having the same mannose motif according to the same α or β bond linking (1-2), (1-3), (1-6) as the natural oligomannosides of yeast, and which respond to the following formula :

[Manα(1-3)]p[Manα(1-2)]q[Manβ(1-2)]r(α or β)Man-

OR

wherein:

- R represents a connector group, optionally labelled,
- p, q, and r are whole numbers between 0 and 19 and the sum p+q+r is comprise between 1 and 19,
- the three parts of the polymer [Manα(1-3)]p[Manα(1-2)]q[Manβ(1-2)]r can be inverted or repeated,
- grafting of said oligomannoside onto the solid support.

**23.** A process for preparing a solid support according to claim 22, wherein p, q, and r are whole numbers between 0 and 11 and the sum p+q+r is comprise between 1 and 11.

**24.** Process of *in vitro* detection on a sample from a patient of the presence of an infection by an infectious organism or pathogen **characterized in that** at least one composition according to any of claim 1 to 9 is brought into contact with a biological sample capable of containing antibodies directed against the infectious organism or pathogen, then detecting the formation a antigen-antibodies complex.

**25.** Process of specific detection of an infection by *C. albicans* according to claim 24 wherein a composition according to any of claim 1 to 9 is beforehand fixed onto a solid support.

**26.** Process of specific detection of an infection by *C. albicans* according to claim 24 or 25, wherein at least one composition comprising at least one of tetramannosides D-Manβ(1-2)D-Manβ(1-2)D-Manβ(1-2)D-Man of formula (I) and D-Manα(1-2)D-Man α(1-2)D-Manα(1-2)D-Man of formula (II) is brought into contact with a biological sample.

**27.** Process for detecting anti-synthetic oligomannosides antibodies as defined in any of claim 1 to 9 for diagnosing or predicting an infectious and/or inflammatory pathology **characterized in that** at least one synthetic oligomannoside is brought into contact with a biological sample

**28.** Process for detecting anti-S cerevisiae antibodies for the diagnosis of Chron's disease or viral hepatitis **characterized in that** at least one synthetic oligomannoside as defined in any of claim 1 to 9 is brought into contact with a biological sample.

**29.** Process for specific detection by *S. cerevisiae* according to claim 24 wherein a composition comprising the tetramannoside D-Man α(1-3) D-Man α(1-2) D-Man α(1-2) D-Man α (1-2) of formula (III) is brought into contact with a biological sample.

**30.** Kit for carrying out a process according to any of claim 24 to 29 **characterized in that** it comprises at least one synthetic oligomannoside according to any of claim 1 to 9, means for detecting formation of antigen-antibodies complexes and optionally control reagents.

**31.** Use of a oligomannoside as defined in any of claim 1 to 9 for the manufacture of a medicament for treating or preventing candi2diasis.

**32.** Kit according to claim 30 wherein the synthetic oligomannoside is fixed onto a solid support.

**Patentansprüche**

1. Pharmazeutische Zusammensetzung mit mindestens einem synthetischen Oligomannosiden, der die gleichen Mannosemotive mit derselben Verkettung der $\alpha$ oder $\beta$ Bindungen, (1-2), (1-3), (1-6) aufweist wie die natürlichen Oligomannoside der Hefe und folgender Formel entspricht:

$$[Man\alpha(1\text{-}3)]p[Man\alpha(1\text{-}2)]q[Man\beta(1\text{-}2)]r(\alpha \text{ oder } \beta)Man\text{-}OR$$

wobei :

- R ein $C_{15}$ bis $C_{20}$ Alkyl, eine gegebenenfalls markierte Verbindungsgruppe darstellt,
- p, q, und r ganze Zahlen zwischen 0 und 19 sind und die Summe von p+q+r zwischen 1 und 19 liegt,
- die drei Teile des Polymers [Manα(1-3)]p[Manα(1-2)]q[Manβ(1-2)]r umgekehrt oder wiederholt auftreten können.

2. Pharmazeutische Zusammensetzung nach Anspruch 1, wobei p, q, und r ganze Zahlen zwischen 0 und 11 sind und die Summe von p+q+r zwischen 1 und 11 liegt.

3. Pharmazeutische Zusammensetzung nach Anspruch 1 oder 2, in der die Verbindung aus einer chemischen Gruppe besteht, durch die ein synthetischer Oligomannosid und ein Träger, zum Beispiel eine Mikrotitrationsplatte, kovalent oder hydrophobisch gekoppelt werden können.

4. Pharmazeutische Zusammensetzung nach einem beliebigen der Ansprüche 1 bis 3, wobei die Verbindung des Typs ist, der eine Carbonsäuregruppe enthält, die zur Kupplung an einer selbst aktivierten Oberfläche aktiviert werden kann.

5. Pharmazeutische Zusammensetzung nach einem beliebigen der Ansprüche 1 bis 4, wobei der synthetische Oligomannosid homolog zu einem Oligomannosiden der Wand einer Hefe, eines Pilzes, eines Virus oder einer Bakterie ist, deren Zellhülle Oligomannoside enthält.

6. Pharmazeutische Zusammensetzung nach einem beliebigen der Ansprüche 1 bis 5, wobei der synthetische Oligomannosid homolog zu einem Oligomannosiden der Zellhülle von *Candida albicans* oder *Saccharomyces cerevisiae* ist.

7. Pharmazeutische Zusammensetzung nach einem beliebigen der vorhergehenden Ansprüche, wobei der synthetische Oligomannosid folgender Formel (I) entspricht:

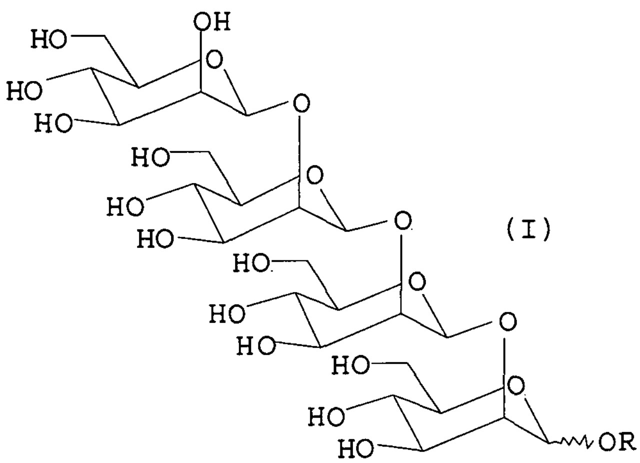

(I)

in der R ein $C_{15}$ bis $C_{20}$ Alkyl, oder eine gegebenenfalls markierte Verbindungsgruppe, oder ein Derivat von dieser

darstellt, in dem eine oder mehrere Hydroyxlgruppen substituiert sind.

8. Pharmazeutische Zusammensetzung nach einem beliebigen der vorhergehenden Ansprüche, wobei der synthetische Oligomannosid folgender Formel (II) entspricht:

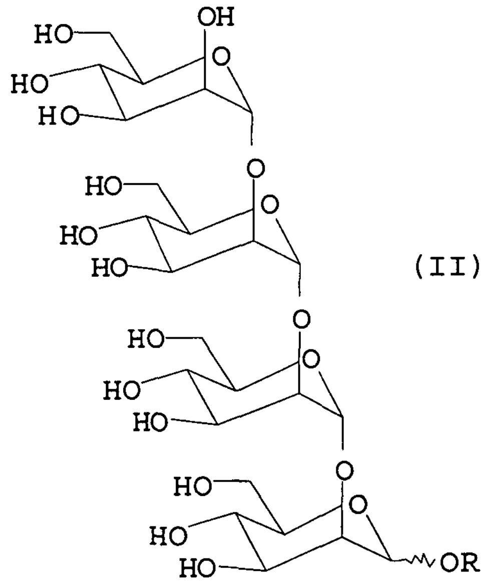

(II)

(II):
in der R dieselbe Bedeutung wie in Formel (I) hat.

9. Pharmazeutische Zusammensetzung nach einem beliebigen der vorhergehenden Ansprüche, wobei der synthetische Oligomannosid folgender Formel (III) entspricht:

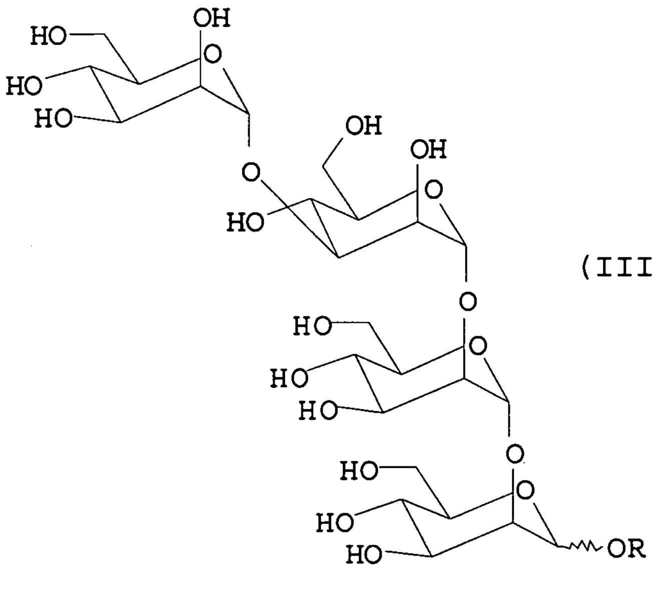

(III)

in der R dieselbe Bedeutung wie in Formel (I) hat.

10. Verwendung einer pharmazeutischen Zusammensetzung mit mindestens einem synthetischen Oligomannosiden, der die gleichen Mannosemotive mit derselben Verkettung der α- oder β- Bindungen, (1-2), (1-3), (1-6) aufweist, wie die natürlichen Oligomannoside der Hefe und folgender Formel entspricht:

$$[Man\alpha(1\text{-}3)]p[Man\alpha(1\text{-}2)]q[Man\beta(1\text{-}2)]r(\alpha \text{ oder } \beta)Man\text{-}OR$$

wobei:

- R ein $C_{15}$ bis $C_{20}$ Alkyl, ein Wasserstoffatom, eine gegebenenfalls markierte Verbindungsgruppe darstellt,
- p, q, und r ganze Zahlen zwischen 0 und 19 sind und die Summe von p+q+r zwischen 1 und 19 liegt,
- die drei Teile des Polymers [Manα(1-3)]p[Manα(1-2)]q[Manβ(1-2)]r umgekehrt oder wiederholt auftreten können,

zur Herstellung eines Medikaments, das die Kolonisation durch infektiöse oder pathogene Krankheitserreger, deren Membranen Oligomannoside enthalten, vermeiden soll.

**11.** Verwendung einer pharmazeutischen Zusammensetzung nach Anspruch 10, in der R ein $C_{15}$ bis $C_{20}$ Alkyl ist.

**12.** Verwendung einer pharmazeutischen Zusammensetzung nach Anspruch 10 oder 11, wobei p, q, und r ganze Zahlen zwischen 0 und 11 sind und die Summe von p+q+r zwischen 1 und 11 liegt.

**13.** Verfahren zur Herstellung eines synthetischen Oligomannosiden wie in einem beliebigen der Ansprüchen 1 bis 9 definiert, **dadurch gekennzeichnet, dass** er darin besteht, geschützte Disaccharide und Monosaccharide zu kondensieren.

**14.** Verfahren zur Herstellung eines synthetischen Oligomannosiden nach Anspruch 13, wobei die Kondensation geschützter Dimannoside gemäss einer Diblockstrategie durchgeführt wird.

**15.** Verfahren nach Anspruch 13 oder 14 mit folgenden :

a) Herstellung eines Diblocks :

- von dem mindestens einer der beiden Blöcke den mittleren Block darstellt, in dem die freien Hydroxylfunktionen jedes Monosacchariden durch eine oder mehrere identische oder unterschiedliche Schutzgruppen ersetzt werden, ausser jener, die zur Kondensation mit einem anderen Diblock erforderlich ist, die durch eine abgehende Gruppe aktiviert wird,
- von dem, einer der beiden Blöcke einen Endblock darstellt, in dem die freien Hydroxylfunktionen jedes Monosacchariden durch eine oder mehrere identische oder unterschiedliche Schutzgruppen ersetzt werden, ausser jener, die zur Kondensation mit einem anderen Diblock erforderlich ist, und gegebenenfalls jener, welche durch eine Verbindungsgruppe zur Bindung des Oligomannosiden an einen Träger ersetzt wird,

b) Kondensierung der besagten Diblöcke, dann Aufhebung des Schutzes des so erhaltenen Oligomannsiden.

**16.** Verfahren nach Anspruch 15, wobei im Schritt (a), mindestens ein Dimannosid Manα(1-2)Man mit folgender Formel (IV) hergestellt wird :

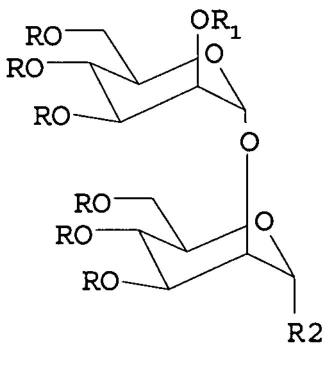

(IV)

in der :

- R eine permanente Schutzgruppe ist,
- R1 eine vorübergehende Schutzgruppe ist,
- R2 ist :

. im Falle eines mittleren Blocks, eine abgehende Gruppe und in diesem Fall kann der Block mit dem Rest des Polymers an α oder β verbunden sein;
. im Falle eines Endblocks, eine Gruppe unter folgenden, eine Alkylgruppe, eine Benzylgruppe oder auch eine Verbindung an α oder β.

**17.** Ein Verfahren nach Anspruch 15, wobei im Schritt (a), mindestens ein Dimannosid Manβ(1-2)Man mit folgender Formel (V) hergestellt wird :

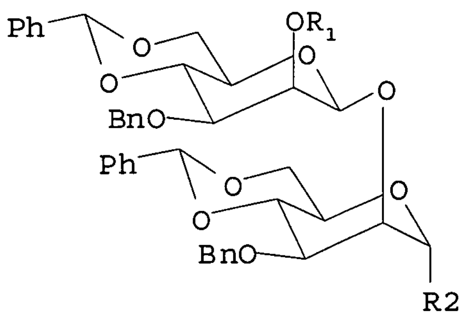

(V)

in der :

- R1 eine vorübergehende Schutzgruppe ist,
- R2 ist :

. im Falle eines mittleren Blocks, eine abgehende Gruppe und in diesem Fall kann der Block mit dem Rest des Polymers an α oder β verbunden sein;
. im Falle eines Endblocks, eine Gruppe unter folgenden, eine Alkylgruppe, eine Benzylgruppe oder auch eine Verbindung an α oder β.

**18.** Ein Verfahren nach Anspruch 15, wobei im Schritt (a), mindestens ein Dimannosid Manβ(1-3)Man mit folgender Formel (VI) hergestellt wird :

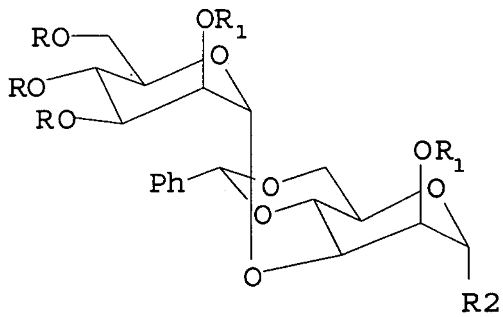

(VI

in der :

- R eine permanente Schutzgruppe ist,
- R1 eine vorübergehende Schutzgruppe ist,

- R2 ist :

  . im Falle eines mittleren Blocks, eine abgehende Gruppe und in diesem Fall kann der Block mit dem Rest des Polymers an α verbunden sein;
  . im Falle eines Endblocks, eine Gruppe unter folgenden, eine Alkylgruppe, eine Benzylgruppe oder auch eine Verbindung an α oder β.

**19.** Verfahren zur Herstellung eines Tetramannosiden D-Man β(1-2) D-Man β(1-2) D-Man β(1-2) D-Man entsprechend Formel (I) nach Anspruch 15, wobei zwei Blöcke Manβ(1-2) der Formel (V) kondensiert werden, von denen einer ein mittlerer Diblock ist, in dem R2 eine abgehende Gruppe ist, die eine Verbindung β bildet, und der andere ein End-Diblock ist, in dem R2 eine -SPh Gruppe ist.

**20.** Verfahren zur Herstellung eines Tetramannosiden D-Manα(1-2)D-Manα(1-2)D-Manα(1-2)D-Man der Formel (II) nach Anspruch 15, wobei zwei Blöcke Manα(1-2)Man der Formel (IV) kondensiert werden, von denen einer ein mittlerer Diblock ist, in dem R2 eine abgehende Gruppe ist, und der andere ein End-Diblock ist, indem R2 eine -SPh Gruppe ist.

**21.** Verfahren zur Herstellung eines Tetramannosiden D-Manα(1-3) D-Manα(1-2) D-Manα(1-2) D-Manα(1-2) der Formel (III) nach Anspruch 15, wobei ein Diblock Manα(1-3)Man der Formel (VI) und ein Diblock Manα(1-2)Man der Formel (IV) kondensiert werden, wobei der mittlere Diblock ein Manα(1-3)Man der Formel (VI) ist, in der R2 eine abgehende Gruppe ist, und der End-Diblock ein Manα(1-2)Man ist, in dem R2 eine R-Gruppe wie in Formel (III) definiert darstellt.

**22.** Ein Verfahren zur Herstellung eines festen Trägers mit folgenden Schritten:

- Synthese eines Oligomannosiden, der das gleiche Mannosemotif mit derselben Verkettung der α- oder β-Bindungen (1-2), (1-3), (1-6) wie die natürlichen Oligomannoside von Hefe aufweist, und folgender Formel entspricht :

[Manα(1-3)]p[Manα(1-2)]q[Manβ(1-2)]r(α oder β)Man-OR

wobei:

- R eine gegebenenfalls markierte Verbindungsgruppe darstellt,
- p, q, und r ganze Zahlen zwischen 0 und 19 sind und die Summe von p+q+r zwischen 1 und 19 liegt,
- die drei Teile des Polymers [Manα(1-3)]p[Manα (1-2)]q[Manβ(1-2)]r umgekehrt oder wiederholt auftreten können, und
- das Propfen des besagten Oligomannosiden an einen festen Träger.

**23.** Ein Verfahren zur Herstellung eines festen Trägers nach Anspruch 22, wobei p, q, und r - p, q, und r ganze Zahlen zwischen 0 und 11 sind und die Summe von p+q+r zwischen 1 und 11 liegt.

**24.** Verfahren zum *in vitro* Nachweis des Vorhandenseins einer Infektion durch einen infektiösen oder pathogenen Erreger in einer Probe eines Patienten, **dadurch gekennzeichnet, dass** mindestens eine Verbindung nach einem beliebigen der Ansprüche 1 bis 9 mit der biologischen Probe in Kontakt gebracht wird, die gegen infektiöse oder pathogene Organismen gerichtete Antikörper enthalten kann, worauf die Bildung von Antigen-Antikörper-Komplexen nachgewiesen wird.

**25.** Verfahren zum spezifischen Nachweis einer *C. albicans* Infektion nach Anspruch 24, wobei eine Verbindung nach einem beliebigen der Ansprüche 1 bis 9 zuvor auf einem festen Träger fixiert wird.

**26.** Verfahren zum spezifischen Nachweis einer *C. albicans* Infektion nach Anspruch 24 oder 25, wobei mindestens eine Verbindung mit mindestens einem der Tetramannosiden D-Manβ(1-2)D-Manβ(1-2)D-Manβ(1-2)D-Man der Formel (I) und D-Manα(1-2)D-Man α(1-2)D-Manα(1-2)D-Man der Formel (II) mit einer biologischen Probe in Kontakt gebracht wird.

**27.** Verfahren zum Nachweis von antisynthethischen Oligomannosiden Antikörpern entsprechend der Definition in einem beliebigen der Ansprüche 1 bis 9 zur Diagnose oder Vorhersage einer infektiösen und/oder entzündlichen Pathologie, **dadurch gekennzeichnet, dass** mindestens ein synhetischer Oligomannosid mit einer biologischen Probe in Kontakt gebracht wird.

**28.** Verfahren zum Nachweis von anti-S cerevisiae Antikörpern für die Diagnose der Morbus Chron oder viraler Hepatitis, **dadurch gekennzeichnet, dass** mindestens ein synthetischer Oligomannosid entsprechend der Definition in einem beliebigen der Ansprüche 1 bis 9 mit einer biologischen Probe in Kontakt gebracht wird.

**29.** Verfahren zum spezifischen Nachweis einer *S. cerevisiae* Infektion nach Anspruch 24, wobei eine Verbindung mit einem Tetramonnosiden D-Man $\alpha$(1-3) D-Man $\alpha$(1-2) D-Man $\alpha$(1-2) D-Man $\alpha$ (1-2) der Formel (III) mit einer biologischen Probe in Kontakt gebracht wird.

**30.** Ein Kit zur Durchführung eines Verfahrens nach einem beliebigen der Ansprüche 24 bis 29, **dadurch gekennzeichnet, dass** er mindestens einen synthetischen Oligomannosiden, gemäss einem beliebigen der Ansprüche 1 bis 9 enthält, Mittel zum Nachweis der Bildung von Antigen-Antikörper-Komplexen und gegebenenfalls Kontrollreagenzien.

**31.** Verwendung eines Oligomannosiden nach der Definition in einem beliebigen der Ansprüche 1 bis 9 zur Herstellung eines Medikaments zur Behandlung oder Vorbeugung von Candidiasen.

**32.** Kit nach Anspruch 30, wobei ein synthetischer Oligomannosid auf einem festen Träger fixiert wird.

Figure 1

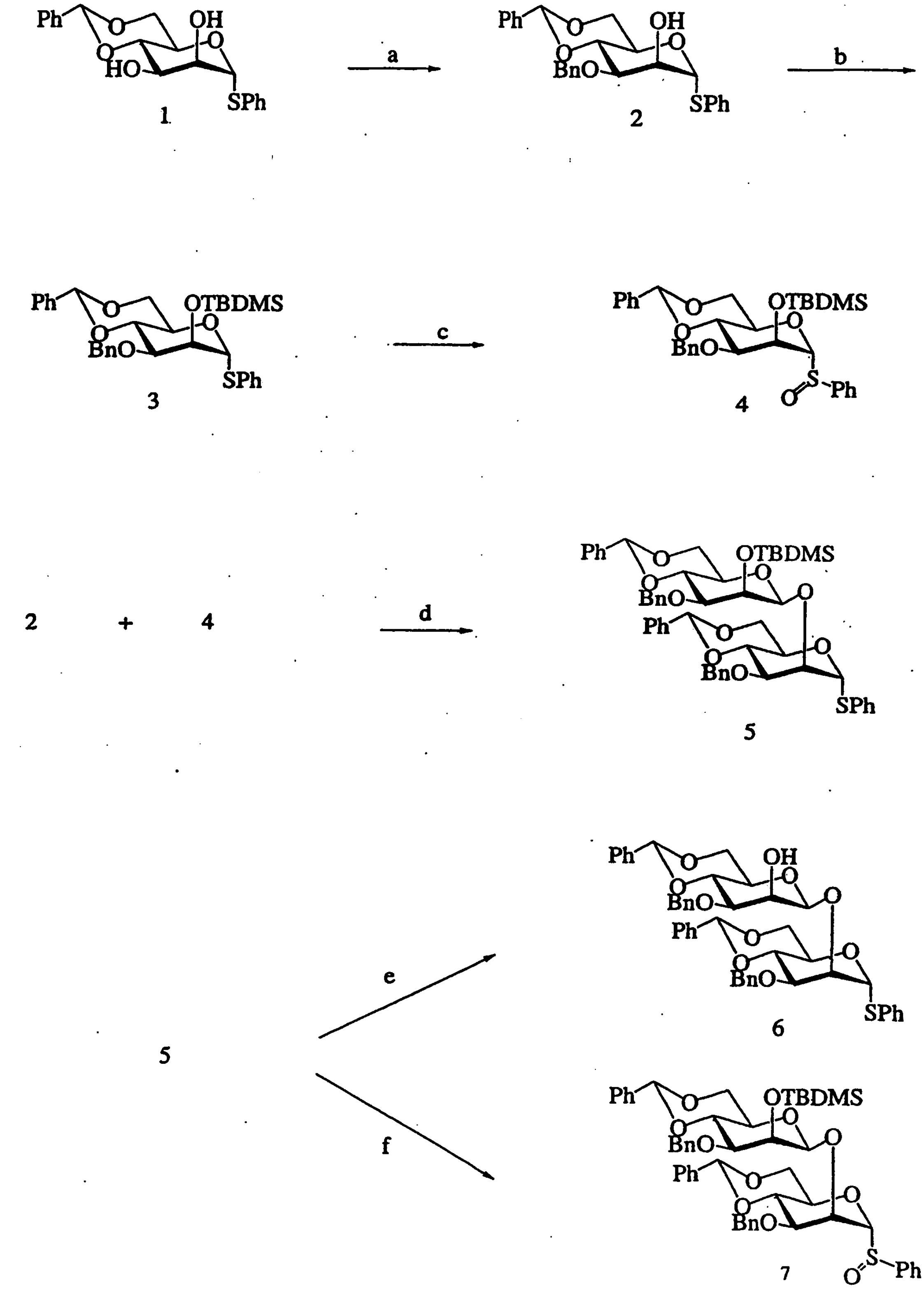


1 2 3 4 2 + 4 5 5 6 7

Figure 1 suite

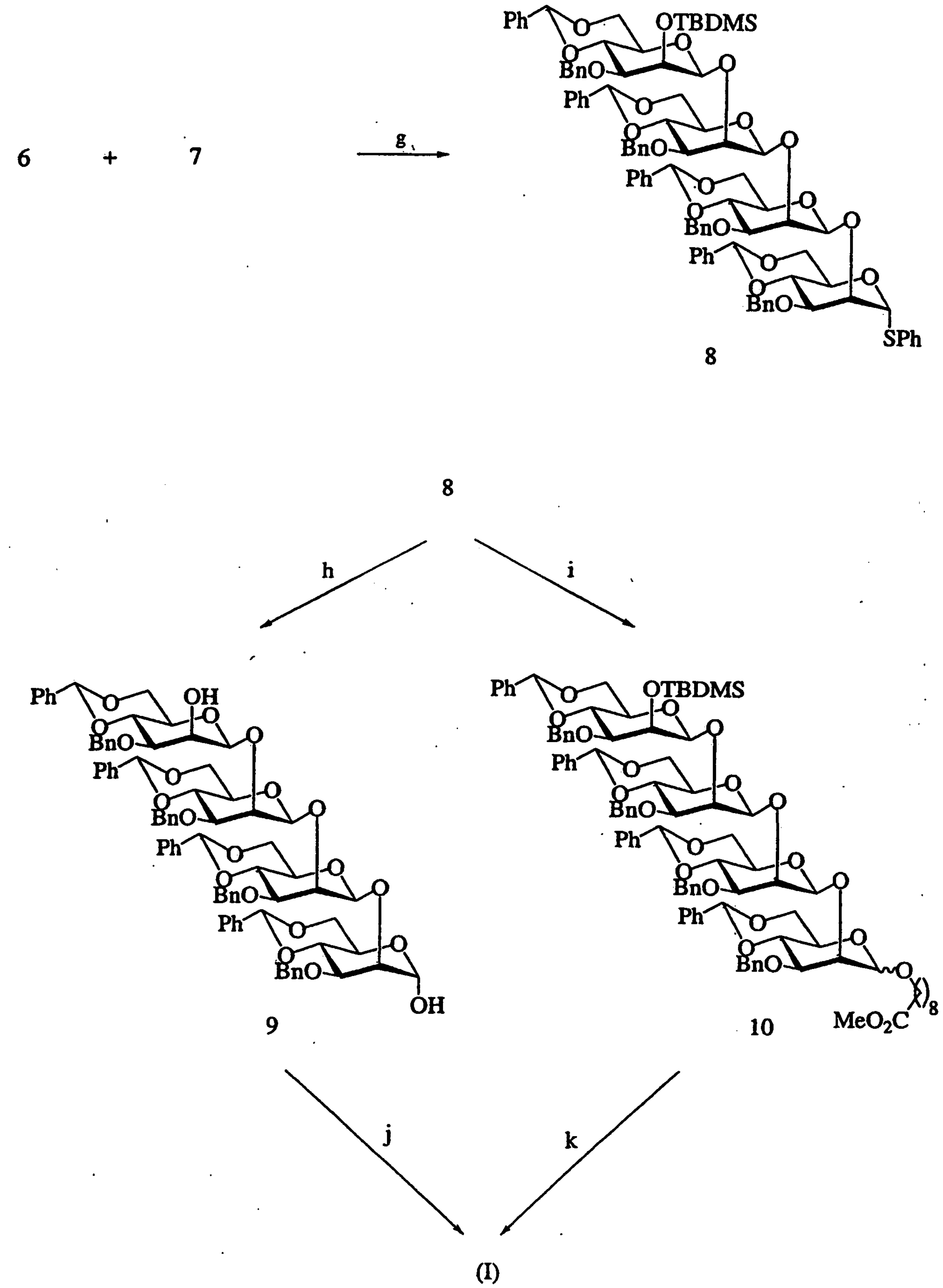


6 + 7

8

8

9

10

(I)

Figure 2

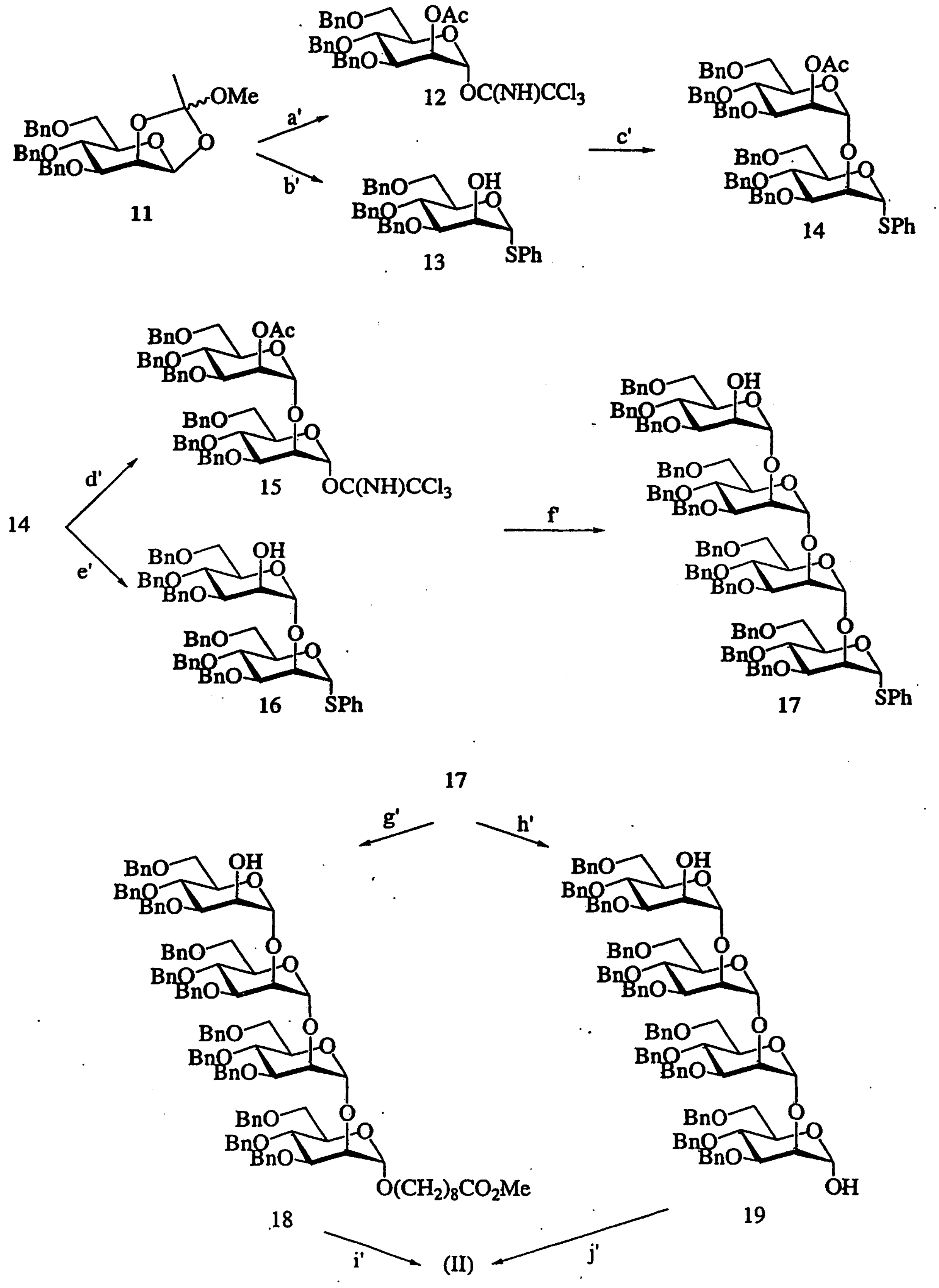

Figure 3

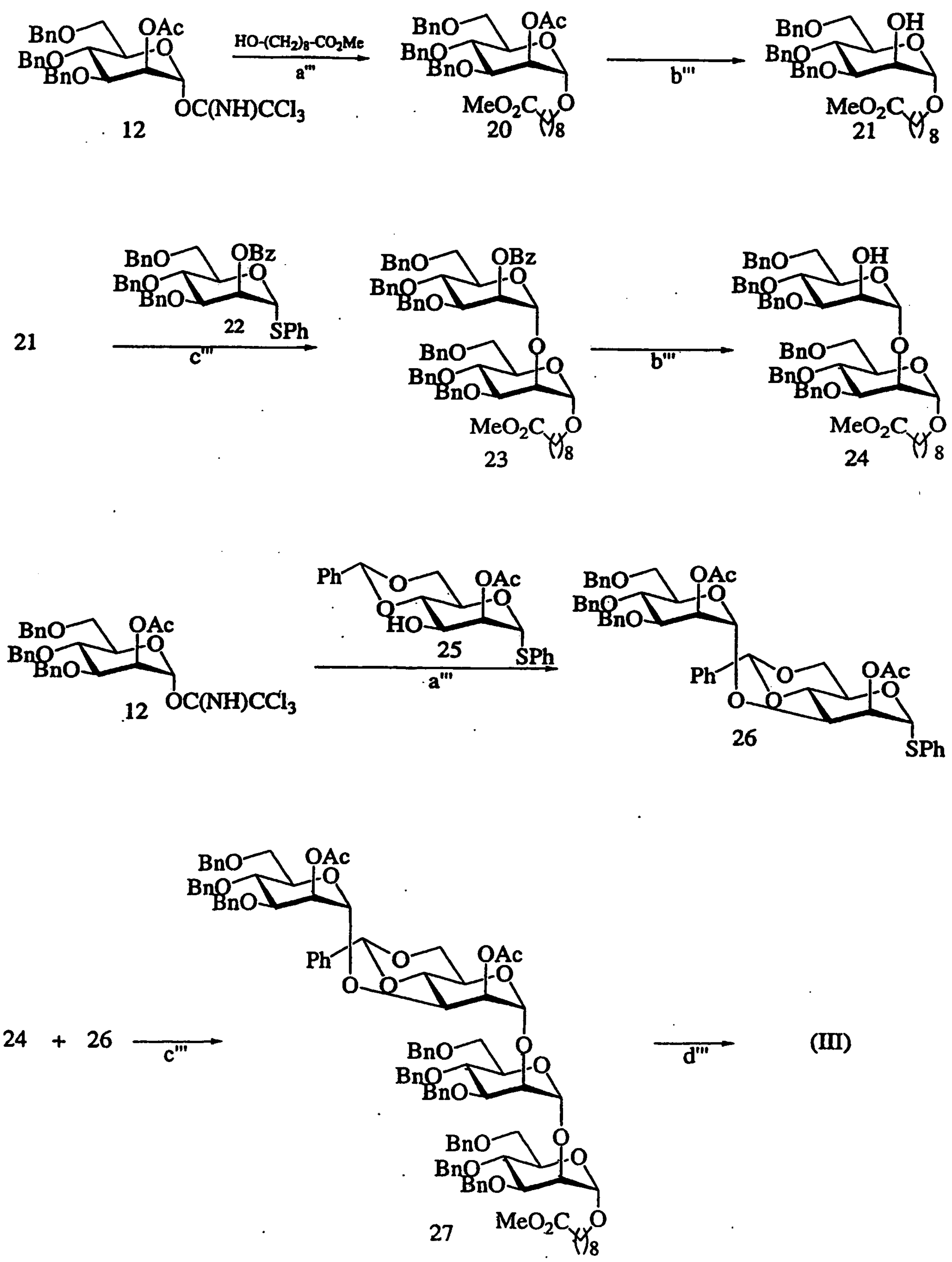


12 20 21

21 22 23 24

12 25 26

24 + 26 27 (III)

Figure 4

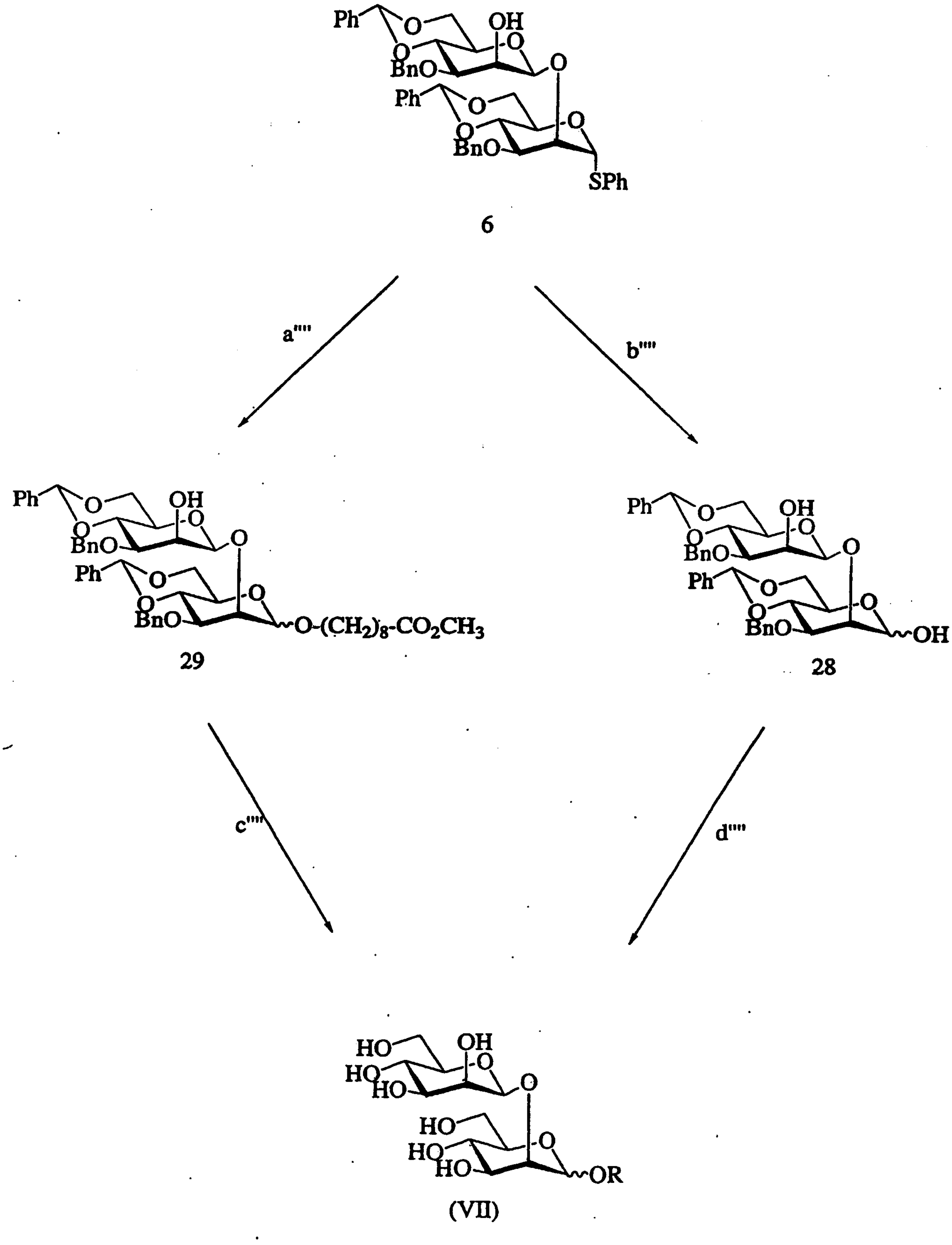


6

29

28

(VII)

Fig.5

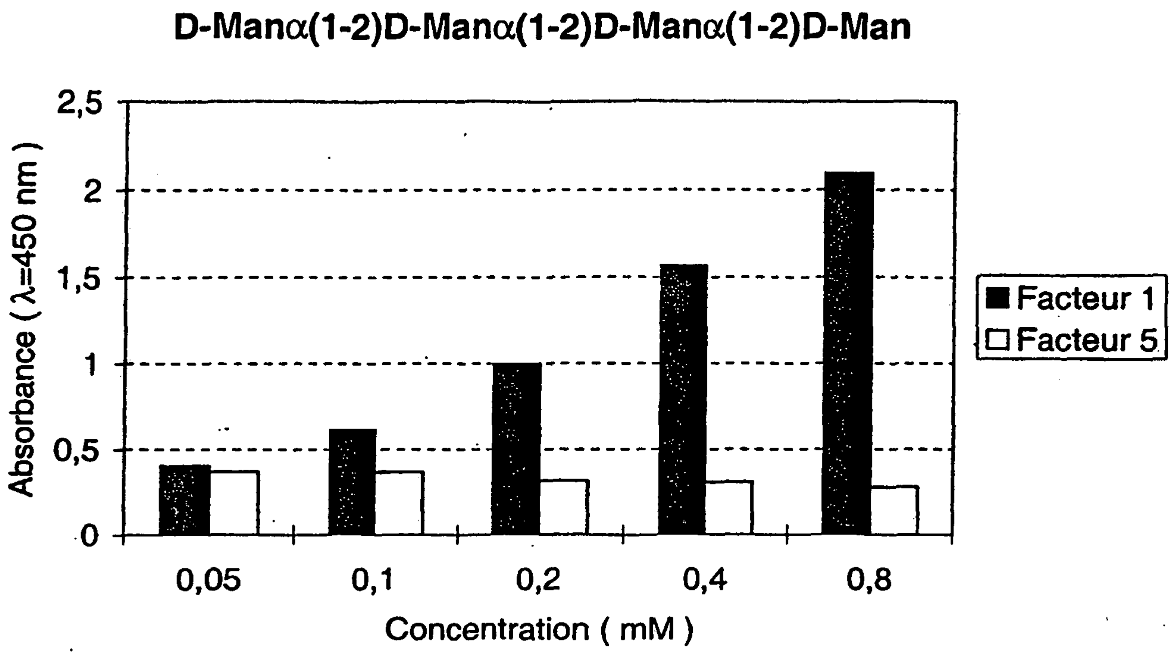
D-Manα(1-2)D-Manα(1-2)D-Manα(1-2)D-Man
Absorbance ( λ=450 nm )
2,5
2
1,5
1
0,5
0
0,05
0,1
0,2
0,4
0,8
Concentration ( mM )
Facteur 1
Facteur 5

Fig.6

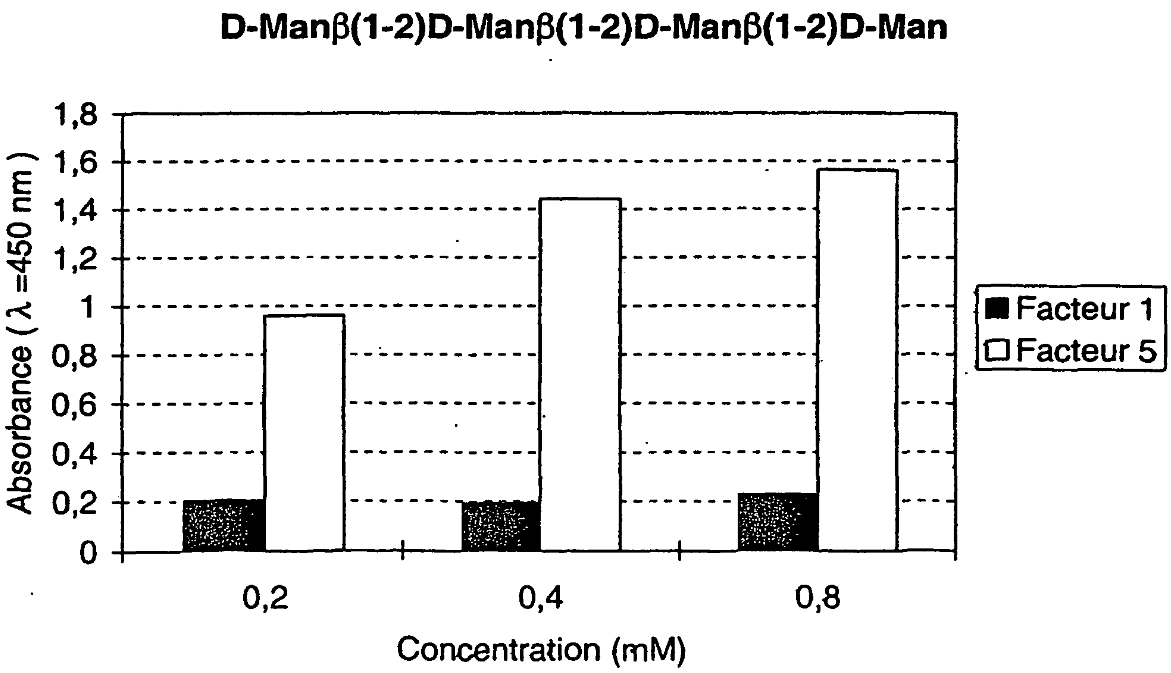
D-Manβ(1-2)D-Manβ(1-2)D-Manβ(1-2)D-Man
Absorbance ( λ =450 nm )
1,8
1,6
1,4
1,2
1
0,8
0,6
0,4
0,2
0
0,2
0,4
0,8
Concentration (mM)
Facteur 1
Facteur 5

Fig.7

D-Manβ(1-2)D-Manβ(1-2)D-Manβ(1-2)D-Man

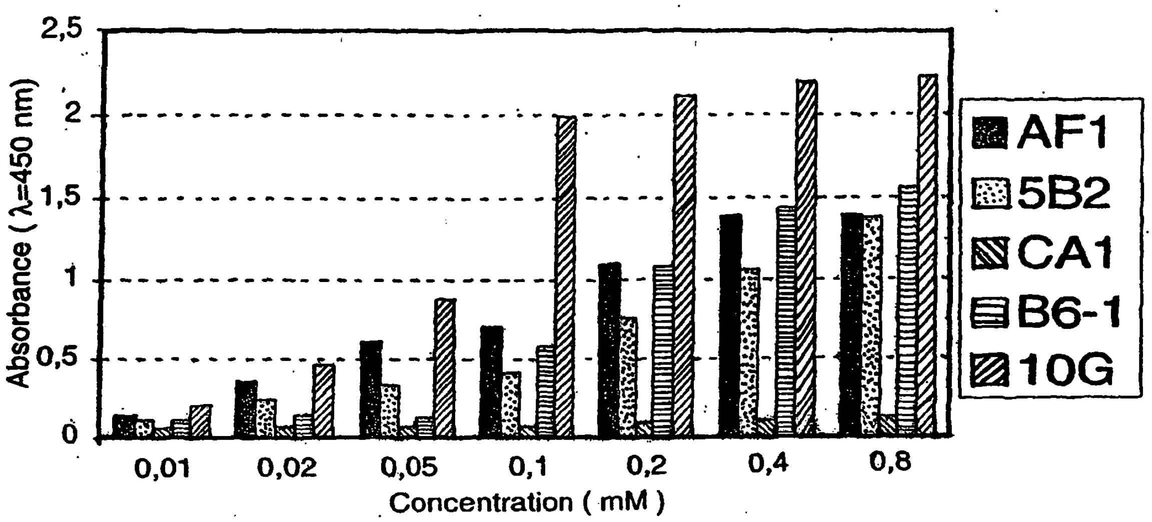


Fig.8

D-Manα(1-3)D-Manα(1-2)D-Manα(1-2)D-Man

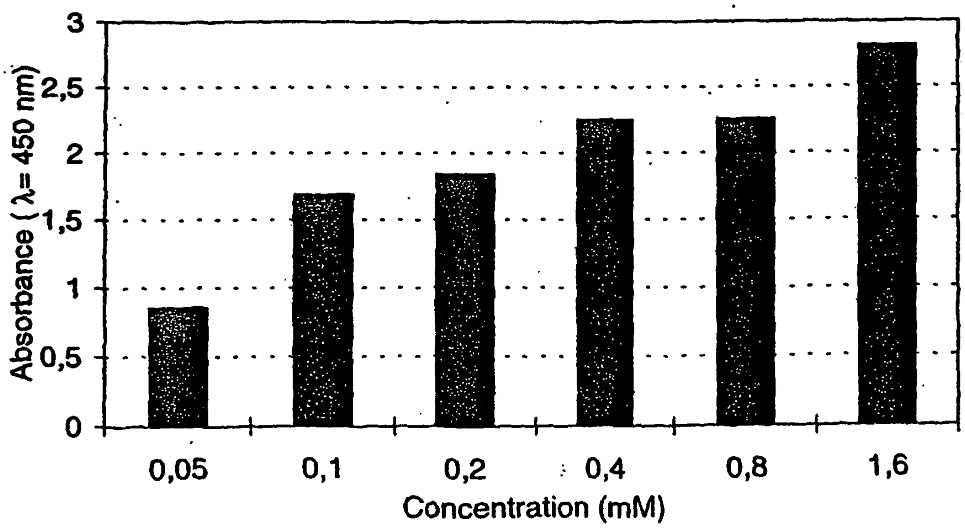

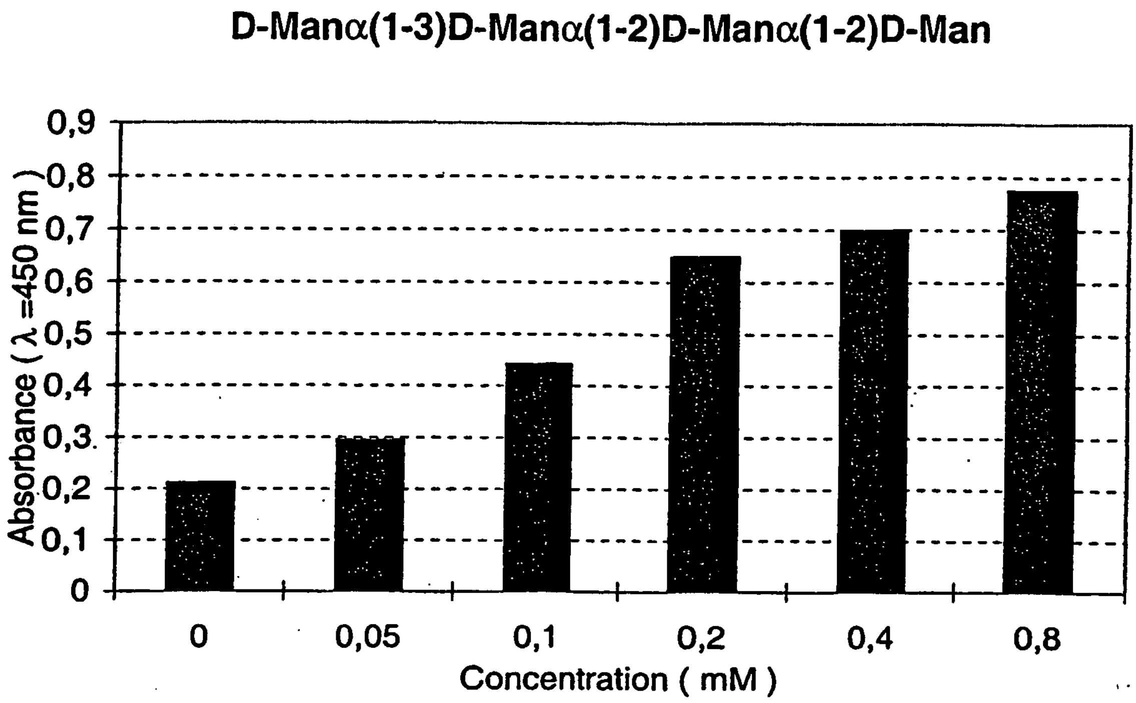
D-Manα(1-3)D-Manα(1-2)D-Manα(1-2)D-Man
Absorbance ( λ =450 nm )
0,9
0,8
0,7
0,6
0,5
0,4
0,3
0,2
0,1
0
0
0,05
0,1
0,2
0,4
0,8
Concentration ( mM )

Fig.9

Fig. 10

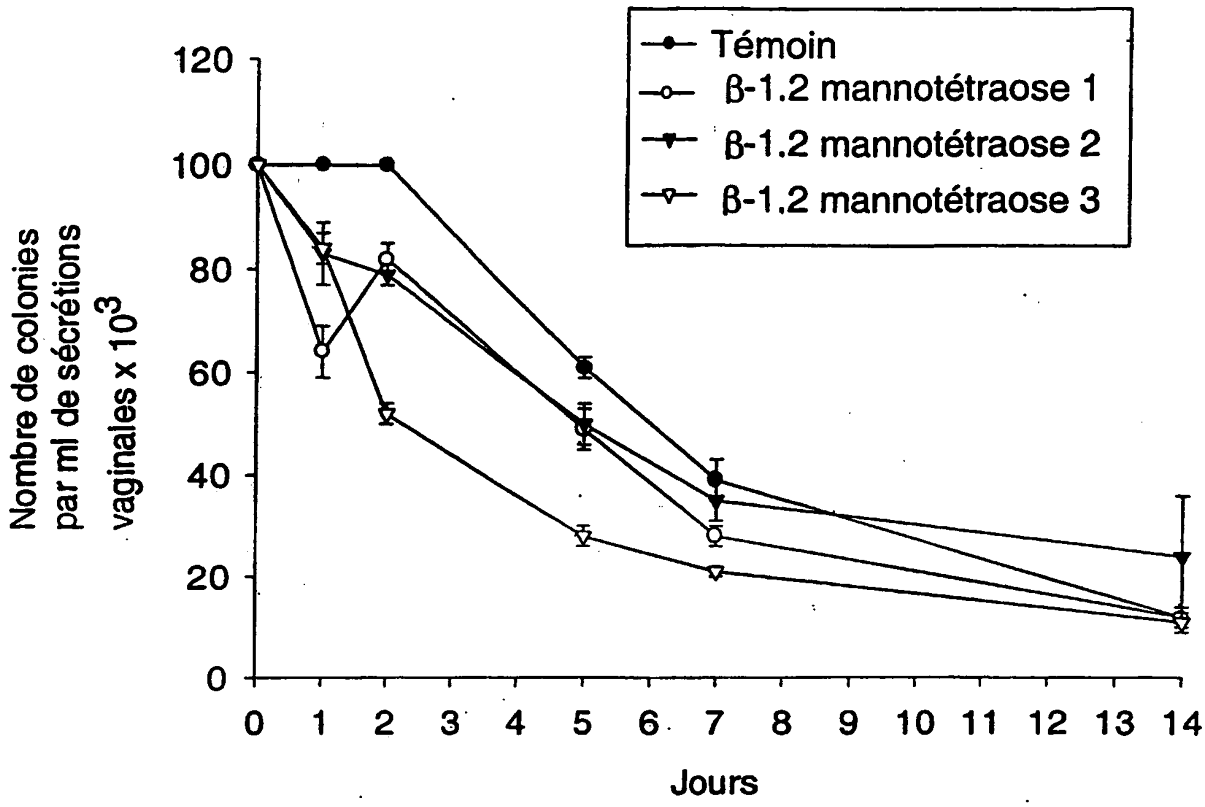
Témoin
β-1.2 mannotétraose 1
β-1.2 mannotétraose 2
β-1.2 mannotétraose 3
Nombre de colonies par ml de sécrétions vaginales x 10$^3$
0
20
40
60
80
100
120
0 1 2 3 4 5 6 7 8 9 10 11 12 13 14
Jours

Fig. 11

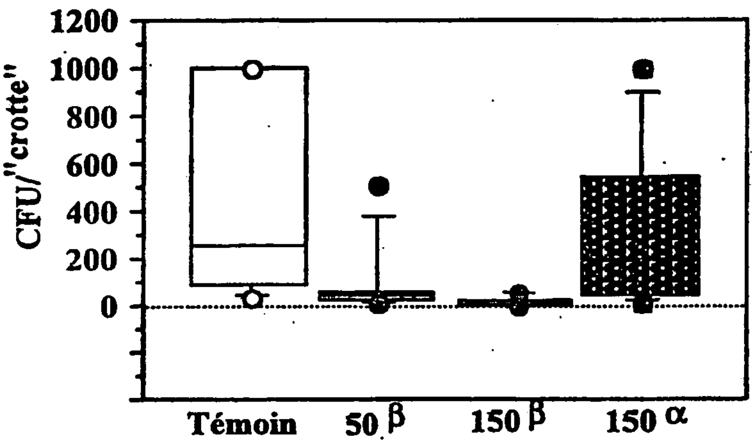

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **Sendid, B. et al.** *J. Clin. Microbiol.,* 1999, vol. 37 (5), 1510-7
- **Sendid, B. et al.** *Clin. Diagn. Lab. Immunol.,* 1996, vol. 3 (2), 219-26
- **Jouault, T.** *Clin. Diagn. Lab. Immunol.,* 1997, vol. 4 (3), 328-33
- **Lemieux, R.U. et al.** *J. Am. Chem. Soc.,* 1975, vol. 97, 4076-4083
- **Stutz, A. et al.** *Carbohydr.Res.,* 1985, vol. 137, 282-290
- **Maity, S. K. et al.** *Tetrahedron,* 1994, vol. 50, 6965-6974
- **Lemieux, R. U. et al.** *J. Am. Chem. Soc.,* 1975, vol. 97, 4076-4083
- **Gerlach, H. et al.** *Helv. Chim. Acta,* 1978, vol. 61, 1226-1231
- **Z Szurmai ; L Balatoni ; A.Liptak.** *Carbohydr. Res.,* 1994, vol. 254, 301-309
- **T Oshitari ; S Kobayashi.** *Tetrahedron Lett,* 1995, 1089-92
- **H Franzyk ; M Medal ; H Paulsen ; K Bock.** *J. Chem Soc. Perkin Trans I,* 1995, 2883-98
- **R. Albert ; K Dax ; R. Pleschko ; A Stütz.** *Carbohydr. Res.,* 1985, vol. 137, 282-290
- **H. Gerlach ; P. Künzler ; K. Oertle.** *Helv Chim. Acta,* 1978, vol. 61, 1226-1231
- **F Yamazaki ; S Sato ; T Nukuda ; Y. Ito ; T Ogawa.** *Carbohydr. Res.,* 1990, vol. 201, 31-50
- **Y-M. Zhang ; J.-M. Mallet ; P. Sinaÿ.** *Carbohydr. Res.,* 1992, vol. 236, 73-88
- **N. E. Franks ; R Montgomery.** *Carbohydr. Res.,* 1968, vol. 6, 286-98
- **P. J. Garegg ; H. Hultberg ; T Norberg.** *Carbohydr. Res.,* 1981, vol. 96, 59-64
- *Nunc TechNote,* vol. 4 (27-28
- **Timkovich, R.** *Anal. Biochem.,* 1977, vol. 79, 139-143
- **Staros, J. V. et al.** *Anal. Biochem.,* 1986, vol. 156, 220-222
- **Rasmussen, S. E.** *Ann. Biol. Clin.,* 1990, vol. 48, 647-50
- **Suzuki, S. et al.** *Curr. Top. Med. Mycol.,* 1997, vol. 8, 57-70
- **Cailliez, J. C. et al.** *Ann. Inst. Pasteur. Microbiol.,* 1988, vol. 139, 171-88
- **Hopwood, V. et al.** *Infect. Immun.,* 1986, vol. 54, 222-227
- **Poulain, D. et al.** *Mycoses,* 1991, vol. 34, 221-226
- **Trinel, P.A.** *Infect. Immun.,* 1992, vol. 60, 3845-3851
- **De Bernardis, F. et al.** *J. Clin. Microbiol.,* 1993, vol. 31, 3142-3146
- **De Bernardis, F. et al.** *Infect. Immun.,* 1994, vol. 62, 509-519
- **Girmenia, C. et al.** *J. Clin. Microbiol.,* 1997, vol. 35, 903-906
- **Torosantucci, A. et al.** *J. Gen. Microbiol.,* 1990, vol. 136, 2155-2263
- **Li, R. et al.** *J. Gen. Microbiol.,* 1991, vol. 137, 455-464
- **Li, R. et al.** *J. Biol. Chem.,* 1993, vol. 268, 18293-8
- **Kanbe, T. et al.** *Infect. Immun.,* 1994, vol. 62, 1662-8
- **Han, Y. et al.** *Infect. Immun.,* 1995, vol. 63, 2714-9
- **Han, Y. et al.** *J. Infect. Dis.,* 1997, vol. 175, 1169-75
- **Han, Y. et al.** *Infect. Immun.,* 1997, vol. 65, 4100-7
- **s Han, Y. et al.** *Infect. Immun.,* 1998, vol. 66, 5771-6
- **Zhang, M. X. et al.** *Infect. Immun.,* 1998, vol. 66, 6027-9
- **Jacquinot, P. M. et al.** *FEMS Microbiol. Lett.,* 1998, vol. 169, 131-8
- **Sendid, B. et al.** *Clin. Diagn. Lab. Immunol.,* 1996, vol. 3, 219-26
- **Young, M. et al.** *Glycoconj. J.,* 1998, vol. 15, 815-22
- **F. de Bernardis et al.** *Infection and Immunity,* 1998, vol. 66, 3317-3325